# EUROPÄISCHE PATENTSCHRIFT

(11) **EP 2 350 026 B1**
(45) Veröffentlichungstag und Bekanntmachung des Hinweises auf die Patenterteilung: **18.06.2014**
(21) Anmeldenummer: 09778874.9
(22) Anmeldetag: 09.10.2009
(51) Int. Cl.: C07D 239/47, A61K 31/505, A61P 35/00

(54) **SULFOXIMINSUBSTITUIERTE ANILINOPYRIMIDINDERATIVE ALS CDK-INHIBITOREN, DEREN HERSTELLUNG UND VERWENDUNG ALS ARZEINMITTEL**
SULFOXIMINE SUBSTITUTED ANILINE PYRIMIDINE DERIVATIVES AS CDK INHIBITORS, THEIR MANUFACTURE AND USE AS MEDICINE
DÉRIVÉS D'ANILINO-PYRIMIDINE SUBSTITUÉS PAR SULFOXIMINE EN TANT QU'INHIBITEURS DE CDK, LEUR FABRICATION ET LEUR UTILISATION EN TANT QUE MÉDICAMENTS

(30) Priorität: 21.10.2008 EP 08167113
(43) Veröffentlichungstag der Anmeldung: 03.08.2011
(73) Patentinhaber: Bayer Intellectual Property GmbH, 40789 Monheim (DE)
(72) Erfinder: LÜCKING, Ulrich, 10317 Berlin (DE); JAUTELAT, Rolf, 16548 Glienicke/Nordbahn (DE); SIEMEISTER, Gerhard, 13503 Berlin (DE); SCHULZE, Julia, 10407 Berlin (DE); LIENAU, Philip, 10997 Berlin (DE)
(74) Vertreter: BIP Patents
(86) Internationale Anmeldenummer: PCT/EP2009/007247
(87) Internationale Veröffentlichungsnummer: WO 2010/046035

(56) Entgegenhaltungen:
- WO-A-2005/037800
- WO-A-2007/071455
- WO-A-2007/140957
- VERMA S ET AL: "Substituted aminobenzimidazole pyrimidines as cyclin-dependent kinase inhibitors" BIOORGANIC & MEDICINAL CHEMISTRY LETTERS, PERGAMON, ELSEVIER SCIENCE, GB, Bd. 15, Nr. 8, 15. April 2005 (2005-04-15) , Seiten 1973-1977, XP025314402 ISSN: 0960-894X [gefunden am 2005-04-15]

## Beschreibung

Die vorliegende Erfindung betrifft ausgewählte sulfoximinsubsituierte Anilino-Pyrimidinderivate, deren Verfahren zur Herstellung sowie deren Verwendung als Medikament zur Behandlung verschiedener Erkrankungen.

Die Zyklin-abhängigen Kinasen (cyclin-dependent kinase, CDK) sind eine Enzymfamilie, die eine wichtige Rolle bei der Regulation des Zellzyklusses spielt und somit ein besonders interessantes Ziel für die Entwicklung kleiner inhibitorischer Moleküle darstellt. Selektive Inhibitoren der CDKs können zur Behandlung von Krebs oder anderen Erkrankungen, die Störungen der Zellproliferation zur Ursache haben, verwendet werden.

Pyrimidine und Analoga sind bereits als Wirkstoffe beschrieben wie beispielsweise die 2-Anilino-Pyrimidine als Fungizide (DE 4029650) oder substituierte Pyrimidinderivate zur Behandlung von neurologischen oder neurodegenerativen Erkrankungen (WO 99/19305). Als CDK-Inhibitoren werden unterschiedlichste Pyrimidinderivate beschrieben, beispielsweise 2-Amino-4-substituierte Pyrimidine (WO 01/ 14375), Purine (WO 99/02162), 5-Cyano-Pyrimidine (WO 02/04429), Anilinopyrimidine (WO 00/12486) und 2-Hydroxy-3-N,N-dimethylaminopropoxy-Pyrimidine (WO 00/39101).

Insbesondere wurden in WO 02/096888 und WO 03/076437 Pyrimidinderivate offenbart, die inhibitorische Wirkungen bezüglich CDKs aufweisen.

Verbindungen, die eine Phenylsulfonamid-Gruppe enthalten, sind als Inhibitoren der humanen Carboanhydrasen (insbes. Carboanhydrase-2) bekannt und werden als Diuretica u.a. zur Behandlung von Glaucom eingesetzt. Das Stickstoffatom und die Sauerstoffatome des Sulfonamids binden über Wasserstoffbrücken mit dem Zink²⁺-Ion und der Aminosäure Thr 199 im aktiven Zentrum der Carboanhydrase-2 und blockieren dadurch deren enzymatische Funktion (A. Casini, F. Abbate, A. Scozzafava, C.T. Supuran, Bioorganic. Med. Chem. Lett. 2003, 1, 2759). Die klinische Verwendung von CDK-Inhibitoren, die eine Phenylsulfonamid-Gruppe enthalten, könnte durch die Möglichkeit der Inhibition der Carboanhydrasen und ein daraus resultierendes Nebenwirkungsspektrum eingeschränkt sein.

Beispiele für Sulfoximin-Wirkstoffe sind sulfonimidoyl-modifizierte Triazole als Fungizide (H. Kawanishi, H. Morimoto, T. Nakano, T. Watanabe, K. Oda, K. Tsujihara, Heterocycles 1998, 49, 181) oder Arylalkylsulfoximine als Herbizide und Pestizide (Shell International Research, Ger. P. 2 129 678).

WO 2005/037800 offenbart offene sulfoximinsubsituierte Anilino-Pyrimidinderivate als Inhibitoren der Zyklin-abhängigen Kinasen. Beispielhaft belegt sind Strukturen, die in der 5-Position des Pyrimidins entweder nicht oder mit Halogen, insbesondere mit Brom substituiert sind. Einen 5-Trifluormethylsubstituenten weist keine der spezifisch offenbarten Strukturen auf.

Ausgehend von diesem Stand der Technik besteht die Aufgabe der vorliegenden Erfindung Verbindungen bereitzustellen, die nicht nur CDK potent inhibieren, sondern die auch das Tumorwachstum effektiv hemmen. Zwar ist die potente CDK-Inhibition eine notwendige, aber nicht hinreichende Vorraussetzung für eine effektive Tumorhemmung. Hierfür sind weitere Eigenschaften der Strukturen notwendig, wie zum Beispiel die Penetrationseigenschaften in die Tumorzelle.

Es wurde nun gefunden, dass Verbindungen der allgemeinen Formel (I) 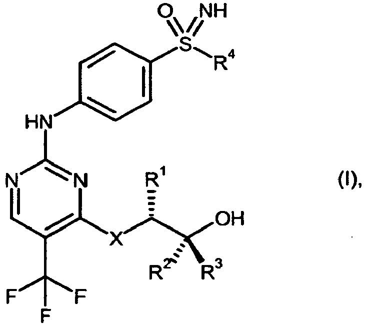 in der
- X: für -O- oder -NH- steht, und
- R¹: für eine Methyl-, Ethyl-, Propyl- oder Isopropylgruppe steht, und
- R² und R³: unabhängig voneinander für Wasserstoff, eine Methyl- oder Ethylgruppe stehen, und
- R⁴: für eine C₁-C₆-Alkylgruppe oder einen C₃-C₇-Cycloalkylring steht,

sowie deren Salze, Diatereomere und Enantiomere,
nicht nur CDK potent inhibieren, sondern auch das Tumorwachstum besonders effektiv hemmen.

Verbindungen, in denen X für -O- steht, werden mit der Formel (la) zusammengefasst.

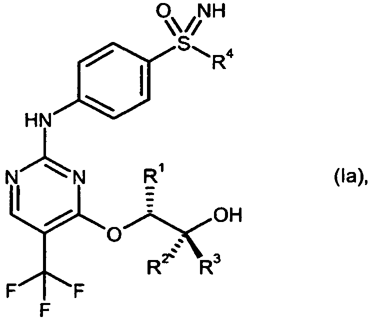

Verbindungen, in denen X für -NH- steht, werden mit der Formel (Ib) zusammengefasst.

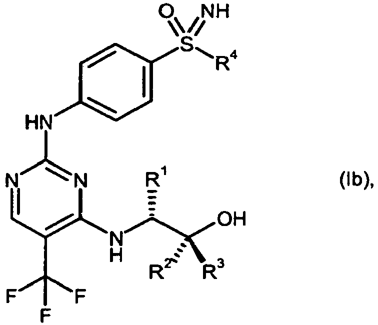

Der Anmeldung liegen folgende Definitionen zugrunde:

### C₁-C₆-Alkyl

Unter einer C₁-C₆-Alkylgruppe ist jeweils ein geradkettiger oder verzweigter Alkylrest zu verstehen, wie beispielsweise ein Methyl-, Ethyl-, Propyl- Isopropyl-, Butyl-, Isobutyl-, sek. Butyl-, tert. Butyl-, Pentyl-, Isopentyl- oder ein Hexylrest.

### C₃-C₇-Cycloalkyl

Unter einem C₃-C₇-Cycloalkylring ist ein Cyclopropyl- Cyclobutyl-, Cyclopentyl-, Cyclohexyl- oder ein Cycloheptylring zu verstehen.

In der allgemeinen Formel (I) kann X stehen für -O- oder -NH-.

Bevorzugt steht X für -O-.

In der allgemeinen Formel (I) kann R¹ stehen für eine Methyl-, Ethyl-, Propyl- oder Isopropylgruppe.

Bevorzugt steht R¹ für eine Methylgruppe.

In der allgemeinen Formel (I) können R² und R³ unabhängig voneinander stehen für Wasserstoff, eine Methyl- oder Ethylgruppe.

Bevorzugt stehen R² und R³ unabhängig voneinander für Wasserstoff oder eine Methylgruppe.

Besonders bevorzugt steht R² für eine Methylgruppe und R³ für Wasserstoff oder eine Methylgruppe.

In der allgemeinen Formel (I) kann R⁴ stehen für einen C₁-C₆-Alkylrest oder einen C₃-C₇-Cycloalkylring. -

Bevorzugt steht R⁴ für eine Methyl- oder Ethylgruppe oder für einen Cyclopropylring.

Eine bevorzugte Untergruppe der Verbindungen gemäß der allgemeinen Formel (I) sind Verbindungen gemäß allgemeiner Formel (I),
in der
- X: für -O- oder -NH- steht, und
- R¹: für eine Methylgruppe steht, und
- R²: für eine Methylgruppe steht, und
- R³: für Wasserstoff oder eine Methylgruppe steht, und
- R⁴: eine Methyl- oder Ethylgruppe oder für einen Cyclopropylring steht,
sowie deren Salze, Diatereomere und Enantiomere.

Die erfindungsgemäßen Verbindungen eignen sich zur Behandlung
- von Krebs, wie solide Tumore, Tumormetastasen, und Hämatologische Tumoren, insbesondere:
   Kopf- und Halstumore; Lungen- und Bronchialtumore; Gastrointestinaltumore wie z.B. Magenkarzinom, Kolorektales Karzinom, Pankreaskarzinom, Hepatozelluläres Karzinom; Endokin aktive Tumore; Mammakarzinome und
   Gynäkologische Tumore; Urogenitaltumore, wie z.B. Nierenzellkarzinom, Hamblasenkarzinom, Prostatakarzinom; Tumore der Haut; Sarkome; Leukämien und Lymphome.
- von viralen Erkrankungen, sowie
- von kardiovaskulären Erkrankungen wie Stenosen, Arteriosklerosen und Restenosen, Stent-induzierte Restenosen.

Die Formulierung der erfindungsgemäßen Verbindungen zu pharmazeutischen Präparaten erfolgt in an sich bekannter Weise, indem man den oder die Wirkstoffe mit den in der Galenik gebräuchlichen Hilfsstoffen in die gewünschte Applikationsform überführt.

Als Hilfsstoffe können dabei beispielsweise Trägersubstanzen, Füllstoffe, Sprengmittel, Bindemittel, Feuchthaltemittel, Gleitmittel, Ab- und Adsorptionsmittel, Verdünnungsmittel, Lösungsmittel, Cosolventien, Emulgatoren, Lösungsvermittler, Geschmackskorrigentien, Färbemittel, Konservierungs-, Stabilisierungs-, Netzmittel, Salze zur Veränderung des osmotischen Drucks oder Puffer zum Einsatz kommen. Dabei ist auf Remington's Pharmaceutical Science, 15th ed. Mack Publishing Company, East Pennsylvania (1980) hinzuweisen.

Die pharmazeutischen Formulierungen können
in fester Form, zum Beispiel als Tabletten, Dragees, Pillen, Suppositorien, Kapseln, transdermale Systeme oder
in halbfester Form , zum Beispiel als Salben, Cremes, Gele, Suppositiorien, Emulsionen oder
in flüssiger Form, zum Beispiel als Lösungen, Tinkturen, Suspensionen oder Emulsionen vorliegen.

Hilfsstoffe im Sinne der Erfindung können beispielsweise Salze, Saccharide (Mono-, Di-, Tri-, Oligo-, und/oder Polysaccharide), Proteine, Aminosäuren, Peptide, Fette, Wachse, Öle, Kohlenwasserstoffe sowie deren Derivate sein, wobei die Hilfsstoffe natürlichen Ursprungs sein können oder synthetisch bzw. partial synthetisch gewonnen werden können.

Für die orale oder perorale Applikation kommen insbesondere Tabletten, Dragees, Kapseln, Pillen, Pulver, Granulate, Pastillen, Suspensionen, Emulsionen oder Lösungen in Frage.

Für die parenterale Applikation kommen insbesondere Suspensionen, Emulsionen und vor allem Lösungen in Frage.

### Herstellung der erfindungsgemäßen Verbindungen

Sulfoximine besitzen in Bezug auf Struktur und Konfiguration in der Regel eine hohe Stabilität (C. Bolm, J.P. Hildebrand, J. Org. Chem. 2000, 65, 169).

Diese Eigenschaften der funktionellen Gruppe erlauben oftmals auch drastische Reaktionsbedingungen und ermöglichen die einfache Derivatisierung der Sulfoximine am Imin-Stickstoff und dem α-Kohlenstoff. Enantiomerenreine Sulfoximine werden auch als Auxiliare in der diastereoselektiven Synthese verwendet ((a) S.G. Pyne, Sulfur Reports 1992, 12, 57; (b) C.R. Johnson, Aldrichimica Acta 1985, 18, 3).

Die Darstellung enantiomerenreiner Sulfoximine ist z.B. über die Racematspaltung mit enantiomerenreiner Campher 10-sulfonsäure beschrieben ((a) C.R. Johnson, C.W. Schroeck, J. Am. Chem. Soc. 1973, 95, 7418; (b) C.S. Shiner, A.H. Berks, J. Org. Chem. 1988, 53, 5542). Eine weitere Methode zur Darstellung optisch aktiver Sulfoximine besteht in der stereoselektiven Iminierung von optisch aktiven Sulfoxiden ((a) C. Bolm, P. Müller, K. Harms, Acta Chem. Scand. 1996, 50, 305; (b) Y. Tamura, J. Minamikawa, K. Sumoto, S. Fujii, M. Ikeda, J. Org. Chem. 1973, 38, 1239; (c) H. Okamura, C. Bolm, Org. Lett. 2004, 6, 1305).

Für Übersichtsartikel zu Sulfoximinen siehe z.B.: a) M. Regglin, C. Zur, Synthesis 2000, 1; (b) C.R. Johnson, Aldrichimica Acta 1985, 18, 3).

Die nachfolgenden Beispiele erläutern die Herstellung der erfindungsgemäßen Verbindungen, ohne den Umfang der beanspruchten Verbindungen auf diese Beispiele zu beschränken.

### Herstellung der Verbindungen der Formel (Ia) (4-O Derivate)

Die erfindungsgemäßen Verbindungen können hergestellt werden durch ein Verfahren, das durch folgende Schritte gekennzeichnet ist:
a) Oxidation einer Verbindung der Formel **(IVd)** zum Sulfoxid der Formel **(IVc).** 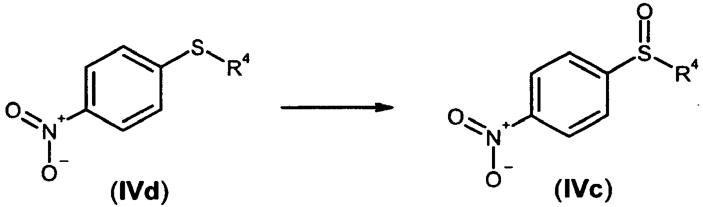
b₁) Direkte Iminierung des Sulfoxides der Formel **(IVc)** zu einem geschützten Sulfoximin der Formel **(IVa).** 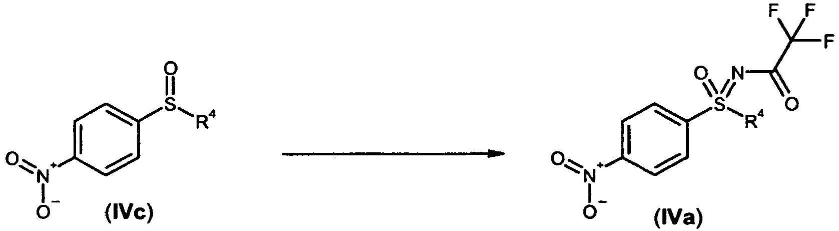 oder
b₂) Iminierung des Sulfoxides der Formel **(IVc)** zu einem ungeschützten Sulfoximin der Formel **(IVb)** und anschließende Einführung der Schutzgruppe zu einer Verbindung der Formel **(IVa).** 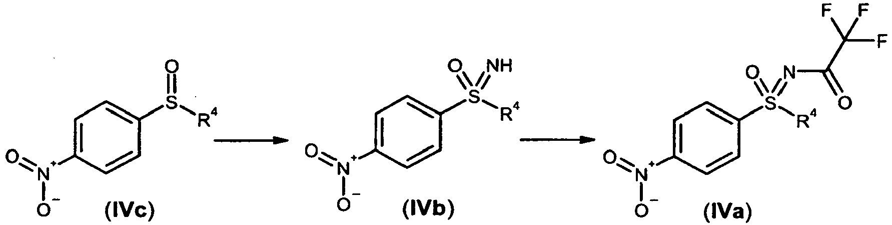
c) Reduktion der Verbindung der Formel **(IVa)** zu einer Verbindung der Formel **(IV)** 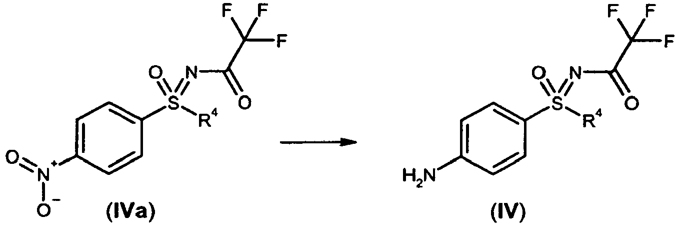
d) Funktionalisierung der 4-Position von 2,4-Dichlor-5-jod-pyrimidin **(VII)** durch Umsetzung mit einem mono-geschützten Diol der Formel **(VI)** unter Bildung eines Intermediates der Formel (Va). 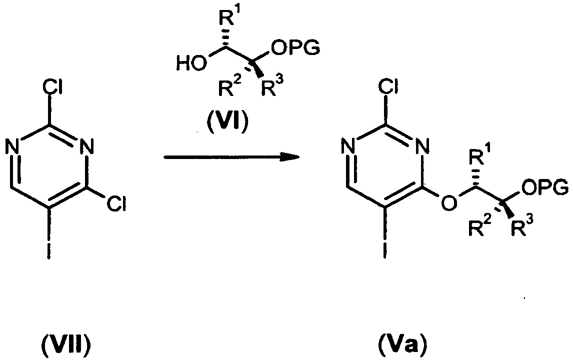
e) Herstellung des 5-CF₃ Intermediates **(V).** 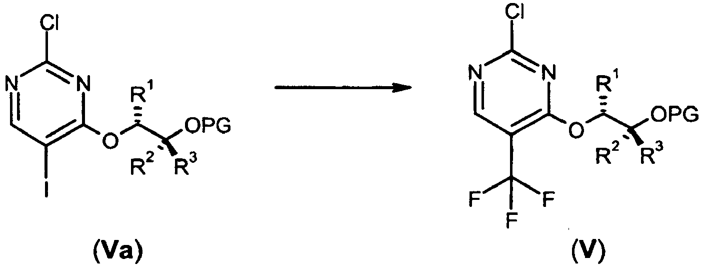
f) Kupplung der Verbindungen der Formel **(IV)** und **(V)** zum Intermediat der Formel **(III).** 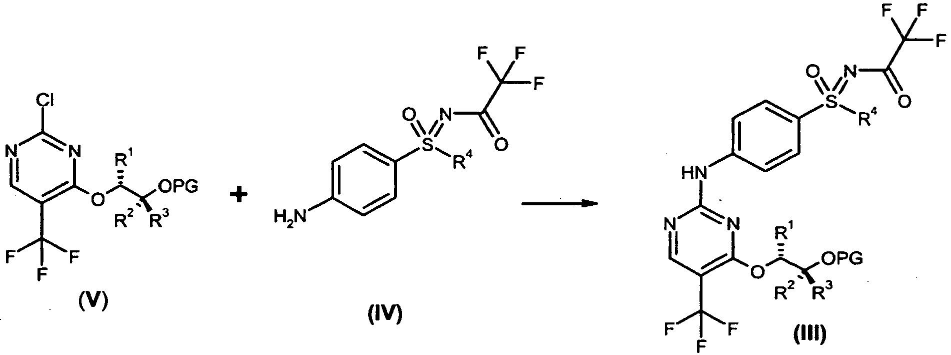
g) Abspaltung der Schutzgruppe (PG) unter Bildung von **(II).** 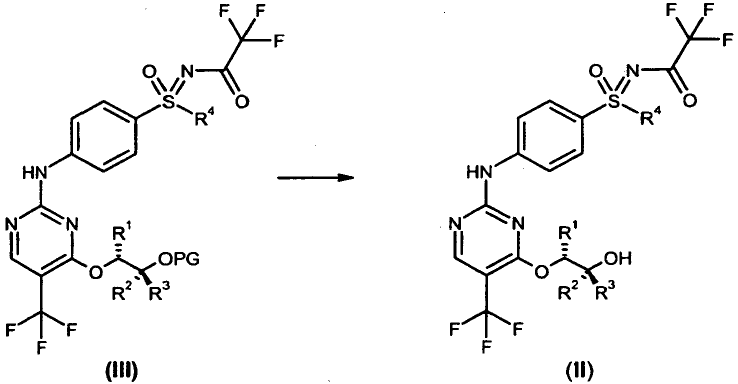
h) Abspaltung der Schutzgruppe am Sulfoximin unter Bildung von **(Ia).** 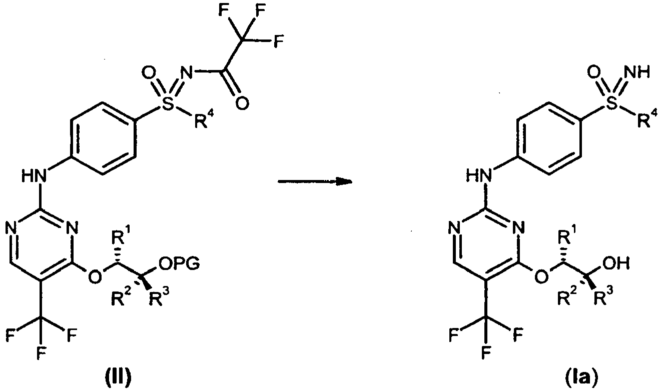
wobei die Substituenten R¹, R², R³ und R⁴ die in der allgemeinen Formel (I) angegebenen Bedeutungen aufweisen.

### Verfahrensschritt a)

Eine Verbindung der Formel **(IVd)** wird zum Sulfoxid der Formel **(IVc)** oxidiert. Für die Überführung eines Thioethers in ein Sulfoxid stehen zahlreiche Methoden, auch stereoselektive, zur Verfügung (siehe z.B.: (a) M.H. Ali et al, Synthesis 1997, 764; b) M.C. Carreno, Chem. Rev. 1995, 95, 1717; c) I. Patel et al, Org. Proc. Res. Dev. 2002, 6, 225; c) N. Khiar et al, Chem. Rev. 2003, 103, 3651). Besonders geeignet für die Darstellung von Verbindungen der Formel **(IVc)** ist die beschriebene Verwendung von Perjodsäure / Eisen(III)chlorid.

### Verfahrensschritt b₁)

Die Umsetzung eines Sulfoxides der Formel **(IVc)** mit Trifluoracetamid in Kombination mit Jodbenzol-diacetat, Magnesiumoxid und katalytischen Mengen von Rhodium(II)-acetat Dimer ermöglicht die Darstellung eines geschützten Sulfoximins der Formel **(IVa).** Diese Reaktion ist stereospezifisch und verläuft unter Retention der Konfiguration am Stereozentrum (siehe: (a) H. Okamura, C. Bolm, Org. Lett. 2004, 6, 1305).

### Verfahrensschritt b₂)

Hierbei erfolgt zunächst die Umsetzung eines Sulfoxides der Formel **(IVc)** zu einem ungeschützten Sulfoximin der Formel **(IVb).** Geeignete Methoden sind beispielsweise:
a) Die Umsetzung des Sulfoxides mit Natriumazid / konz. Schwefelsäure (siehe z.B.: (a) C.R. Johnson, P.E. Rogers, J. Org. Chem. 1973, 38, 1793).
b) Die Umsetzung des Sulfoxides mit Natriumazid / Oleum (siehe z.B.: (a) N.V. Kondratenko, O.A. Radchenko, L.M. Yagupol'ski, J. Org. Chem. USSR 1984, 20, 2051).
c) Die Umsetzung des Sulfoxides mit o-Mesitylensulfonylhydroxylamin (MSH) (siehe z.B.: (a) C.R. Johnson, R.A. Kirchhoff, H.G. Corkins, J. Org. Chem. 1974, 39, 2458). Die anschließende Einführung der Schutzgruppe unter Bildung von Verbindungen der Formel **(IVa)** kann beispielsweise wie beschrieben durch die Umsetzung mit Trifluoracetanhydrid unter basischen Bedingungen erfolgen.

### Verfahrensschritt c)

Für die anschließende Reduktion der aromatischen Nitrogruppe zu einer Verbindung der Formel **(IV)** stehen prinzipiell eine Reihe von Reaktionsbedingungen zur Verfügung (siehe z.B.: R.C. Larock, Comprehensive Organic Transformations, VCH, New York, 1989, 411). Besonders geeignet ist beispielsweise die beschriebene Verwendung von Titan(III)chlorid oder die Hydrierung unter Verwendung von Palladium auf Kohle.

### Verfahrensschritt d)

Die Umsetzung von 2,4-Dichlor-5-jod-pyrimidin **(VII)** mit einem Alkohol der Formel **(VI)** ermöglicht die Darstellung eines Intermediates der Formel **(Va)** (siehe z.B.: U. Lücking et al, WO2007/071455).

### Verfahrensschrit e)

Für den Austausch eines Halogens gegen eine Trifluormetyhlgruppe in einem stickstoffhaltigen Heteroaromaten stehen prinzipiell verschiedene Methoden zur Verfügung (siehe z.B: a) G.E. Carr, R.D. Chambers, T.F. Holmes, J. Chem. Soc. Perkin Trans. 1, 1988, 921; b) F. Cottet, M. Schlosser, Eur. J. Org. Chem. 2002, 327; c) F.G. Njoroge et al, J. Med. Chem 1997, 40, 4290).

Für die Einführung in die 5-Position des Pyrimidins **(Va)** unter Bildung von Intermediat **(V)** ist besonders die beschriebene Verwendung von Kupfer(I)jodid, Kaliumfluorid und (Trifluormethyl)-trimethylsilan in N-Methyl-2-pyrrolidinon und THF geeignet.

### Verfahrensschritt f)

Ein 2-Chlor-pyrimidin der Formel **(V)** kann mit einem Anilin der Formel **(IV)** zu einem Intermediat der Formel **(III)** umgesetzt werden (siehe z.B.: (a) J. Bryant et al, WO 2004/048343).

### Verfahrensschritt g)

Die Abspaltung der Schutzgruppe (PG) aus dem Intermediat **(III)** liefert das Intermediat **(II)** (siehe z.B.: P.J. Kocienski, Protecting Groups, Georg Thieme Verlag Stuttgart, New York, 1994).

Für die beschriebene Abspaltung einer Benzylgruppe ist die beschriebene Hydrierung besonders geeignet. Die Abspaltung einer THP-Gruppe kann ggf. bereits unter den Bedingungen des Verfahrensschrittes f) erfolgen.

### Verfahrensschritt h)

Die Abspaltung der Trifluoracetogruppe am Sulfoximin **(II)** liefert die Verbindung der Formel **(Ia).** Besonders geeignet ist hierfür die beschriebene Methodik unter Verwendung von Kaliumcarbonat in Methanol bei Raumtemperatur (siehe z.B.: (a) H. Okamura, C. Bolm, Org. Lett. 2004, 6, 1305).

### Allgemeine Hinweise

Alle Reaktionen mit oxidations- oder hydrolyseempfindlichen Verbindungen wurden unter Argon und mit getrockneten Lösungsmitteln durchgeführt.

Die Substanzbenennung mit Ausnahme der Sulfoximinderivate erfolgte unter Verwendung des Programms *Autonom 2000 Name,* welches in MDL ISIS Draw implementiert ist. *Autonom 2000 Name* akzeptiert keine Sulfoximine, daher erfolgte die Benennung der Sulfoximine anhand der IUPAC Regeln (IUPAC, Nomenclature of Organic Chemistry, 1979 Edition, C-6.3. Sulfoxides and Sulfones, Rule C-633, 633.1 Sulfimide and Sulfoximide).

**Abkürzungen**

| **Abkürzung** | **Bedeutung** |
|---|---|
| Ac | Acetyl |
| Aloc | Allyloxycarbonyl |
| Boc | tert-Butyloxycarbonyl |
| BOM | Benzyloxymethyl |
| br | Broad |
| CI | Chemical ionisation |
| d | Dublett |
| dd | Dublett vom Dublett |
| DCM | Dichlormethan |
| DMF | *N,N-*Dimethylformamid |
| DMSO | Dimethylsulfoxid |
| ESI | Electrospray ionisation |
| HPLC | High performance liquid chromatography |
| m | Multiplett |
| MEM | (2-Methoxyethoxy)methyl |
| MOM | Methoxymethyl |
| MS | Mass spectrometry |
| MTM | Methylthiomethyl |
| NMP | N-Methyl-2-pyrrolidinon |
| NMR | Nuclear magnetic resonance spectroscopy : Chemische Verschiebung (δ) wird in ppm angegeben. |
| Pg | Protecting group umfassend Gruppen wie z.B. TMS, TES, TBDMS, TBDPS, TIPS, Benzyl, PMB, Trityl, Allyl, Aloc, MOM, MTM, MEM, BOM, SEM, THP. |
| PMB | p-Methoxybenzyl |
| q | Quartett |
| s | Singulett |
| SEM | β-(Trimethylsilyl)ethoxymethyl |
| TBDMS | Tert.-Butylsilyldimethyl |
| TBDPS | Tert.-Butylsilyldiphenyl |
| TEA | Triethylamin |
| TES | Triethylsilyl |
| THF | Tetrahydrofuran |
| THP | Tetrahydropyranyl |
| TIPS | Triisopropyl |
| TMS | Trimethylsilyl |
| tr | Triplett |

### Beispiel 1

### (RS)-S-Cyclopropyl-S-(4-{[4-{[(1R,2R)-2-hydroxy-1-methylpropyl]oxy}-5-(trifluormethyl)pyrimidin-2-yl]amino}phenyl)sulfoximid

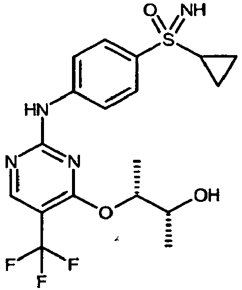

### 1a) Herstellung der Zwischenprodukte

### Verbindung 1.1

### 1-Cyclopropylsulfanyl-4-nitro-benzol

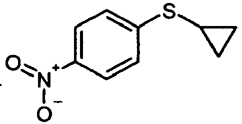

Eine Lösung von 3,00 g (40,5 mmol) Cyclopropanthiol (Herstellung nach: E. Block et al, J. Am. Chem. Soc. 1992, 114, 3492) in 100 ml THF / 100 ml Diethylether wurde portionsweise mit 1,78 g (44,6 mmol) Natriumhydrid (60%) versetzt und 30 Minuten bei Raumtemperatur gerührt. Anschließend erfolgte die portionsweise Zugabe von 6,00 g (38,7 mmol) 1-Fluor-4-nitro-benzol. Der Ansatz wurde 2 Stunden bei 40°C gerührt. Nach dem Abkühlen wurde der Ansatz in Wasser gegeben und mit Benzol extrahiert (3x). Die vereinten organischen Phasen wurden eingeengt und der Rückstand chromatographisch (Hexan / Essigester 95:5) gereinigt. Man erhielt 4,6 g (23,6 mmol; Ausbeute: 61%) des Produktes.
¹H-NMR (400 MHz, DMSO): δ = 8.12 (m, 2H), 7.54 (m, 2H), 2.35 (m, 1 H), 1.16 (m, 2H), 0.61 (m, 2H).

### Verbindung 1.2

### (RS)-1-Cyclopropansulfinyl-4-nitro-benzol

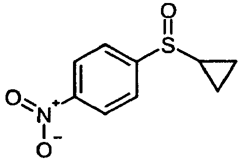

Ein Ansatz mit 7,2 g (36,88 mmol) 1-Cyclopropylsulfanyl-4-nitro-benzol in 140 ml Acetonitril wurde mit 179 mg (1,11 mmol) Eisen(III)-chlorid versetzt und 15 Minuten bei Raumtemperatur gerührt. Anschließend versetzte man bei 25 °C portionsweise mit 9,25 g (40,57 mmol) Perjodsäure. Der Ansatz wurde 30 Minuten gerührt und anschließend unter Rühren auf eine gekühlte, gesättigte Natriumthiosulfat-Lösung gegeben. Man extrahierte mit Essigester (2x). Die vereinten organischen Phasen wurden getrocknet (Na₂SO₄), filtriert und eingeengt. Der erhaltene Rückstand wurde chromatographisch (Hexan / Essigester 1:1) gereinigt. Man erhielt 5,93 g (28,07 mmol; Ausbeute: 76 %) des Produktes.
¹H-NMR (400 MHz, DMSO):δ = 8.41 (m, 2H), 7.98 (m, 2H), 2.60 (m, 1 H), 1.01 (m, 3H), 0.86 (m, 1H).

### Verbindung 1.3

### (RS)-S-Cyclopropyl-S-(4-nitrophenyl)-N-(trifluoracetyl)sulfoximid

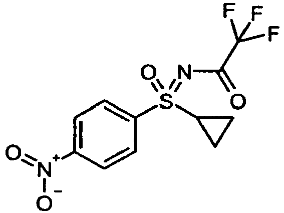

Eine Suspension aus 15,1 g (71,53 mmol) (RS)-1-Cyclopropansulfinyl-4-nitro-benzol, 17,8 g (157,37 mmol) Trifluoracetamid, 38,0 g (118,02 mmol) Jodbenzoldiacetat und 12,7 g (314,73 mmol) Magnesiumoxid in 801 ml DCM wurde unter Argon mit 1,58 g (3,58 mmol) Rhodium(II)acetat Dimer versetzt und über Nacht bei Raumtemperatur gerührt. Der Ansatz wurde über Celite abgesaugt und eingeengt. Der verbleibende Rückstand wurde chromatographisch (Hexan / Essigester 2:1) gereinigt. Man erhielt 18,0 g (55,97 mmol; Ausbeute: 78 %) des Produktes.
¹H-NMR (400 MHz, DMSO): δ = 8.49 (m, 2H), 8.25 (m, 2H), 3.56 (m, 1 H), 1.51 (m, 1H), 1.41 (m, 1 H), 1.18 (m, 2H).

### Verbindung 1.4

### (RS)-S-(4-Aminophenyl)-S-cyclopropyl-N-(trifluoracetyl)sulfoximid

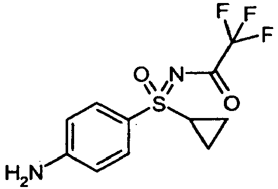

Eine Lösung von 6,9 g (21,44 mmol) (RS)-S-Cyclopropyl-S-(4-nitrophenyl)-N-(trifluoracetyl)sulfoximid in 214 ml Ethanol und 39 ml THF wurde mit 1,4 g Palladium auf Kohle (10%ig / 50% wasserfeucht) versetzt und 1 Stunde unter Normaldruck bei 25 °C hydriert. Man versetzte erneut mit 1,4 g Palladium auf Kohle und hydrierte weitere 4,5 Stunden bei Normaldruck. Der Ansatz wurde filtriert, das Filtrat erneut mit 1,4 g Palladium auf Kohle versetzt und abschließend 45 Minuten hydriert. Der Ansatz wurde filtriert und eingeengt. Man erhielt 5,8 g (19,91 mmol; Ausbeute: 93 %) des Produktes.
¹H-NMR (400 MHz, DMSO): δ = 7.53 (m, 2H), 6.71 (m, 2H), 6.40 (br, 2H), 3.21 (m, 1 H), 1.28 (m, 2H), 1.08 (m, 2H).

### Verbindung 1.5

### (2R,3R)-3-Benzyloxy-butan-2-ol

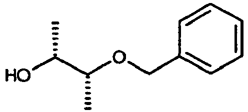

Eine Lösung von 4,00 g (44,4 mmol) (2R,3R)-Butan-2,3-diol in 300 ml THF wurde bei Raumtemperatur mit 5,00 g (44,6 mmol) Kalium-tert.-butylat versetzt und der Ansatz 15 Minuten refluxiert. Der Ansatz wurde auf ca. 50°C abgekühlt und mit 5,3 ml (44,6 mmol) Benzylbromid versetzt. Man refluxierte für 3 Stunden, danach wurde über Nacht bei Raumtemperatur gerührt. Der Ansatz wurde mit Essigester und Natriumchlorid-Lösung verdünnt und anschließend mit 1 N Chlorwasserstoff-Lösung (1x) und Natriumchlorid-Lösung (2x) gewaschen. Die organische Phase wurde getrocknet (Na₂SO₄), filtriert und eingeengt. Der erhaltene Rückstand wurde chromatographisch (Hexan / Essigester 1:1) gereinigt. Man erhielt 3,41 g (18,9 mmmol; Ausbeute: 43 %) des Produktes.
¹H-NMR (400 MHz, DMSO): δ = 7.35 (m, 4H), 7.28 (m, 1 H), 4.52 (m, 3H), 3.67 (m, 1 H), 3.37 (m, 1 H), 1.05 (d, 3H), 1.01 (d, 3H).

### Verbindung 1.6

### 4-((1R,2R)-2-Benzyloxy-1-methyl-propoxy)-2-chlor-5-jod-pyrimidin

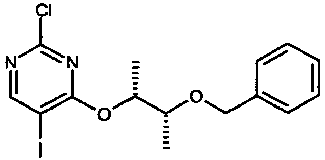

8,55 g (47,4 mmol) (2R,3R)-3-Benzyloxy-butan-2-ol in 56 ml Diethylether wurden bei 0°C unter Rühren portionsweise mit 2,07 g Natriumhydrid (55%) versetzt. Nach 10 Minuten wurde das Eisbad entfernt und weitere 3 Minuten bei Raumtemperatur gerührt. Die gebildete Suspension wurde bei 0°C zu einer Lösung von 6,52 g (23,7 mmol) 2,4-Dichlor-5-jod-pyrimidin gegeben. Der Ansatz wurde 4 Stunden bei 40°C gerührt und anschließend mit verdünnter Natriumchlorid-Lösung versetzt. Man extrahierte mit Essigester (2x). Die vereinten organischen Phasen wurden getrocknet (Na₂SO₄), filtriert und eingeengt. Der erhaltene Rückstand wurde chromatographisch (Hexan / Essigester 4:1). Man erhielt 4,12 g (9,8 mmol; Ausbeute: 41%) des Produktes.
¹H-NMR (400 MHz, DMSO): δ = 8.78 (s, 1 H), 7.29 (m, 5H), 5.27 (m, 1 H), 4.64 (d, 1 H), 4.53 (d, 1 H), 3.73 (m, 1 H), 1.30 (d, 3H), 1.19 (d, 3H).

### Verbindung 1.7

### 4-((1R,2R)-2-Benzyloxy-1-methyl-propoxy)-2-chlor-5-trifluormethyl-pyrimidin

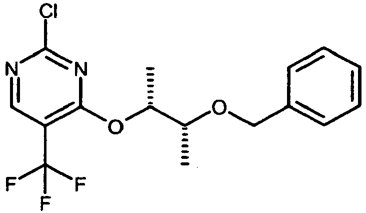

Eine Lösung von 4,66 g (11,1 mmol) 4-((1R,2R)-2-Benzyloxy-1-methyl-propoxy)-2-chlor-5-jod-pyrimidin in 15,8 ml NMP und 15,8 ml THF wurde bei Raumtemperatur unter Rühren mit 3,82 g (20,0 mmol) Kupfer(I)jodid, 0,97 g (16,7 mmol) Kaliumfluorid und 2,47 ml (16,7 mmol) (Trifluormethyl)-trimethylsilan versetzt. Der Ansatz wurde 5,5 Stunden bei 80°C gerührt. Nach dem Erkalten wurde der Ansatz in verdünnte Natriumchlorid-Lösung gegeben und mit Essigester extrahiert (2x). Die vereinten organischen Phasen wurden getrocknet (Na₂SO₄), filtriert und eingeengt. Der erhaltene Rückstand wurde chromatographisch (Hexan / Essigester 4: 1). Man erhielt 2,17 g (6,0 mmol; Ausbeute: 54%) des Produktes.
¹H-NMR (400 MHz, DMSO): δ = 8.81 (s, 1 H), 7.21 (m, 5H), 5.40 (m, 1 H), 4.57 (d, 1 H), 4.42 (d, 1 H), 3.70 (m, 1 H), 1.28 (d, 3H), 1.13 (d, 3H).

### Verbindung 1.8

### (RS)-S-(4-{[4-{[(1R,2R)-2-(Benzyloxy)-1-methylpropyl]oxy}-5-(trifluormethyl) pyrimidin-2-yl]amino}phenyl)-S-cyclopropyl-N-(trifluoracetyl)sulfoximid

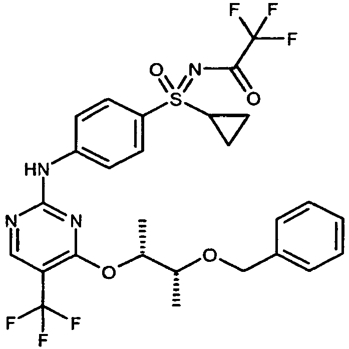

1,39 g (3,85 mmol) 4-((1R,2R)-2-Benzyloxy-1-methyl-propoxy)-2-chlor-5-trifluormethyl-pyrimidin und 1,35 g (4,62 mmol) (RS)-S-(4-Aminophenyl)-S-cyclopropyl-N-(trifluoracetyl)sulfoximid in 18,8 ml Acetonitril wurden mit 0,96 ml einer 4N Lösung von Chlorwasserstoff in Dioxan versetzt und 5 Stunden bei 80°C gerührt. Nach dem Abkühlen wurde der Ansatz mit Essigester verdünnt und mit gesättigter Natriumhydrogencarbonat-Lösung und gesättigter Natriumchlorid-Lösung gewaschen, getrocknet (Na₂SO₄), filtriert und eingeengt. Der erhaltene Rückstand wurde chromatographisch (Hexan / Essigester 4:1) gereinigt. Man erhielt 1,32 g (2,14 mmol, Ausbeute: 56%) des Produktes.
¹H-NMR (400 MHz, DMSO): δ = 10.71 (s, 1 H), 8.84 (s, 1 H), 8.08 (m, 2H), 7.93 (m, 2H), 7.26 (m, 5H), 5.52 (m, 1 H), 4.62 (d, 1 H), 4.51 (d, 1 H), 3.78 (m, 1 H), 3.35 (m, 1 H), 1.37 (m, 5H), 1.16 (m, 5H).

### Verbindung 1.9

### (RS)-S-Cyclopropyl-S-(4-{[4-{[(1R,2R)-2-hydroxy-1-methylpropyl]oxy}-5-(trifluormethyl)pyrimidin-2-yl]amino}phenyl)-N-(trifluoracetyl)sulfoximid

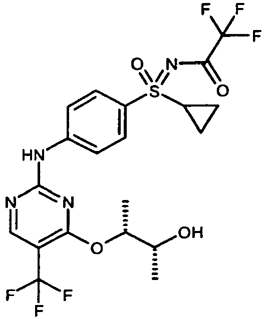

Eine Lösung von 1,31 g (2,12 mmol) (RS)-S-(4-{[4-{[(1R,2R)-2-(Benzyloxy)-1-methylpropyl]oxy}-5-(trifluormethyl) pyrimidin-2-yl]amino}phenyl)-S-cyclopropyl-N-(trifluoracetyl)sulfoximid in 66 ml Ethanol wurde mit 1,64 g Palladium auf Kohle (10%ig) versetzt und unter Normaldruck bei Raumtemperatur hydriert. Der Ansatz wurde filtriert und eingeengt. Man erhielt 0,88 g (1,67 mmol; Ausbeute: 79 %) des Produktes.
¹H-NMR (400 MHz, DMSO): δ = 10.65 (s, 1 H), 8.58 (s, 1 H), 8.04 (m, 2H), 7.89 (m, 2H), 5.28 (m, 1 H), 4.86 (d, 1 H), 3.82 (m, 1 H), 3.35 (m, 1 H), 1.45 (m, 5H), 1.15 (m, 5H).

### 1b) Herstellung des Endproduktes

863 mg (1,64 mmol) (RS)-S-Cyclopropyl-S-(4-{[4-{[(1R,2R)-2-hydroxy-1-methylpropyl]oxy}-5-(trifluormethyl)pyrimidin-2-yl]amino}phenyl)-N-(trifluoracetyl)sulfoximid in 35 ml Methanol wurden mit 1130 mg (8,20 mmol) Kaliumcarbonat versetzt und 1,5 Stunden bei Raumtemperatur gerührt. Man verdünnte mit gesättigter Natriumchlorid-Lösung und extrahierte mit Essigester (3x). Die vereinten organischen Phasen wurden getrocknet (Na₂SO₄), filtriert und eingeengt. Man erhielt 709 mg (1,64 mmol) des Rohproduktes.
¹H-NMR (400 MHz, DMSO): δ = 10.50 (s, 1 H), 8.59 (s, 1 H), 7.94 (m, 2H), 7.84 (m, 2H), 5.32 (m, 1 H), 4.91 (d, 1 H), 4.07 (s, 1 H), 3.86 (m, 1 H), 2.63 (m, 1 H), 1.30 (d, 3H), 1.11 (m, 4H), 0.91 (m, 3H).
MS: 431 (ES+).

Das Diastereomerengemisch wurde mittels präparativer HPLC in die reinen Stereoisomere aufgetrennt:

| | |
|---|---|
| Säule: | Chiralpak IA 5µ 250x30 mm |
| Eluenten: | Hexan / Ethanol 8:2 |
| Fluß: | 40,0 mL/min |
| Detektor: | UV 254 nm |
| Temperatur: | Raumtemperatur |
| Retentionszeit: | 10,8-13,4 min; Stereoisomer 1 (=Beispiel 1-SI-1) |
| | 13,6-18,5 min; Stereoisomer 2 (=Beispiel 1-SI-2) |

### Beispiel 2

### (RS)-S-(4-{[4-{[(1R,2R)-2-Hydroxy-1-methylpropyl]oxy}-5-(trifluormethyl)pyrimidin-2-yl]amino}phenyl)-S-methylsulfoximid

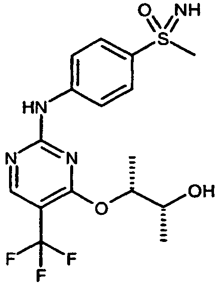

### 2a) Herstellung der Zwischenprodukte

### Verbindung 2.1

### (RS)-S-(4-Nitrophenyl)-S-methylsulfoximid

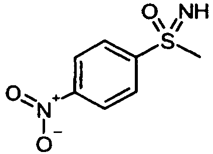

1,56 g (8,42 mmol) 1-(Methylsulfinyl)-4-nitrobenzol in 20 ml DCM wurden mit 0,70 g (10,76 mmol) Natriumazid versetzt. Der Ansatz wurde bei 0 °C langsam mit 2,3 ml konzentrierter Schwefelsäure versetzt und anschließend auf 45 °C erwärmt. Nach 16 h wurde der Ansatz auf Raumtemperatur abgekühlt, mit Wasser versetzt und gegen DCM extrahiert. Die wässrige Phase wurde mit 15%iger Natriumhydroxid-Lösung auf pH 11 gestellt und gegen DCM extrahiert (2x). Die vereinten organischen Phasen wurden getrocknet (Na₂SO₄), filtriert und eingeengt. Man erhielt 1,08 g (5,39 mmol; Ausbeute: 63 %) des Produktes.
¹H-NMR (400 MHz, DMSO): δ = 8.43 (m, 2H), 8.17 (m, 2H), 4.62 (s, 1 H), 3.18 (s, 3H).

### Verbindung 2.2

### (RS)-S-Methyl-S-(4-nitrophenyl)-N-(trifluoracetyl)sulfoximid

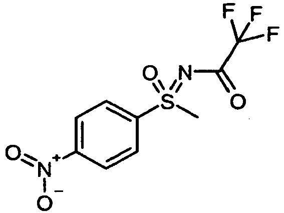

Eine Lösung von 1000 mg (4,99 mmol) (RS)-S-(4-Nitrophenyl)-S-methylsulfoximid, 55 mg (0,45 mmol) DMAP und 0,76 ml (5,49 mmol) Triethylamin in 32 ml DCM wurde unter Eiskühlung tropfenweise mit 1,00 ml (7,08 mmol) Trifluoracetanhydrid versetzt. Der Ansatz wurde weitere 2 Stunden im Eisbad gerührt. Man verdünnte mit DCM und wusch mit halbkonzentrierter Natriumchlorid-Lösung. Die organische Phase wurde getrocknet (Na₂SO₄), filtriert und eingeengt. Der erhaltene Rückstand wurde chromatographisch (Hexan / Essigester 60:40) gereinigt. Das erhaltene Produkt wurde abschließend mit Diisopropylether verrührt. Der Feststoff wurde abgesaugt und getrocknet. Man erhielt 1444 mg (4,87 mmol; Ausbeute: 98%) des Produktes.
¹H-NMR (400 MHz, DMSO): δ = 8.50 (m, 2H), 8.24 (m, 2H), 3.87 (s, 3H).

### Verbindung 2.3

### (RS)-S-(4-Aminophenyl)-S-methyl-N-(trifluoracetyl)sulfoximid

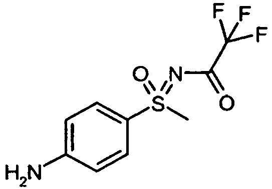

Eine Lösung von 1,34 g (4,52 mmol) (RS)-S-Methyl-S-(4-nitrophenyl)-N-(trifluoracetyl)sulfoximid in 45 ml Ethanol und 8 ml THF wurde mit 292 mg Palladium auf Kohle (10%ig / 50% wasserfeucht) versetzt und 45 Minuten unter Normaldruck bei 24 °C hydriert. Der Ansatz wurde filtriert und eingeengt. Der erhaltene Rückstand wurde mit Diisopropylether verrührt. Der Feststoff wurde abgesaugt und getrocknet. Man erhielt 1,07 g (4,03 mmol; Ausbeute: 89 %) des Produktes.
¹H-NMR (400 MHz, DMSO): δ = 7.54 (m, 2H), 6.67 (m, 2H), 6.35 (s, 2H), 3.55 (s, 3H).

### Verbindung 2.4

### (RS)-S-(4-{[4-{[(1R,2R)-2-(Benzyloxy)-1-methylpropyl]oxy}-5-(trifluormethyl) pyrimidin-2-yl]amino}phenyl)-S-methyl-N-(trifluoracetyl)sulfoximid

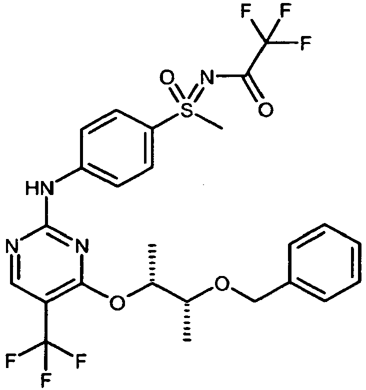

1,40 g (3,88 mmol) 4-((1R,2R)-2-Benzyloxy-1-methyl-propoxy)-2-chlor-5-trifluormethyl-pyrimidin und 1,20 g (4,51 mmol) (RS)-S-(4-Aminophenyl)-S-methyl-N-(trifluoracetyl)sulfoximid in 19,0 ml Acetonitril wurden mit 0,97 ml einer 4N Lösung von Chlorwasserstoff in Dioxan versetzt und 6 Stunden bei 80°C gerührt. Nach dem Abkühlen wurde der Ansatz mit Essigester verdünnt und mit gesättigter Natriumhydrogencarbonat-Lösung und gesättigter Natriumchlorid-Lösung gewaschen, getrocknet (Na₂SO₄), filtriert und eingeengt. Der erhaltene Rückstand wurde chromatographisch (Hexan / Essigester 1:1) gereinigt. Man erhielt 1,76 g (2,98 mmol, Ausbeute: 77%) des Produktes.
¹H-NMR (400 MHz, DMSO): δ = 10.66 (s, 1 H), 8.60 (s, 1 H), 8.02 (m, 2H), 7.93 (m, 2H), 7.21 (m, 5H), 5.46 (m, 1H), 4.57 (d, 1 H), 4.46 (d, 1 H), 3.72 (m, 1 H), 3.68 (s, 3H), 1.31 (d, 3H), 1.16 (d, 3H).

### Verbindung 2.5

### (RS)-S-(4-{[4-{[(1R,2R)-2-Hydroxy-1-methylpropyl]oxy}-5-(trifluormethyl) pyrimidin-2-yl]amino}phenyl)-S-methyl-N-(trifluoracetyl)sulfoximid

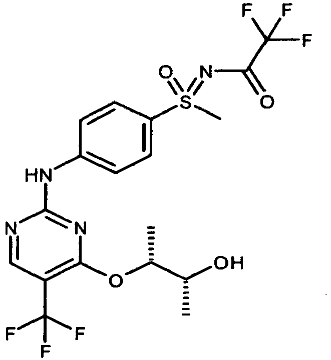

Eine Lösung von 1,75 g (2,96 mmol) (RS)-S-(4-{[4-{[(1R,2R)-2-(Benzyloxy)-1-methylpropyl]oxy}-5-(trifluormethyl)pyrimidin-2-yl]amino}phenyl)-S-methyl-N-(trifluoracetyl)sulfoximid in 35 ml Ethanol wurde mit 0,18 g Palladium auf Kohle (10%ig) versetzt und unter Normaldruck bei Raumtemperatur hydriert. Der Ansatz wurde filtriert und eingeengt. Man erhielt 1,40 g des Rohproduktes.
¹H-NMR (400 MHz, DMSO): δ = 10.65 (s, 1 H), 8.58 (s, 1 H), 8.04 (m, 2H), 7.93 (m, 2H), 5.28 (m, 1 H), 4.86 (d, 1 H), 3.83 (m, 1 H), 3.70 (s, 3H), 1.26 (d, 3H), 1.07 (d, 3H).

### 2b) Herstellung des Endproduktes

1,39 g (2,78 mmol) (RS)-S-(4-{[4-{[(1R,2R)-2-Hydroxy-1-methylpropyl]oxy}-5-(trifluormethyl) pyrimidin-2-yl]amino}phenyl)-S-methyl-N-(trifluoracetyl)sulfoximid in 60 ml Methanol wurden mit 1,92 g (13,89 mmol) Kaliumcarbonat versetzt und 1,5 Stunden bei Raumtemperatur gerührt. Man verdünnte mit gesättigter Natriumchlorid-Lösung und extrahierte mit Essigester (2x). Die vereinten organischen Phasen wurden getrocknet (Na₂SO₄), filtriert und eingeengt. Der Rückstand wurde chromatographisch (DCM / EtOH 9:1) gereinigt. Man erhielt 862 mg (2,13 mmol; Ausbeute: 77%) des Produktes.
¹H-NMR (400 MHz, DMSO): δ = 10.47 (s, 1 H), 8.55 (s, 1 H), 7.90 (m, 2H), 7.83 (m, 2H), 5.27 (m, 1 H), 4.86 (d, 1H), 4.04 (s, 1 H), 3.82 (m, 1 H), 3.00 (s, 3H), 1.26 (d, 3H), 1.07 (d, 3H).

Das Diastereomerengemisch wurde mittels präparativer HPLC in die reinen Stereoisomere aufgetrennt:

| | |
|---|---|
| Säule: | Chiralpak IC 5µ 250x20 mm |
| Eluenten: | Hexan / Ethanol 8:2 |
| Puffer: | Hexan/ 0.1% DEA |
| Fluß: | 25,0 mUmin |
| Detektor: | UV 280 nm |
| Temperatur: | Raumtemperatur |
| Retentionszeit: | 9,5-12,1 min; Stereoisomer 1 (=Beispiel 2-SI-1) |
| | 13,1-16,0 min; Stereoisomer 2 (=Beispiel 2-SI-2) |

### Beispiel 3

### (RS)-S-(4-{[4-{[(R)-2-Hydroxy-1,2-dimethylpropyl]oxy}-5-(trifluormethyl)pyrimidin-2-yl]amino}phenyl)-S-methylsulfoximid

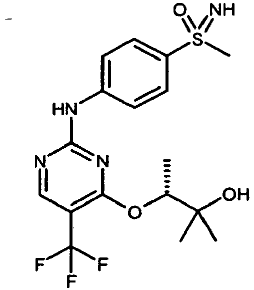

### 3a) Herstellung der Zwischenprodukte

### Verbindung 3.1.

### (R)-2-Methyl-butan-2,3-diol

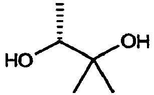

Eine Lösung von 10,0 g (96,1 mmol) (R)-(+)-Milchsäuremethylester in 20 ml THF wurde langsam zu 160 ml (480,0 mmol) einer eisgekühlten 3 N Lösung von Methylmagnesiumchlorid in THF getropft. Der Ansatz wurde zunächst langsam auf Raumtemperatur erwärmt und anschließend 30 Minuten refluxiert. Nach dem Abkühlen wurde der Ansatz auf eine gesättigte Ammoniumchlorid-Lösung gegeben und mit Essigester extrahiert (3x). Die vereinten organischen Phasen wurden über einem Whatman Filter filtriert und eingeengt. Man erhielt 4,5 g (43,1 mmol) des Rohproduktes, das ohne weitere Reinigung eingesetzt wurde.
¹H-NMR (400 MHz, DMSO): δ = 4.21 (d, 1 H), 3.93 (s, 1 H), 3.29 (m, 1 H), 0.97 (m, 9H).

### Verbindung 3.2.

### (R)-3-(2-Chlor-5-jod-pyrimidin-4-yloxy)-2-methyl-butan-2-ol

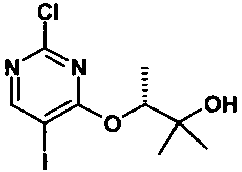

Eine Lösung von 4,40 g (42,3 mmol) (R)-2-Methyl-butan-2,3-diol in 83 ml Diethylether wurde unter Rühren bei 0°C portionsweise mit 1,84 g (42,3 mmol) Natriumhydrid (55%) versetzt und 10 Minuten gerührt. Man rührte weitere 3 Minuten bei Raumtemperatur und gab den Ansatz anschließend zu einer eisgekühlten Lösung von 9,68 g (35,2 mmol) 2,4-Dichlor-5-jod-pyrimidin in 97 ml Acetonitril. Der Ansatz wurde 4 Stunden bei 40°C gerührt und nach dem Erkalten mit Eis und gesättigter NaCl-Lösung versetzt. Man extrahierte mit Essigester (3x). Die vereinten organischen Phasen wurden getrocknet (Na₂SO₄), filtriert und eingeengt. Der erhaltene Rückstand wurde chromatographisch (Hexan / Essigester 4:1) gereinigt. Man erhielt 4,96 g (14,5 mmol; Ausbeute: 41 %) des Produktes.
¹H-NMR (400 MHz, DMSO): δ = 8.73 (s, 1 H), 4.96 (q, 1 H), 4.62 (s, 1 H), 1.21 (d, 3H), 1.13 (s, 6H).
ES: 343 (CI+).

### Verbindung 3.3.

### 2-Chlor-4-[(R)-1,2-dimethyl-2-(tetrahydro-pyran-2-yloxy)-propoxy]-5-jod-pyrimidin

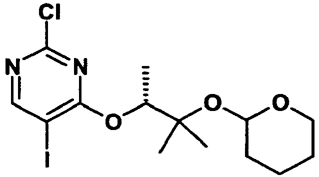

Eine Lösung von 4,96 g (14,5 mmol) (R)-3-(2-Chlor-5-jod-pyrimidin-4-yloxy)-2-methyl-butan-2-ol in 30 ml DCM wurde mit 2,64 ml (29,0 mmol) Dihydropyran und 0,36 g (1,5 mmol) Pyridiniumtosylat versetzt und 22 Stunden bei Raumtemperatur gerührt. Der Ansatz wurde mit DCM verdünnt und mit gesättigter Natriumhydrogencarbonat-Lösung gewaschen. Die organische Phase wurde getrocknet (Na₂SO₄), filtriert und eingeengt. Der erhaltene Rückstand wurde chromatographisch (Hexan / Essigester 4:1) gereinigt. Man erhielt 5,50 g (12,9 mmol; Ausbeute: 89%) des Diastereomerengemisches.
¹H-NMR (400 MHz, DMSO): δ = 8.75 (s, 1 H), 8.74 (s, 1 H), 5.15 (m, 2H), 4.91 (m, 2H), 3.70 (m, 2H), 3.30 (m, 2H), 1.31 (m, 30H).

### Verbindung 3.4.

### 2-Chlor-4-[(R)-1,2-dimethyl-2-(tetrahydro-pyran-2-yloxy)-propoxyl-5-trifluormethyl-pyrimidin

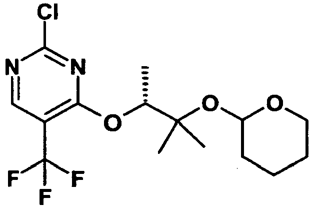

Eine Lösung von 1,00 g (2,34 mmol) 2-Chlor-4-[(R)-1,2-dimethyl-2-(tetrahydro-pyran-2-yloxy)-propoxy]-5-jod-pyrimidin in 3,3 ml NMP und 3,3 ml THF wurde bei Raumtemperatur mit 1,61 g (8,44 mmol) Kupfer(I)jodid, 0,41 g (7,03 mmol) Kaliumfluorid und 1,04 ml (7,03 mmol) (Trifluormethyl)-trimethylsilan versetzt. Der Ansatz wurde 2 Stunden bei 90°C gerührt. Nach dem Erkalten wurde der Ansatz in verdünnte Natriumchlorid-Lösung gegeben und mit Essigester extrahiert (3x). Die vereinten organischen Phasen wurden getrocknet (Na₂SO₄), filtriert und eingeengt. Der erhaltene Rückstand wurde chromatographisch (Hexan / Essigester 4:1) gereinigt. Man erhielt 0,53 g (1,43 mmol; Ausbeute: 61 %) des Produktes.
¹H-NMR (400 MHz, DMSO): δ = 8.84 (s, 1 H), 5.32 (m, 1 H), 4.85 (m, 1 H), 3.68 (m, 1 H), 3.30 (m, 1 H), 1.31 (m, 15H).

### 3b) Herstellung des Endproduktes

200 mg (0,54 mmol) 2-Chlor-4-[(R)-1,2-dimethyl-2-(tetrahydro-pyran-2-yloxy)-propoxy]-5-trifluormethyl-pyrimidin und 87 mg (0,33 mmol) (RS)-S-(4-Aminophenyl)-S-methyl-N-(trifluoracetyl)sulfoximid in 5 ml Ethanol wurden 6 Stunden bei 70°C gerührt. Der Ansatz wurde zur Trockene einrotiert und der Rückstand in 11,6 ml Methanol aufgenommen. Die Lösung wurde mit 373 mg (2,70 mmol) Kaliumcarbonat versetzt und 1,5 Stunden bei Raumtemperatur gerührt. Man verdünnte mit gesättigter Natriumchlorid-Lösung und extrahierte mit Essigester (2x). Die vereinten organischen Phasen wurden getrocknet (Na₂SO₄), filtriert und eingeengt. Der Rückstand wurde mittels HPLC gereinigt. Man erhielt 31 mg (0,07 mmol; Ausbeute: 14%) des Produktes.

| | | | |
|---|---|---|---|
| Säule: | XBridge C18 5µ 100x30 mm | | |
| Eluent A: | H₂O / 0.1% HCOOH | | |
| Eluent B: | Acetonitril | | |
| Gradient: | 0 min | 70%A | 30%B |
| | 1.00 min | 70%A | 30%B |
| | 7.50 min | 40%A | 60%B |
| | 7.52 min | 1%A | 99%B |
| | 10.00 min | 1%A | 99%B |
| Fluß: | 50.0 mL/min | | |
| Detektion: | DAD scan range 210-400 nm; | | |
| | MS ESI+, ESI-, scan range 160-1000 m/z | | |
| Temperatur: | Rt | | |

¹H-NMR (400 MHz, DMSO): δ = 10.48 (s, 1 H), 8.56 (s, 1 H), 7.90 (m, 2H), 7.83 (m, 2H), 5.13 (q, 1 H), 4.67 (s, 1 H), 4.06 (s, 1 H), 3.01 (s, 3H), 1.28 (d, 3H), 1.12 (m, 6H).

### Herstellung der Verbindungen der allgemeinen Formel (Ib) (4-N Derivate)

Die erfindungsgemäßen Verbindungen können hergestellt werden durch ein Verfahren, das durch folgende Schritte gekennzeichnet ist:
a) Oxidation einer Verbindung der Formel **(IVd)** zum Sulfoxid der Formel **(IVc).** 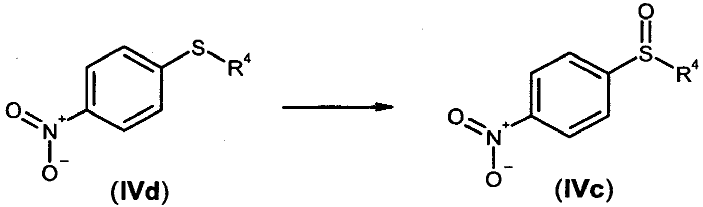
b₁) Direkte Iminierung des Sulfoxides der Formel **(IVc)** zu einem geschützten Sulfoximin der Formel **(IVa).** 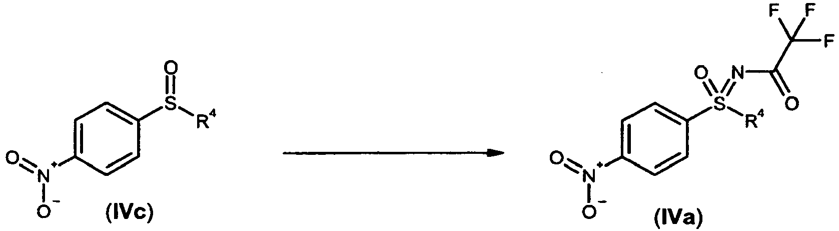 oder
b₂) Iminierung des Sulfoxides der Formel **(IVc)** zu einem ungeschützten Sulfoximin der Formel **(IVb)** und anschließende Einführung der Schutzgruppe zu einer Verbindung der Formel **(IVa).** 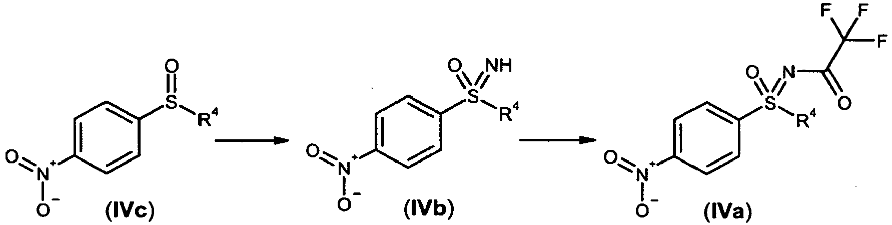
c) Reduktion der Verbindung der Formel **(IVa)** zu einer Verbindung der Formel **(IV)** 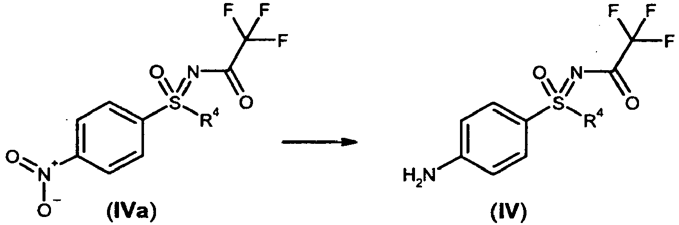
d) Funktionalisierung der 4-Position von 2,4-Dichlor-5-trifluormethyl-pyrimidin **(VIIb)** durch Umsetzung mit einem Amin der Formel **(VIa)** unter Bildung eines Intermediates der Formel **(Vb).** 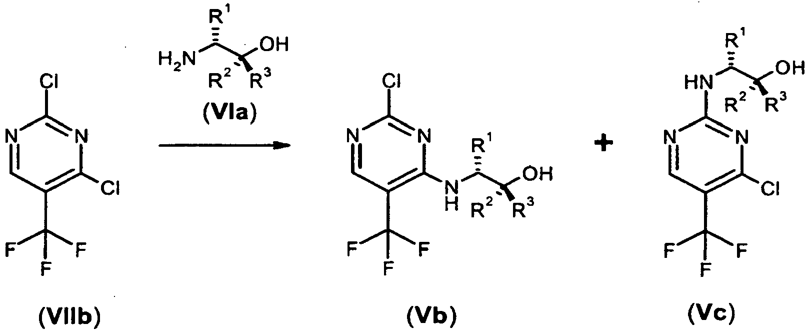
e) Kupplung der Verbindungen der Formel **(Vb)** und **(IV)** zum Intermediat der Formel **(IIb).** 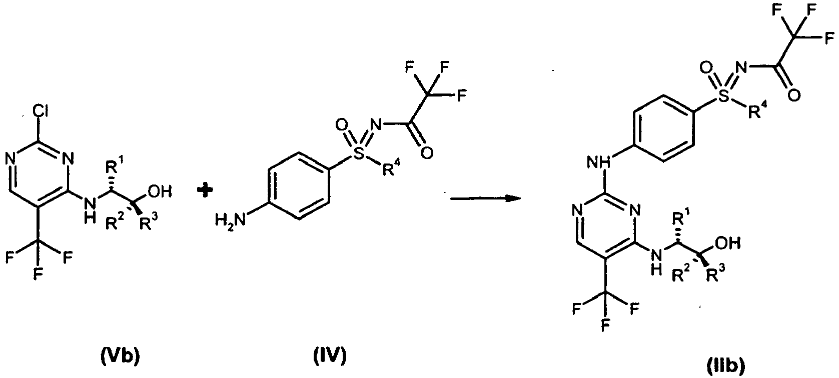
f) Abspaltung der Schutzgruppe am Sulfoximin unter Bildung von **(Ib).** 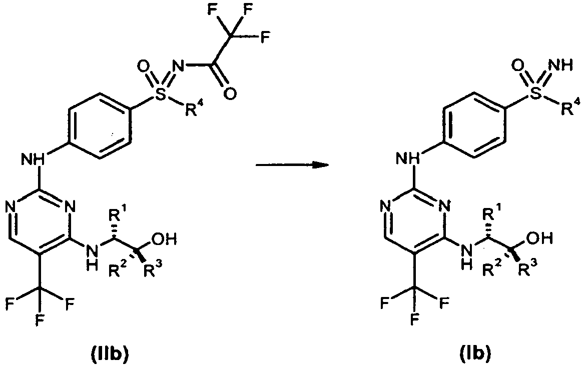
wobei die Substituenten R¹, R², R³ und R⁴ die in der allgemeinen Formel (I) angegebenen Bedeutungen aufweisen.

### Verfahrensschritte a)-c)

Diese Verfahrensschritte sind identisch zu den Verfahrensschritten a)-d) zur Herstellung von Verbindungen gemäß allgemeiner Formel (Ia).

### Verfahrensschritt d)

Die Umsetzung von 2,4-Dichlor-5-trifluormethyl-pyrimidin **(VIIb)** mit einem Amin der Formel **(VIa)** liefert ein Gemisch der Produkte **(Vb)** und **(Vc).** Das gewünschte Produkt (Vb) kann z.B. chromatographisch abgetrennt werden (siehe z.B.: (a) J. Bryant et al, WO 2004/048343).

### Verfahrensschritt e)

Ein 2-Chlor-pyrimidin der Formel **(Vb)** kann mit einem Anilin der Formel **(IV)** zu einem Intermediat der Formel **(IIb)** umgesetzt werden (siehe z.B.: (a) J. Bryant et al, WO 2004/048343).

### Verfahrensschritt f)

Die Abspaltung der Trifluoracetogruppe am Sulfoximin **(IIb)** liefert die Verbindung der Formel **(Ib).** Besonders geeignet ist hierfür die beschriebene Methodik unter Verwendung von Kaliumcarbonat in Methanol bei Raumtemperatur.

### Beispiel 4

### (RS)-S-Cyclopropyl-S-(4-{[4-{[(1R,2R)-2-hydroxy-1-methylpropyl]amino} -5-(trifluormethyl)pyrimidin-2-yl]amino}phenyl)sulfoximid

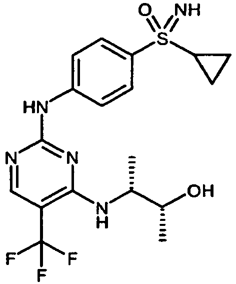

### 4a) Herstellung der Zwischenprodukte

### Verbindung 4.1

### (2R,3R)-3-(2-Chlor-5-trifluormethyl-pyrimidin-4-ylamino)-butan-2-ol

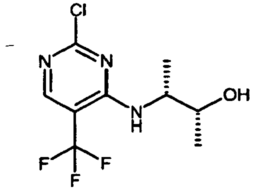

56,5 g (260,35 mmol) 2,4-Dichlor-5-trifluormethyl-pyrimidin und 32,7 g (260,35 mmol) (2R,3R)-3-Amino-butan-2-ol Hydrochlorid in 1035 ml Acetonitril wurden bei 0°C tropfenweise mit 72,2 ml (520,71 mmol) Triethylamin versetzt. Der Ansatz wurde über Nacht langsam auf Raumtemperatur erwärmt. Der Ansatz wurde in halbkonzentrierte Natriumchlorid-Lösung gegeben und mit Essigester extrahiert (2x). Die vereinten organischen Phasen wurden getrocknet (Na₂SO₄), filtriert und eingeengt. Der verbleibende Rückstand wurde chromatographisch (Hexan / Essigester 0-100%) gereinigt. Man erhielt 18,6 g (68,97 mmol; Ausbeute: 27 %) des Produktes.
¹H-NMR (400 MHz, DMSO): δ = 8.38 (s, 1 H), 6.71 (d, 1 H), 5.00 (d, 1 H), 4.08 (m, 1 H), 3.71 (m, 1 H), 1.12 (d, 3H), 1.01 (d, 3H).

Die Herstellung von (RS)-S-(4-Aminophenyl)-S-cyclopropyl-N-(trifluoracetyl)sulfoximid ist beschrieben als Verbindung 1.4.

### 4b) Herstellung des Endproduktes

250 mg (0,86 mmol) (RS)-S-(4-Aminophenyl)-S-cyclopropyl-N-(trifluoracetyl)sulfoximid und 231 mg (0,86 mmol) (2R,3R)-3-(2-Chlor-5-trifluormethyl-pyrimidin-4-ylamino)-butan-2-ol in 3,75 ml Acetonitril wurden mit 0,21 ml einer 4N Lösung von Chlorwasserstoff in Dioxan versetzt und 3,5 Stunden bei 60°C gerührt. Der Ansatz wurde zur Trockene eingeengt. Man versetzte mit 18,4 ml Methanol und 590 mg (4,28 mmol) Kaliumcarbonat und rührte eine Stunde bei Raumtemperatur. Man verdünnte mit gesättigter Natriumchlorid-Lösung und extrahierte mit Essigester (2x). Die vereinten organischen Phasen wurden getrocknet (Na₂SO₄), filtriert und eingeengt. Der verbleibende Rückstand wurde chromatographisch (DCM / MeOH 4:1) gereinigt. Man erhielt 242 mg (0,56 mmol; Ausbeute: 65%) des Produktes.
¹H-NMR (400 MHz, DMSO): δ = 10.04 (s, 1 H), 8.25 (s, 1 H), 7.91 (m, 2H), 7.74 (m, 2H), 6.05 (d, 1 H), 5.07 (d, 1 H), 4.14 (m, 1 H), 3.97 (s, 1 H), 3.77 (m, 1 H), 2.56 (m, 1 H), 1.19 (d, 3H), 1.05 (m, 4H), 0.86 (m, 3H).
MS: 430 (ESI+).

Das Diastereomerengemisch wurde mittels präparativer HPLC in die reinen Stereoisomere aufgetrennt:

| | |
|---|---|
| Säule: | Chiralpak IA 5µ 250x20 mm |
| Eluenten: | Hexan / 2-Propanol 50:50 |
| Puffer: | Hexan/ 0,1% DEA |
| Fluß: | 15,0 mUmin |
| Detektor: | UV 254 nm |
| Temperatur: | Raumtemperatur |
| Retentionszeit: | 5,9-6,6 min; Stereoisomer 1 (=Beispiel 4-SI-1) |
| | 7,1-8,8 min; Stereoisomer 2 (=Beispiel 4-SI-2) |

### Beispiel 5

### (RS)-S-Cyclopropyl-S-(4-{[4-{[(R)-2-hydroxy-1,2-dimethylpropyl]amino} -5-(trifluormethyl)pyrimidin-2-yl]amino}phenyl)sulfoximid

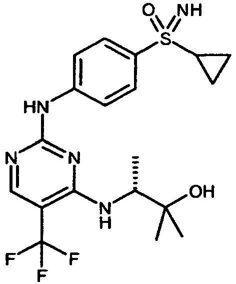

### 5a) Herstellung der Zwischenprodukte

### Verbindung 5.1

### (R)-3-(2-Chlor-5-trifluormethyl-pyrimidin-4-ylamino)-2-methyl-butan-2-ol

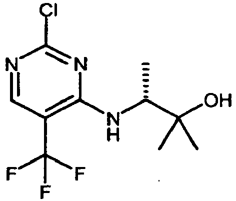

3,6 g (35,03 mmol) (R)-3-Amino-2-methyl-butan-2-ol wurden tropfenweise zu einer Lösung von 7,6 g (35,03 mmol) 2,4-Dichlor-5-trifluormethyl-pyrimidin in 139 ml Acetonitril gegeben. Bei 0 °C wurden nun 9,7 ml (70,1 mmol) Triethylamin zugetropft und der Ansatz langsam über Nacht auf Raumtemperatur erwärmt. Man rührte weitere 2 Tage bei Raumtemperatur. Der Ansatz wurde in halbkonzentrierte Natriumchlorid-Lösung gegeben und mit Essigester extrahiert (2x). Die vereinten organischen Phasen wurden getrocknet (Na₂SO₄), filtriert und eingeengt. Der verbleibende Rückstand wurde mittels präparativer HPLC gereinigt. Man erhielt 3,0 g (10,65 mmol; Ausbeute: 30 %) des Produktes.

| | |
|---|---|
| Säule: | XBridge C18 5µ 150x20 mm |
| Eluent A: | H₂O / 0.2% NH₃ |
| Eluent B: | Acetonitril |
| Gradient: | 70%A+30%B(2') 30->60%B(10') 60->99%B(0.1') |
| Fluß: | 50,0 mL/min |
| Detektor: | DAD (200-400 nm) TAC ; MS-ESI+ (160-1000 m/z) TIC |
| Temperatur: | Raumtemperatur |
| Retentionszeit: | 5,6-6,4 min |

¹H-NMR (400 MHz, DMSO): δ = 8.42 (s, 1 H), 6.52 (d, 1 H), 5.01 (s, 1 H), 4.10 (m, 1 H), 1.11 (m, 9H).

Die Herstellung von (RS)-S-(4-Aminophenyl)-S-cyclopropyl-N-(trifluoracetyl)sulfoximid ist beschrieben als Verbindung 1.4.

### 5b) Herstellung des Endproduktes

400 mg (1,37 mmol) (RS)-S-(4-Aminophenyl)-S-cyclopropyl-N-(trifluoracetyl)sulfoximid und 388 mg (1,37 mmol) (R)-3-(2-Chlor-5-trifluormethyl-pyrimidin-4-ylamino)-2-methyl-butan-2-ol in 6,0 ml Acetonitril wurden mit 0,34 ml einer 4N Lösung von Chlorwasserstoff in Dioxan versetzt und 3,5 Stunden bei 60°C gerührt. Der Ansatz wurde zur Trockene eingeengt. Man versetzte mit 29,4 ml Methanol und 950 mg (6,84 mmol) Kaliumcarbonat und rührte eine Stunde bei Raumtemperatur. Man verdünnte mit gesättigter Natriumchlorid-Lösung und extrahierte mit Essigester (2x). Die vereinten organischen Phasen wurden getrocknet (Na₂SO₄), filtriert und eingeengt. Man erhielt 600 mg (1,35 mmol) des Rohproduktes.
¹H-NMR (400 MHz, DMSO): δ = 10.08 (s, 1 H), 8.30 (s, 1 H), 7.94 (m, 2H), 7.80 (m, 2H), 6.07 (d, 1 H), 4.95 (s, 1 H), 4.16 (m, 1 H), 4.02 (s, 1 H), 2.62 (m, 1 H), 1.20 (m, 6H), 1.10 (m, 4H), 0.89 (m, 3H).
MS: 444 (ESI+).

Das Diastereomerengemisch wurde mittels präparativer HPLC in die reinen Stereoisomere aufgetrennt:

| | |
|---|---|
| Säule: | Chiralpak AD-H 5µ 250x20 mm |
| Eluenten: | Hexan / 2-Propanol 60:40 |
| Puffer: | Hexan/ 0,1% DEA |
| Fluß: | 20,0 mL/min |
| Detektor: | UV 280 nm |
| Temperatur: | Raumtemperatur |
| Retentionszeit: | 5,1-6,3 min; Stereoisomer 1 (=Beispiel 5-SI-1) |
| | 8,0-10,8 min; Stereoisomer 2 (=Beispiel 5-SI-2) |

### Beispiel 6

### (RS)-S-Ethyl-S-(4-{[4-{[(1R,2R)-2-hydroxy-1-methylpropyl]amino} -5-(trifluormethyl)pyrimidin-2-yl]amino}phenyl)sulfoximid

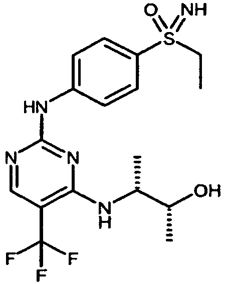

### 6a) Herstellung der Zwischenprodukte

### Verbindung 6.1

### 1-Ethylsulfanyl-4-nitro-benzol

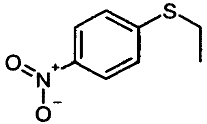

Eine Lösung von 4,27 g (106,76 mmol) Natriumhydroxid in 320 ml Ethanol wurde unter Wasserkühlung mit 16,56 g (106,72 mmol) 4-Nitrothiophenol versetzt und 15 Minuten bei Raumtemperatur gerührt. Anschießend wurden unter Wasserkühlung 8,63 ml (106,79 mmol) Ethyljodid zugegeben und der Ansatz über Nacht bei Raumtemperatur gerührt. Der Ansatz wurde in gesättigte Natriumchlorid-Lösung gegeben und mit Essigester extrahiert (2x). Die vereinten organischen Phasen wurden getrocknet (Na₂SO₄), filtriert und eingeengt. Anschließend wurde in DCM gelöst und erneut filtriert und zur Trockene eingeengt. Man erhielt 16,86 g (92,02 mmol) des Rohproduktes.
¹H-NMR (400 MHz, DMSO): δ = 8.14 (m, 2H), 7.49 (m, 2H), 3.14 (q, 2H), 1.31 (tr, 3H).

### Verbindung 6.2

### (RS)-1-Ethylsulfinyl-4-nitro-benzol

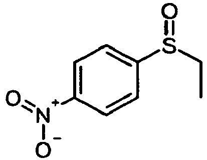

Ein Ansatz mit 16,86 g (92,02 mmol) 1-Ethylsulfanyl-4-nitro-benzol in 75 ml Acetonitril wurde mit 428 mg (2,64 mmol) Eisen(III) chlorid versetzt und 10 Minuten bei Raumtemperatur gerührt. Anschließend versetzte man portionsweise mit 22,44 g (98,44 mmol) Perjodsäure, so dass die Temperatur 30°C nicht überschritt. Der Ansatz wurde 50 Minuten gerührt und anschließend unter Rühren in ein Gemisch aus 170 ml DCM, 500 ml Eiswasser und 100 g Natriumthiosulfat Pentahydrat gegeben. Man extrahierte mit DCM (2x). Die vereinten organischen Phasen wurden getrocknet (Na₂SO₄), filtriert und eingeengt. Der erhaltene Rückstand wurde aus Essigester / Hexan umkristallisiert. Man erhielt 12,49 g (62,69 mmol; Ausbeute: 68 %) des Produktes.
¹H-NMR (400 MHz, DMSO): δ = 8.35 (m, 2H), 7.88 (m, 2H), 3.12 (m, 1 H), 2.84 (m, 1 H), 0.99 (tr, 3H).

### Verbindung 6.3

### (RS)-S-Ethyl-S-(4-nitrophenyl)sulfoximid

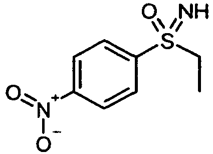

6,00 g (30,12 mmol) (RS)-1-Ethylsulfinyl-4-nitro-benzol wurden im Eisbad vorsichtig mit 30,5 ml Oleum (20% SO₃) versetzt. Anschließend wurden unter Argon vorsichtig 2,35 g (36,14 mmol) Natriumazid portionsweise unter Rühren zugegeben und der Ansatz dann auf 45°C erwärmt. Nach 6 Stunden wurde der Ansatz auf Raumtemperatur abgekühlt und vorsichtig auf Eis gegossen. Der Ansatz wurde mit Natriumhydrogencarbonat basisch gestellt und mit Essigester extrahiert (2x). Die vereinten organischen Phasen wurden getrocknet (Na₂SO₄), filtriert und eingeengt. Man erhielt 5,74 g (26,79 mmol; Ausbeute: 89%) des Produktes.
¹H-NMR (400 MHz, DMSO): δ = 8.37 (m, 2H), 8.09 (m, 2H), 4.56 (s, 1 H), 3.18 (q, 2H), 1.04 (tr, 3H).

### Verbindung 6.4

### (RS)-S-Ethyl-S-(4-nitrophenyl)-N-(trifluoracetyl)sulfoximid

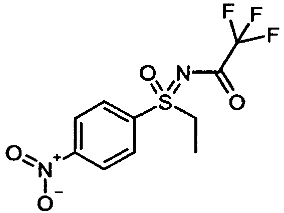

Eine Lösung von 5,72 g (26,70 mmol) (RS)-S-Ethyl-S-(4-nitrophenyl)sulfoximid und 4,07 ml (29,37 mmol) Triethylamin in 175 ml DCM wurde unter Eiskühlung tropfenweise mit 4,53 ml (32,04 mmol) Trifluoracetanhydrid versetzt. Der Ansatz wurde weitere 3 Stunden im Eisbad gerührt, wobei die Temperatur auf ca. 10°C anstieg. Man verdünnte mit DCM und wusch mit halbkonzentrierter Natriumchlorid-Lösung. Die organische Phase wurde getrocknet (Na₂SO₄), filtriert und eingeengt. Man erhielt 8,17 g (26,33 mmol) des Produktes.
¹H-NMR (400 MHz, DMSO): δ = 8.52 (m, 2H), 8.22 (m, 2H), 3.99 (m, 2H), 1.16 (tr, 3H).

### Verbindung 6.5

### (RS)-S-(4-Aminophenyl)-S-ethyl-N-(trifluoracetyl)sulfoximid

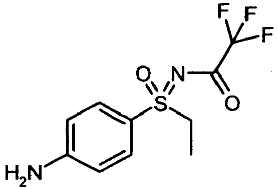

Eine Lösung von 4,05 g (13,05 mmol) (RS)-S-Ethyl-S-(4-nitrophenyl)-N-(trifluoracetyl)sulfoximid in 191 ml THF wurde unter Eiskühlung langsam mit 88,5 ml einer 15%igen Lösung von Titan(III)chlorid in etwa 10%iger Salzsäure versetzt. Der Ansatz wurde 3,5 Stunden bei Raumtemperatur gerührt, mit Essigester verdünnt und anschließend mit halbkonzentrierter Natriumchlorid-Lösung gewaschen (3x). Die organische Phase wurde getrocknet (Na₂SO₄), filtriert und eingeengt. Man erhielt 3,17 g (11,31 mmol) des Produktes.
¹H-NMR (400 MHz, DMSO): δ = 7.48 (m, 2H), 6.68 (m, 2H), 3.64 (m, 2H), 1.06 (tr, 3H).

Die Herstellung von (2R,3R)-3-(2-Chlor-5-trifluormethyl-pyrimidin-4-ylamino)-butan-2-ol ist beschrieben als Verbindung 4.1.

### 6b) Herstellung des Endproduktes

400 mg (1,43 mmol) (RS)-S-(4-Aminophenyl)-S-ethyl-N-(trifluoracetyl)sulfoximid und 385 mg (1,43 mmol) (2R,3R)-3-(2-Chlor-5-trifluormethyl-pyrimidin-4-ylamino)-butan-2-ol in 7,0 ml Acetonitril wurden mit 0,36 ml einer 4N Lösung von Chlorwasserstoff in Dioxan versetzt und 4,5 Stunden bei 60°C gerührt. Der Ansatz wurde zur Trockene eingeengt. Man versetzte mit 19,0 ml Methanol und 608 mg (4,40 mmol) Kaliumcarbonat und rührte 1 Stunde bei Raumtemperatur. Man verdünnte mit gesättigter Natriumchlorid-Lösung und extrahierte mit Essigester (2x). Die vereinten organischen Phasen wurden getrocknet (Na₂SO₄), filtriert und eingeengt. Man erhielt 590 mg (1,41 mmol) des Rohproduktes.
¹H-NMR (400 MHz, DMSO): δ = 10.10 (s, 1 H), 8.30 (s, 1 H), 7.96 (m, 2H), 7.78 (m, 2H), 6.10 (d, 1H), 5.11 (d, 1 H), 4.19 (m, 1 H), 4.00 (s, 1 H), 3.82 (m, 1 H), 3.07 (q, 2H), 1.24 (d, 3H), 1.06 (m, 6H).
MS: 418 (ESI+).

Das Diastereomerengemisch wurde mittels präparativer HPLC in die reinen Stereoisomere aufgetrennt:

| | |
|---|---|
| Säule: | Chiralpak AD-H 5µ 250x20 mm |
| Eluenten: | Hexan / 2-Propanol 60:40 |
| Puffer: | Hexan/ 0,1% DEA |
| Fluß: | 20,0 mL/min |
| Detektor: | UV 280 nm |
| Temperatur: | Raumtemperatur |
| Retentionszeit: | 6,2-6,8 min; Stereoisomer 1 (=Beispiel 6-SI-1) |
| | 7,2-8,9 min; Stereoisomer 2 (=Beispiel 6-SI-2) |

### Beispiel 7

### (RS)-S-Ethyl-S-(4-{[4-{[(R)-2-hydroxy-1,2-dimethylpropyl]amino} -5-(trifluormethyl)pyrimidin-2-yl]amino}phenyl)sulfoximid

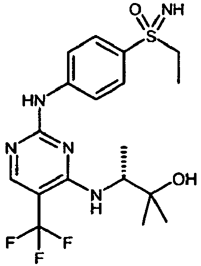

### 7a) Herstellung der Zwischenprodukte

Die Herstellung von (RS)-S-(4-Aminophenyl)-S-ethyl-N-(trifluoracetyl)sulfoximid ist beschrieben als Verbindung 6.5.

Die Herstellung von (R)-3-(2-Chlor-5-trifluormethyl-pyrimidin-4-ylamino)-2-methylbutan-2-ol ist beschrieben als Verbindung 5.1.

### 7b) Herstellung des Endproduktes

400 mg (1,43 mmol) (RS)-S-(4-Aminophenyl)-S-ethyl-N-(trifluoracetyl)sulfoximid und 405 mg (1,43 mmol) (R)-3-(2-Chlor-5-trifluormethyl-pyrimidin-4-ylamino)-2-methylbutan-2-ol in 7,0 ml Acetonitril wurden mit 0,36 ml einer 4N Lösung von Chlorwasserstoff in Dioxan versetzt und 4,5 Stunden bei 60°C gerührt. Der Ansatz wurde zur Trockene eingeengt. Man versetzte mit 25,0 ml Methanol und 788 mg (5,70 mmol) Kaliumcarbonat und rührte eine Stunde bei Raumtemperatur. Man verdünnte mit gesättigter Natriumchlorid-Lösung und extrahierte mit Essigester (2x).

Die vereinten organischen Phasen wurden getrocknet (Na₂SO₄), filtriert und eingeengt. Man erhielt 620 mg (1,43 mmol) des Rohproduktes.
¹H-NMR (400 MHz, DMSO): δ =10.06 (s, 1 H), 8.28 (s, 1 H), 7.92 (m, 2H), 7.74 (m, 2H), 6.03 (d, 1 H), 4.90 (s, 1 H), 4.12 (m, 1 H), 3.96 (s, 1 H), 3.03 (q, 2H), 1.16 (m, 6H), 1.08 (m, 3H), 1.02 (tr, 3H).
MS: 432 (ESI+).

Das Diastereomerengemisch wurde mittels präparativer HPLC in die reinen Stereoisomere aufgetrennt:

| | |
|---|---|
| Säule: | Chiralpak AD-H 5µ 250x20 mm |
| Eluenten: | A:Hexan B:2-Propanol |
| Puffer: | Hexan/ 0,1% DEA |
| Gradient: | 20->40%B(20')+40%B(5') |
| Fluß: | 10,0 mL/min |
| Detektor: | UV 280 nm |
| Temperatur: | Raumtemperatur |
| Retentionszeit: | 17,5-19,8 min; Stereoisomer 1 (=Beispiel 7-SI-1) |
| | 20,1-22,0 min; Stereoisomer 2 (=Beispiel 7-SI-2) |

### Beispiel 8

### (RS)-S-(4-{[4-{[(1R,2R)-2-Hydroxy-1-methylpropyl]amino}-5-(trifluormethyl) pyrimidin-2-yl]amino}phenyl)-S-methylsulfoximid

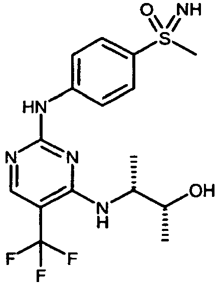

### 8a) Herstellung der Zwischenprodukte

Die Herstellung von (RS)-S-(4-Aminophenyl)-S-methyl-N-(trifluoracetyl)sulfoximid ist beschrieben als Verbindung 2.3.

Die Herstellung von (2R,3R)-3-(2-Chlor-5-trifluormethyl-pyrimidin-4-ylamino)-butan-2-ol ist beschrieben als Verbindung 4.1.

### 8b) Herstellung des Endproduktes

399 mg (1,50 mmol) (RS)-S-(4-Aminophenyl)-S-methyl-N-(trifluoracetyl)sulfoximid und 404 mg (1,50 mmol) (2R,3R)-3-(2-Chlor-5-trifluormethyl-pyrimidin-4-ylamino)-butan-2-ol in 7,3 ml Acetonitril wurden mit 0,38 ml einer 4N Lösung von Chlorwasserstoff in Dioxan versetzt und 9 Stunden bei 60°C gerührt. Der Ansatz wurde zur Trockene eingeengt. Man versetzte mit 32,2 ml Methanol und 1040 mg (7,50 mmol) Kaliumcarbonat und rührte 1,5 Stunden bei Raumtemperatur. Man verdünnte mit gesättigter Natriumchlorid-Lösung und extrahierte mit Essigester (3x). Die vereinten organischen Phasen wurden getrocknet (Na₂SO₄), filtriert und eingeengt. Man erhielt 565 mg (1,40 mmol) des Rohproduktes.
¹H-NMR (400 MHz, DMSO): 10.09 (s, 1 H), 8.30 (s, 1 H), 7.96 (m, 2H), 7.83 (m, 2H), 6.10 (d, 1H), 5.11 (d, 1 H), 4.18 (m, 1 H), 4.03 (s, 1 H), 3.82 (m, 1 H), 3.03 (s, 3H), 1.25 (d, 3H), 1.10 (d, 3H).

Das Diastereomerengemisch wurde mittels präparativer HPLC in die reinen Stereoisomere aufgetrennt:

| | |
|---|---|
| Säule: | Chiralpak IC 5µ 250x20 mm |
| Eluenten: | Hexan / Ethanol 50:50 |
| Puffer: | Hexan/ 0,1% DEA |
| Fluß: | 20,0 mL/min |
| Detektor: | UV 254 nm |
| Temperatur: | Raumtemperatur |
| Retentionszeit: | 5,1-5,8 min; Stereoisomer 1 (=Beispiel 8-SI-1) |
| | 6,1-6,7 min; Stereoisomer 2 (=Beispiel 8-SI-2) |

### Beispiel 9

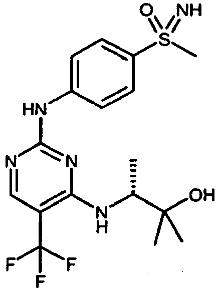

### 9a) Herstellung der Zwischenprodukte

Die Herstellung von (RS)-S-(4-Aminophenyl)-S-methyl-N-(trifluoracetyl)sulfoximid ist beschrieben als Verbindung 2.3.

Die Herstellung von (R)-3-(2-Chlor-5-trifluormethyl-pyrimidin-4-ylamino)-2-methyl-butan-2-ol ist beschrieben als Verbindung 5.1.

### 9b) Herstellung des Endproduktes

399 mg (1,50 mmol) (RS)-S-(4-Aminophenyl)-S-methyl-N-(trifluoracetyl)sulfoximid und 425 mg (1,50 mmol) (R)-3-(2-Chlor-5-trifluormethyl-pyrimidin-4-ylamino)-2-methyl-butan-2-ol in 7,3 ml Acetonitril wurden mit 0,38 ml einer 4N Lösung von Chlorwasserstoff in Dioxan versetzt und 4 Stunden bei 60°C gerührt. Der Ansatz wurde zur Trockene eingeengt. Man versetzte mit 32,2 ml Methanol und 1040 mg (7,50 mmol) Kaliumcarbonat und rührte 1,5 Stunden bei Raumtemperatur. Man verdünnte mit gesättigter Natriumchlorid-Lösung und extrahierte mit Essigester (2x). Die vereinten organischen Phasen wurden getrocknet (Na₂SO₄), filtriert und eingeengt. Man erhielt 600 mg (1,44 mmol) des Rohproduktes.
¹H-NMR (400 MHz, DMSO): δ = 10.05 (s, 1 H), 8.26 (s, 1 H), 7.91 (m, 2H), 7.79 (m, 2H), 6.03 (d, 1 H), 4.91 (s, 1 H), 4.11 (m, 1 H), 3.99 (s, 1 H), 2.99 (s, 3H), 1.16 (m, 6H), 1.10 (m, 3H).
MS: 418 (ESI+).

Das Diastereomerengemisch wurde mittels präparativer HPLC in die reinen Stereoisomere aufgetrennt:

| | |
|---|---|
| Säule: | Chiralpak IC 5µ 250x20 mm |
| Eluenten: | Hexan / Ethanol 80:20 |
| Fluß: | 30,0 mL/min |
| Detektor: | UV 254 nm |
| Temperatur: | Raumtemperatur |
| Retentionszeit: | 6,0-6,7 min; Stereoisomer 1 (=Beispiel 9-SI-1) |
| | 7,1-8,9 min; Stereoisomer 2 (=Beispiel 9-SI-2) |

### Herstellung der Vergleichssubstanzen

Die in-vitro und in-vivo-Effektivität der erfindungsgemäßen Verbindungen gekennzeichnet u.a. durch einen 5-CF₃-Substituenten in Position 5 des Pyrimidins wurden mit ihren 5-Br-Analoga verglichen, die entweder explizit in WO 2005/037800 offenbart sind oder aber unter deren generische Offenbarung fallen.

### V11 = 5-Br-Vergleichsubstanz in Beispiel 11

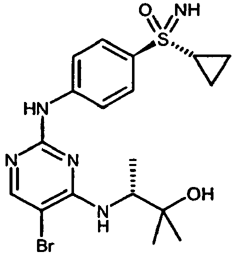

Die Vergleichssubstanz in Beispiel 11 ist das aktivere Stereoisomer des Diastereomerengemisches (RS)-S-[4-({5-Brom-4-[(R)-(2-hydroxy-1,2-dimethylpropyl)amino]pyrimidin-2-yl}amino)phenyl]-S-cyclopropyl-sulfoximid, das als Beispiels 1.6 in der Anmeldung WO 2005/037800 (S. 35), offenbart wird.

Für den Vergleich in-vivo wurde in Beispiel 11 das Diastereomer (R)-S-[4-({5-Brom-4-[(R)-(2-hydroxy-1,2-dimethylpropyl)amino]pyrimidin-2-yl}amino)phenyl]-S-cyclopropyl-sulfoximid, das eine stärkere in vitro Effektivität als (S)-S-[4-({5-Brom-4-[(R)-(2-hydroxy-1,2-dimethylpropyl)amino]pyrimidin-2-yl}amino)phenyl]-S-cyclopropyl-sulfoximid aufweist, verwendet.

V11 wurde dazu nach folgender Methode hergestellt:

### V11a) Herstellung des Zwischenproduktes

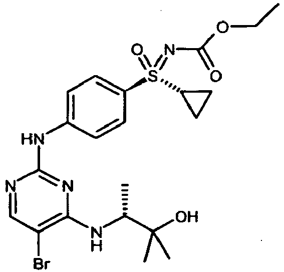

988 mg (3,35 mmol) (R)-3-(5-Brom-2-chlor-pyrimidin-4-ylamino)-2-methyl-butan-2-ol und 750 mg (2,79 mmol) (R)-S-(4-Aminophenyl)-N-(ethoxycarbonyl)-S-cyclopropylsulfoximid (Herstellung nach: U. Lücking et al, WO 2007 / 071455, S. 112, Example 4) in 16,50 ml Butanol und 1,65 ml Methanol wurden mit 0,07 ml einer 4N Lösung von Chlorwasserstoff in Dioxan versetzt und 3 Tage bei 60°C gerührt. Nach dem Abkühlen wurde der Ansatz zur Trockene eingeengt und chromatographisch (DCM / EtOH 9:1) gereinigt. Man erhielt 319 mg (0,61 mmol, Ausbeute: 22%) des Produktes.
¹H-NMR (400 MHz, DMSO): δ = 9.96 (s, 1 H), 8.13 (s, 1 H), 7.92 (m, 2H), 7.73 (m, 2H), 6.28 (d, 1 H), 4.05 (m, 1 H), 3.84 (m, 2H), 2.96 (m, 1 H), 1.05 (m, 16H).

### V11b) Herstellung des Endproduktes

319 mg (0,61 mmol) des Zwischenproduktes in 4,3 ml Ethanol wurden mit 2,0 ml einer frisch hergestellten 1,5M Natriumethanolat-Lösung versetzt und 18 Stunden bei 60°C gerührt. Nach dem Abkühlen wurde der Ansatz in gesättigte Natriumchlorid-Lösung gegeben und mit Essigester extrahiert (3x). Die vereinten organischen Phasen wurden getrocknet (Na₂SO₄), filtriert und eingeengt. Nach abschließender Umkristallisation (DCM / Essigester) erhielt man 215 mg (0,47 mmol; Ausbeute: 78%) des Produktes.
¹H-NMR (400 MHz, DMSO): δ = 9.68 (s, 1H), 8.08 (s, 1 H), 7.86 (m, 2H), 7.70 (m, 2H), 6.07 (d, 1 H), 4.82 (s, 1 H), 4.05 (m, 1H), 3.93 (s, 1 H), 2.55 (m, 1 H), 1.15 (m, 6H), 1.10 (m, 3H), 1.03 (m, 1H), 0.83 (m, 3H).

| | |
|---|---|
| Säule: | Chiralpak AD-H 5µ 150x4,6 mm |
| Eluenten: | Hexan / Ethanol 80.20 |
| Fluß: | 1,0 mL/min |
| Detection: | PDA 280 nm |
| Temperatur: | 25°C |
| Retentionszeit: | 11,64 min |

### V12 = 5-Br-Vergleichssubstanz in Beispiel 12

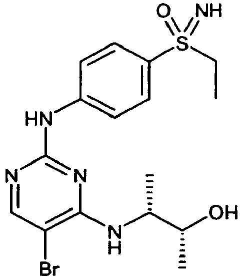

Die Vergleichssubstanz in Beispiel 12 wurde gemäß Beispiel 1.52 der WO 2005/037800 hergestellt. Beispiel 1.52 hat eine stärkere in vitro Effektivität als Beispiel 1.53.

### V13 = 5-Br-Vergleichssubstanz in Beispiel 13

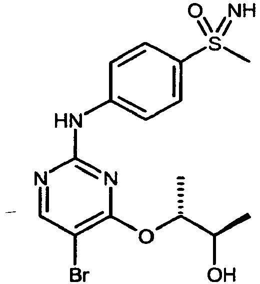

Die Vergleichssubstanz in Beispiel 13 wurde gemäß Beispiel 3.13 der WO 2005/037800 hergestellt. Beispiel 3.13 hat eine stärkere in vitro Effektivität als Beispiel 3.12.

### V14 = 5-Br-Vergleichssubstanz in Beispiel 14

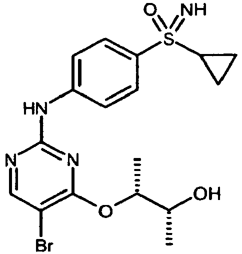

### V14a) Herstellung des Zwischenproduktes

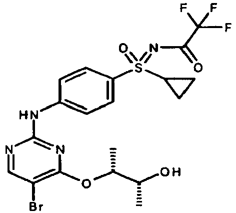

845 mg (3,00 mmol) (2R,3R)-3-(5-Brom-2-chlor-pyrimidin-4-yloxy)-butan-2-ol (Herstellung nach: WO 2005 / 037800, S. 93) und 877 mg (3,00 mmol) (RS)-S-(4-Aminophenyl)-S-cyclopropyl-N-(trifluoracetyl)sulfoximid in 13,1 ml Acetonitril wurden mit 1,5 ml einer 4N Lösung von Chlorwasserstoff in Dioxan versetzt und 5 Stunden bei 80°C gerührt. Man versetzte den Ansatz mit weiteren 422 mg (1,50 mmol) (2R,3R)-3-(5-Brom-2-chlor-pyrimidin-4-yloxy)-butan-2-ol und rührte weiter bei 80°C. Nach 3 Stunden wurde erneut mit 0,75 ml der 4N Lösung von Chlorwasserstoff in Dioxan versetzt und weiter bei 80°C gerührt. Nach 33 Stunden wurde erneut mit 175 mg (0,60 mmol) (RS)-S-(4-Aminophenyl)-S-cyclopropyl-N-(trifluoracetyl)sulfoximid versetzt und abschließend 18 Stunden bei 80°C gerührt.

Nach dem Abkühlen wurde der Ansatz eingeengt und der verbleibende Rückstand chromatographisch gereinigt (DCM / EtOH 9:1). Man erhielt 740 mg (1,38 mmol, Ausbeute: 46%) des Produktes.
¹H-NMR (400 MHz, DMSO): δ = 10.31 (s, 1 H), 8.44 (s, 1 H), 8.01 (m, 2H), 7.84 (m, 2H), 5.19 (m, 1 H), 4.88 (d, 1 H), 3.81 (m, 1 H), 3.31 (m, 1 H), 1.40 (m, 1 H), 1.29 (m, 4H), 1.08 (m, 5H).

### V14b) Herstellung des Endproduktes

735 mg (1,37 mmol) des Zwischenproduktes in 29 ml Methanol wurden mit 950 mg (6,84 mmol) Kaliumcarbonat versetzt und 1,5 Stunden bei Raumtemperatur gerührt. Man verdünnte mit gesättigter Natriumchlorid-Lösung und extrahierte mit Essigester (3x). Die vereinten organischen Phasen wurden getrocknet (Na₂SO₄), filtriert und eingeengt. Man erhielt 607 mg (1,37 mmol) des Rohproduktes.
¹H-NMR (400 MHz, DMSO): δ = 10.08 (s, 1 H), 8.40 (s, 1 H), 7.86 (m, 2H), 7.75 (m, 2H), 5.19 (m, 1 H), 4.87 (d, 1 H), 3.97 (s, 1 H), 3.82 (m, 1 H), 2.56 (m, 1 H), 1.25 (d, 3H), 1.09 (d, 3H), 1.05 (m, 1 H), 0.86 (m, 3H).

Das Diastereomerengemisch wurde mittels präparativer HPLC in die reinen Stereoisomere aufgetrennt:

| | |
|---|---|
| Säule: | Chiralpak IC 5µ 250x20 mm |
| Eluenten: | Hexan / Ethanol 7:3 |
| Fluß: | 20,0 mL/min |
| Detektor: | UV 280 nm |
| Temperatur: | Raumtemperatur |
| Retentionszeit: | 9.6-11.2 min; Stereoisomer 1 |
| | 11.3-14.9 min; Stereoisomer 2 |

Stereoisomer 2 zeigte die stärkere in-vitro Effektivität als Stereoisomer 1 und wurde daher als Vergleichssubstanz in Beispiel 14 verwendet.

### Beispiel 10

### 10.1 Assay 1: CDK1/CycB Kinase Assay

Rekombinante CDK1- und CycB-GST-Fusionsproteine, gereinigt aus Bakulovirus-infizierten Insektenzellen (Sf9), wurden von ProQinase GmbH, Freiburg, gekauft. Das als Kinase-Substrat verwendet Histon IIIS ist über die Fa. Sigma käuflich zu erwerben.

CDK1/CycB (200 ng/Messpunkt) wurde für 10 min bei 22°C in Anwesenheit verschiedener Konzentrationen an Testsubstanzen (0 µM, sowie innerhalb des Bereiches 0,01 - 100 µM) in Assaypuffer [50 mM Tris/HCl pH8,0, 10 mM MgCl₂, 0,1 mM Na ortho-Vanadat, 1,0 mM Dithiothreitol, 0,5 µM Adenosintrisphosphat (ATP), 10 µg/Messpunkt Histon IIIS, 0,2 µCi/Messpunkt ³³P-gamma ATP, 0,05% NP40, 1,25% Dimethylsulfoxid] inkubiert. Die Reaktion wurde durch Zugabe von EDTA-Lösung (250 mM, pH8,0, 15 µl/Messpunkt) gestoppt.

Von jedem Reaktionsansatz wurden 15 µl auf P30 Filterstreifen (Fa. Wallac) aufgetragen, und nicht-eingebautes ³³P-ATP wurde durch dreimaliges Waschen der Filterstreifen für je 10 min in 0,5%iger Phosphorsäure entfernt. Nach dem Trocknen der Filterstreifen für 1 Stunde bei 70°C wurden die Filterstreifen mit Szintillator-Streifen (MeltiLex™ A, Fa. Wallac) bedeckt und für 1 Stunde bei 90°C eingebrannt. Die Menge an eingebautem ³³P (Substratphosphorylierung) wurde durch Szintillationsmessung in einem Gamma-Strahlungsmessgerät (Wallac) bestimmt. Die Messdaten wurden normalisiert auf 0% Inhibition (Enzymreaktion ohne Inhibitor) und 100% Inhibition (alle Assaykomponenten außer Enzym). Die Bestimmung der IC₅₀ Werte erfolgte mittels eines 4-Parameter Fits unter Benutzung firmeneigener Software.

### 10.2 Assay 2: CDK2/CycE Kinase Assay

Rekombinante CDK2- und CycE-GST-Fusionsproteine, gereinigt aus Bakulovirus-infizierten Insektenzellen (Sf9), wurden von ProQinase GmbH, Freiburg, gekauft. Histon IIIS, das als Kinase-Substrat verwendet wurde, wurde bei der Fa. Sigma gekauft.

CDK2/CycE (50 ng/Messpunkt) wurde für 10 min bei 22°C in Anwesenheit verschiedener Konzentrationen an Testsubstanzen (0 µM, sowie innerhalb des Bereiches 0,01 - 100 µM) in Assaypuffer [50 mM Tris/HCl pH8,0, 10 mM MgCl₂, 0,1 mM Na ortho-Vanadat, 1,0 mM Dithiothreitol, 0,5 µM Adenosintrisphosphat (ATP), 10 µg/Messpunkt Histon IIIS, 0,2 µCi/Messpunkt ³³P-gamma ATP, 0,05% NP40, 1,25% Dimethylsulfoxid] inkubiert. Die Reaktion wurde durch Zugabe von EDTA-Lösung (250 mM, pH8,0, 15 µl/Messpunkt) gestoppt.

Von jedem Reaktionsansatz wurden 15 µl auf P30 Filterstreifen (Fa. Wallac) aufgetragen, und nicht-eingebautes ³³P-ATP wurde durch dreimaliges Waschen der Filterstreifen für je 10 min in 0,5%iger Phosphorsäure entfernt. Nach dem Trocknen der Filterstreifen für 1 Stunde bei 70°C wurden die Filterstreifen mit Szintillator-Streifen (MeltiLex™ A, Fa. Wallac) bedeckt und für 1 Stunde bei 90°C eingebrannt. Die Menge an eingebautem ³³P (Substratphosphorylierung) wurde durch Szintillationsmessung in einem Gamma-Strahlungsmessgerät (Wallac) bestimmt. Die Messdaten wurden normalisiert auf 0% Inhibition (Enzymreaktion ohne Inhibitor) und 100% Inhibition (alle Assaykomponenten außer Enzym). Die Bestimmung der IC₅₀ Werte erfolgte mittels eines 4-Parameter Fits unter Benutzung firmeneigener Software.

### 10.3 Assay 3: VEGF Rezeptor-2 Kinase Assay

Rekombinante VEGF Rezeptortyrosinkinase-2 wurde als GST-Fusionsprotein aus Bakulovirus-infizierten Insektenzellen (Sf9) gereinigt. Poly-(Glu4Tyr), das als Kinase-Substrat verwendet wurde, wurde bei der Fa. Sigma gekauft.

VEGF Rezeptortyrosinkinase (90 ng/Messpunkt) wurde für 10 min bei 22°C in Anwesenheit verschiedener Konzentrationen an Testsubstanzen (0 µM, sowie innerhalb des Bereiches 0,001 - 30 µM) in 30 µl Assaypuffer [40 mM Tris/HCl pH5,5, 10 mM MgCl₂, 1 mM MnCl₂, 3 µM Na ortho-Vanadat, 1,0 mM Dithiothreitol, 8 µM Adenosintrisphosphat (ATP), 0,96 µg/Messpunkt poly-(Glu₄Tyr), 0,2 µCi/Messpunkt ³³P-gamma ATP, 1.4% Dimethylsulfoxid] inkubiert. Die Reaktion wurde durch Zugabe von EDTA-Lösung (250 mM, pH8,0, 15 µl/Messpunkt) gestoppt.

Von jedem Reaktionsansatz wurden 15 µl auf P30 Filterstreifen (Fa. Wallac) aufgetragen, und nicht-eingebautes ³³P-ATP wurde durch dreimaliges Waschen der Filterstreifen für je 10 min in 0,5%iger Phosphorsäure entfernt. Nach dem Trocknen der Filterstreifen für 1 Stunde bei 70°C wurden die Filterstreifen mit Szintillator-Streifen (MeltiLexTM A, Fa. Wallac) bedeckt und für 1 Stunde bei 90°C eingebrannt. Die Menge an eingebautem ³³P (Substratphosphorylierung) wurde durch Szintillationsmessung in einem gamma-Strahlungsmessgerät (Wallac) bestimmt. Die Messdaten wurden normalisiert auf 0% Inhibition (Enzymreaktion ohne Inhibitor) und 100% Inhibition (alle Assaykomponenten außer Enzym). Die Bestimmung der IC₅₀ Werte erfolgte mittels eines 4-Parameter Fits unter Benutzung firmeneigener Software.

### 10.4 Assay 4: Proliferationsassay

Kultivierte humane HeLa-MaTu Cervix-Karzinomazellen (bezogen von EPO-GmbH, Berlin) wurden in einer Dichte von 3000 Zellen/Messpunkt in einer 96-Loch Multititerplatte in 200 µl Wachstumsmedium (DMEM/HAMS F12, 2 mM L-Glutamin, 10% Fötales Kälberserum) ausplattiert. Nach 24 Stunden wurden die Zellen einer Platte (Nullpunkt-Platte) mit Kristallviolett gefärbt (s.u.), während das Medium der anderen Platten durch frisches Kulturmedium (200 µl), dem die Testsubstanzen in verschiedenen Konzentrationen (0 µM, sowie im Bereich 0,01 - 30 µM; die finale Konzentration des Lösungsmittels Dimethylsulfoxid betrug 0,5%) zugesetzt waren, ersetzt. Die Zellen wurden für 4 Tage in Anwesenheit der Testsubstanzen inkubiert. Die Zellproliferation wurde durch Färbung der Zellen mit Kristallviolett bestimmt: Die Zellen wurden durch Zugabe von 20 µl/Messpunkt einer 11 %igen GlutaraldehydLösung 15 min bei Raumtemperatur fixiert. Nach dreimaligem Waschen der fixierten Zellen mit Wasser wurden die Platten bei Raumtemperatur getrocknet. Die Zellen wurden durch Zugabe von 100 µl/Messpunkt einer 0,1 %igen Kristallviolett-Lösung (pH durch Zugabe von Essigsäure auf pH3 eingestellt) gefärbt. Nach dreimaligem Waschen der gefärbten Zellen mit Wasser wurden die Platten bei Raumtemperatur getrocknet. Der Farbstoff wurde durch Zugabe von 100 µl/Messpunkt einer 10%igen Essigsäure-Lösung gelöst. Die Extinktion wurde photometrisch bei einer Wellenlänge von 595 nm bestimmt. Die prozentuale Änderung des Zellwachstums wurde durch Normalisierung der Messwerte auf die Extinktionwerte der Nullpunktplatte (=0%) und die Extinktion der unbehandelten (0 µM) Zellen (=100%) berechnet. Die Messdaten wurden normalisiert auf 0% Inhibition (Zellproliferation ohne Inhibitor) und 100% Inhibition (Nullpunktplatte). Die Bestimmung der IC₅₀ Werte erfolgte mittels eines 4-Parameter Fits unter Benutzung firmeneigener Software.

### 10.5 Ergebnisse aus den Enzym- und Proliferationsassay

**Tab.1**

| Bsp. | CDK1/CycB (Assay 1) | CDK2/CycE (Assay 2) | VEGF-R2 (Assay 3) | HeLa-MaTu (Assay 4) |
|---|---|---|---|---|
| | Konzentration der halbmaximale Inhibition der Enzymaktivität bzw. der Zellproliferation, IC₅₀ [nM] | | | |
| 1-SI-1 | 9 | 7 | 114 | 13 |
| 1-SI-2 | 7 | 9 | 163 | 11 |
| 2-SI-1 | 5 | 6 | 84 | 12 |
| 2-SI-2 | 4 | 5 | 281 | 8 |
| 3 | 13 | 10 | | 70 |
| 4-SI-1 | 6 | 6 | 46 | 10 |
| 4-SI-2 | | | | |
| 5-SI-1 | 25 | 8 | 70 | 22 |
| 5-SI-2 | 9 | 8 | 82 | 10 |
| 6-SI-1 | 10 | 5 | 73 | 16 |
| 6-SI-2 | 5 | 5 | 71 | 10 |
| 7-SI-1 | 24 | 4 | 143 | 27 |
| 7-SI-2 | 7 | 5 | 136 | 10 |
| 8-SI-1 | 11 | 6 | 116 | 14 |
| 8-SI-2 | 3 | 4 | 81 | 10 |
| 9-SI-1 | 4 | 5 | 158 | 20 |
| 9-SI-2 | 17 | 3 | 154 | 21 |
| V11 | 8 | 7 | 59 | 13 |
| V12 | 3 | 3 | 32 | 13 |
| V13 | 3 | 4 | 140 | 9 |
| V14 | 4 | 7 | 91 | 10 |

Die Ergebnisse aus den Enzym- und Proliferationsassays zeigen keine systematische Überlegenheit der erfindungsgemäßen Verbindungen gegenüber den Verbindungen des Standes der Technik.

### Vergleich in-vivo

Die in-vivo Effektivität von Beispiel 5-SI-2 wurde in Beispiel 11 mit V11 vergleichend untersucht.

Die in-vivo Effektivität von Beispiel 6-SI-2 wurde in Beispiel 12 mit Beispiel 1.52 aus WO 2005/037800 (= Vergleichsubstanz V12) vergleichend untersucht.

Die in-vivo Effektivität von Beispiel 2-SI-2 wurde in Beispiel 13 mit Beispiel 3.13 aus WO 2005/037800 (= Vergleichsubstanz V13) vergleichend untersucht.

Die in-vivo Effektivität von Beispiel 1-SI-2 wurde in Beispiel 14 mit V14 vergleichend untersucht.

### Beispiel 11

HeLa-MaTu (EPO-GmbH, Berlin) humane Cervix-Karzinomzellen, die in Zellkultur gezüchtet wurden, wurden subkutan in die Flanke von weiblichen NMRI Nacktmäusen implantiert. Die Behandlung wurde begonnen sobald die Tumoren zu einer Größe von ca. 20 mm² herangewachsen waren. Die Studie wurde beendet sobald die Tumoren in einer der Gruppen eine Größe von ca. 150 mm² erreichten.

Folgende Versuchsgruppen wurden verwendet:
- Gruppe 1:: Kontrolle, Behandlung mit Lösungsvermittler (40% PEG400/60% Wasser)
- Gruppe 2:: Stereoisomer 2 hergestellt gemäß Beispiel 5 (= Beispiel 5-SI-2) 5 mg/kg, oral, 2x täglich an Tag 4, 5, 11, 12)

Die Studie war zur Bestimmung des initialen Ansprechens eines humanen Cervixkarzinom-Xenograftmodells auf die Therapien mit Beispiel 5-SI-2 ausgelegt.

Der wachstumshemmende Effekt der Verbindungen wurde im HeLa-MaTu Cervixtumor-Modell als Xenograft auf NMRI Nacktmäusen geprüft. Beispiel 5-SI-2 wurde in dem Lösungsvermittler 40% Polyethylenglykol (PEG) 400 / 60% Wasser zu einer finalen Konzentration von 0.5 mg/ml vollständig gelöst. Die Behandlung der etablierten Tumore wurde an Tag 4 nach der Inokulation der Tumore begonnen. Die Studie wurde an Tag 17 beendet nachdem die Tumorgröße der Tiere der Gruppe 1 (Kontrolle) die Größe von ca. 150 mm² überschritten hatten.

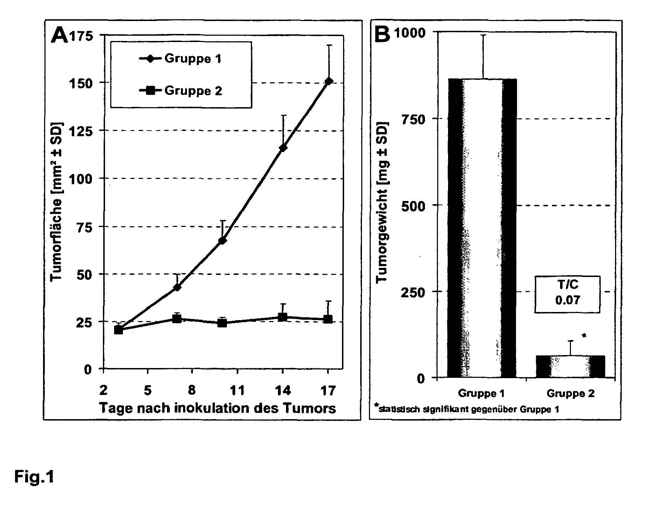

Die Ergebnisse der Studien (Fig. 1) zeigen, dass Beispiel 5-SI-2 in der gewählten Dosierung von 5 mg/kg oral in einem zyklischen Behandlungsschema (an 2 aufeinanderfolgenden Tagen 2x tägliche Behandlung gefolgt von 5 behandlungsfreien Tagen) das Tumorwachstum stark gehemmt (Gruppe 3, Reduktion des Tumorgewichtes am Ende der Studie auf 7% der Kontrollgruppe, p<0.05).

### 5-Br-Vergleichssubstanz (= V11)

HeLa-MaTu (EPO-GmbH, Berlin) humane Cervix-Karzinomzellen, die in Zellkultur gezüchtet wurden, wurden subkutan in die Flanke von weiblichen NMRI Nacktmäusen implantiert. Die Behandlung wurde begonnen sobald die Tumoren zu einer Größe von ca. 20 mm² herangewachsen waren. Die Studie wurde beendet sobald die Tumoren in einer der Gruppen eine Größe von ca. 150 mm² erreichten.

Folgende Versuchsgruppen wurden verwendet:
- Gruppe 1:: Kontrolle, Behandlung mit Lösungsvermittler (30% HPßCD/70% Wasser)
- Gruppe 2:: Verbindung gemäß WO 2005/037800 hergestellt nach oben offenbarter Herstellung V11 (8 mg/kg, oral, 2x täglich an Tag 4, 5, 11, 12)

Die Studie war zur Bestimmung des initialen Ansprechens eines humanen Cervixkarzinom-Xenograftmodells auf die Therapien mit der 5-Br-Vergleichssubstanz V11 ausgelegt. Der wachstumshemmende Effekt der Verbindungen wurde im HeLa-MaTu Cervixtumor-Modell als Xenograft auf NMRI Nacktmäusen geprüft. V11 wurde in dem Lösungsvermittler 30% ß-Hydroxypropyl-Cyclodextrin (HPßCD) / 70% Wasser zu einer finalen Konzentration von 0.8 mg/ml vollständig gelöst. Die Behandlung der etablierten Tumore wurde an Tag 4 nach der Inokulation der Tumore begonnen. Die Studie wurde an Tag 17 beendet nachdem die Tumorgröße der Tiere der Gruppe 1 (Kontrolle) die Größe von ca. 150 mm² überschritten hatten.

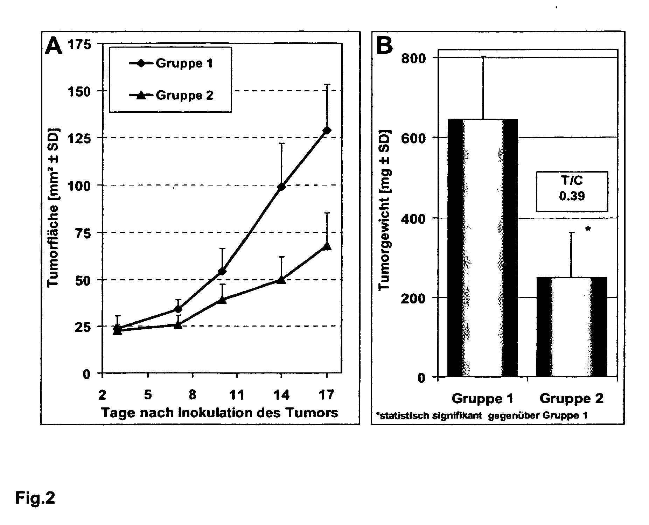

Die Ergebnisse der Studien (Fig. 2) zeigen, dass V11 in der gewählten Dosierung von 8 mg/kg oral 2x täglich an 2 aufeinanderfolgenden Tagen gefolgt von 5 behandlungsfreien Tagen das Tumorwachstum im HeLa-MaTu-Xenograftmodell hemmt (Gruppe 2, Reduktion des Tumorgewichtes am Ende der Studie auf 39% der Kontrollgruppe, p<0.05).

### Schlussfolgerung

Das Stereoisomer 2 des Beispieles 5 (Beispiel 5-SI-2 (5-CF₃)) erreicht in den zyklischen Behandlungsschema bestehend aus zwei täglichen oralen Applikationen mit einer Dosis von 5 mg/kg an zwei aufeinanderfolgenden Tagen gefolgt von 5 behandlungsfreien Tagen eine komplette Tumorwachstumshemmung im HeLa-MaTu Xenograftmodell (Behandlungs-/Kontroll-Verhaltnis T/C=0.07). Die 5-Br-Vergleichssubstanz (= V11) erreicht in dem entsprechenden verträglichen, zyklischen Behandlungsschema bei einer Dosis von 8 mg/kg lediglich eine Tumorwachstumsverzögerung im HeLa-MaTu Xenograftmodell (Behandlungs- /Kontroll-Verhaltnis T/C=0.39). Überraschenderweise zeigt das Beispiel 5-SI-2 (5-CF₃) im Vergleich zu V11 (5-Br) eine höhere Potenz (5 mg/kg Dosis für das Beispiel 5-SI-2 im Vergleich zu 8 mg/kg für V11) und eine deutlich bessere antitumorale Effektivität (komplette Tumorwachstumshemmung mit einem T/C=0.07 für das Beispiel 5-SI-2 im Vergleich zu Tumorwachstumsverlangsamung mit einem T/C=0.39 für V11).

### Beispiel 12

HeLa-MaTu (EPO-GmbH, Berlin) humane Cervix-Karzinomzellen, die in Zellkultur gezüchtet wurden, wurden subkutan in die Flanke von weiblichen NMRI Nacktmäusen implantiert. Die Behandlung wurde begonnen sobald die Tumoren zu einer Größe von ca. 20 mm² herangewachsen waren. Die Studie wurde beendet sobald die Tumoren in einer der Gruppen eine Größe von ca. 150 mm² erreichten.

Folgende Versuchsgruppen wurden verwendet:
- Gruppe 1:: Kontrolle, Behandlung mit Lösungsvermittler (40% PEG 400/60% Wasser)
- Gruppe 2:: Stereoisomer 2 hergestellt gemäß Beispiel 6 (= Beispiel 6-SI-2)
(3 mg/kg, oral, 2x täglich an Tag 4, 5, 11, 12, 17, 18)
- Gruppe 3:: Stereoisomer 2 hergestellt gemäß Beispiel 6 (= Beispiel 6-SI-2)
(4 mg/kg, oral, 2x täglich an Tag 4, 5, 11, 12, 17, 18)
- Gruppe 4:: Stereoisomer 2 hergestellt gemäß Beispiel 6 (= Beispiel 6-SI-2)
(5 mg/kg, oral, 2x täglich an Tag 4, 5, 11, 12, 17, 18)

Die Studie war zur Bestimmung des initialen Ansprechens eines humanen Cervixkarzinom-Xenograftmodells auf die Therapien mit Beispiel 6-SI-2 ausgelegt.

Der wachstumshemmende Effekt der Verbindungen wurde im HeLa-MaTu Cervixtumor-Modell als Xenograft auf NMRI Nacktmäusen geprüft. Beispiel 6-SI-2 wurde in dem Lösungsvermittler 40% Polyethylenglykol 400 (PEG 400) / 60% Wasser zu einer finalen Konzentration von 0.3 mg/ml (Gruppe 2), 0.4 mg/ml (Gruppe 3), oder 0.5 mg/ml (Gruppe 4) vollständig gelöst. Die Behandlung der etablierten Tumore wurde an Tag 4 nach der Inokulation der Tumore begonnen. Die Studie wurde an Tag 20 beendet nachdem die Tumorgröße der Tiere der Gruppe 1 (Kontrolle) die Größe von ca. 150 mm² überschritten hatten.

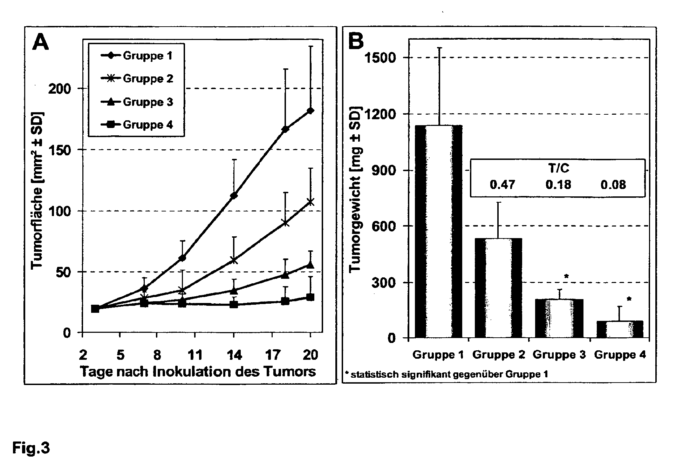

Die Ergebnisse der Studien (Fig. 3) zeigen, dass das Beispiel 6-SI-2 in einem Behandlungsschema bestehend aus zwei täglichen oralen Applikationen an 2 aufeinanderfolgenden Tagen gefolgt von 5 behandlungsfreien Tagen das Tumorwachstum im HeLa-MaTu-Xenograftmodell Dosis-abhängig hemmt. In der höchsten Dosisgruppe (Gruppe 4) wird das Tumorwachstum fast vollständig gehemmt und eine Reduktion des Tumorgewichtes am Ende der Studie auf 8% der Kontrollgruppe erreicht (T/C=0.08, p<0.05).

### 5-Br-Vergleichssubstanz (= V12)

HeLa-MaTu (EPO-GmbH, Berlin) humane Cervix-Karzinomzellen, die in Zellkultur gezüchtet wurden, wurden subkutan in die Flanke von weiblichen NMRI Nacktmäusen implantiert. Die Behandlung wurde begonnen sobald die Tumoren zu einer Größe von ca. 20 mm² herangewachsen waren. Die Studie wurde beendet sobald die Tumoren in einer der Gruppen eine Größe von ca. 150 mm² erreichten.

Folgende Versuchsgruppen wurden verwendet:
- Gruppe 1:: Kontrolle, Behandlung mit Lösungsvermittler (40% PEG 400/60% Wasser)
- Gruppe 2:: Verbindung gemäß Beispiel 1.52 aus WO 2005/037800 (= V12)
(7 mg/kg, oral, 2x täglich an Tag 4, 5, 11, 12, 17, 18, 23, 24)
- Gruppe 3:: Verbindung gemäß Beispiel 1.52 aus WO 2005/037800 (= V12)
(8.5 mg/kg, oral, 2x täglich an Tag 4, 5, 11, 12, 17, 18, 23, 24)
- Gruppe 4:: Verbindung gemäß Beispiel 1.52 aus WO 2005/037800 (= V12)
(10 mg/kg, oral, 2x täglich an Tag 4, 5, 11, 12, 17, 18, 23, 24)

Die Studie war zur Bestimmung des initialen Ansprechens eines humanen Cervixkarzinom-Xenograftmodells auf die Therapien mit der 5-Br-Vergleichssubstanz V12 ausgelegt. Der wachstumshemmende Effekt der Verbindungen wurde im HeLa-MaTu Cervixtumor-Modell als Xenograft auf NMRI Nacktmäusen geprüft. V12 wurde in dem Lösungsvermittler 40% Polyethylenglykol 400 (PEG 400) / 60% Wasser zu einer finalen Konzentration von 0.7 mg/ml (Gruppe 2), 0.85 mg/ml (Gruppe 3), oder 1.0 mg/ml (Gruppe 4) vollständig gelöst. Die Behandlung der etablierten Tumore wurde an Tag 4 nach der Inokulation der Tumore begonnen. Die Studie wurde an Tag 28 beendet nachdem die Tumorgröße der Tiere der Gruppe 1 (Kontrolle) die Größe von ca. 150 mm² überschritten hatten.

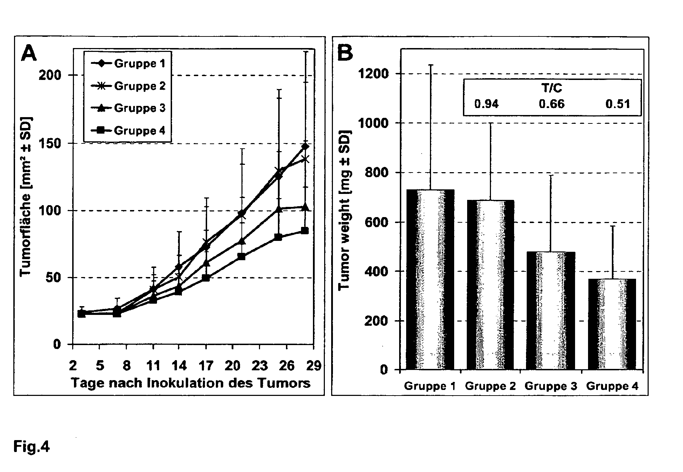

Die Ergebnisse der Studien (Fig. 4) zeigen, dass V12 in einem Behandlungsschema bestehend aus zwei täglichen oralen Applikationen an 2 aufeinanderfolgenden Tagen gefolgt von 5 behandlungsfreien Tagen das Tumorwachstum im HeLa-MaTu-Xenograftmodell Dosis-abhängig schwach hemmt. In der höchsten Dosisgruppe (Gruppe 4) wird das Tumorwachstum auf circa die Hälfte im Vergleich zur Kontrollgruppe gehemmt (T/C=0.51).

### Schlussfolgerung:

Das Stereoisomer 2 des Beispieles 6 (Beispiel 6-SI-2 (5-CF₃)) erreicht in den zyklischen Behandlungsschema bestehend aus zwei täglichen oralen Applikationen mit einer Dosis von 5 mg/kg an zwei aufeinanderfolgenden Tagen gefolgt von 5 behandlungsfreien Tagen eine fast vollständige Tumorwachstumshemmung im HeLa-MaTu Xenograftmodell (Behandlungs-/Kontroll-Verhaltnis T/C=0.08). Die Verbindung gemäß Beispiel 1.52 aus WO 2005/037800 (= V12 (5-Br)) erreicht in dem entsprechenden verträglichen, zyklischen Behandlungsschema bei einer Dosis von 10 mg/kg lediglich eine Tumorwachstumsverzögerung im HeLa-MaTu Xenograftmodell (Behandlungs-/Kontroll-Verhaltnis T/C=0.51). Überraschenderweise zeigt das Beispiel 6-SI-2 (5-CF₃) im Vergleich zu V12 (5-Br) eine höhere Potenz (5 mg/kg Dosis für das Beispiel 6-SI-2 im Vergleich zu 10 mg/kg für V12) und eine deutlich bessere antitumorale Effektivität (komplette Tumorwachstumshemmung mit einem T/C=0.08 für das Beispiel 6-SI-2 im Vergleich zu Tumorwachstumsverlangsamung mit einem T/C=0.51 für V12).

### Beispiel 13

HeLa-MaTu (EPO-GmbH, Berlin) humane Cervix-Karzinomzellen, die in Zellkultur gezüchtet wurden, wurden subkutan in die Flanke von weiblichen NMRI Nacktmäusen implantiert. Die Behandlung wurde begonnen sobald die Tumoren zu einer Größe von ca. 20 mm² herangewachsen waren. Die Studie wurde beendet sobald die Tumoren in einer der Gruppen eine Größe von ca. 160 mm² erreichten.

Folgende Versuchsgruppen wurden verwendet:
- Gruppe 1:: Kontrolle, Behandlung mit Lösungsvermittler (40% PEG 400/60% Wasser)
- Gruppe 2:: Stereoisomer 2 hergestellt gemäß Beispiel 2 (= Beispiel 2-SI-2)
(1.5 mg/kg, oral, 2x täglich an Tag 5, 6, 12, 13, 19, 20)
- Gruppe 3:: Stereoisomer 2 hergestellt gemäß Beispiel 2 (= Beispiel 2-SI-2)
(2.0 mg/kg, oral, 2x täglich an Tag 5, 6, 12, 13, 19, 20)
- Gruppe 4:: Stereoisomer 2 hergestellt gemäß Beispiel 2 (= Beispiel 2-SI-2)
(2.5 mg/kg, oral, 2x täglich an Tag 5, 6, 12, 13, 19, 20)

Die Studie war zur Bestimmung des initialen Ansprechens eines humanen Cervixkarzinom-Xenograftmodells auf die Therapien mit Beispiel 2-SI-2 ausgelegt.

Der wachstumshemmende Effekt der Verbindungen wurde im HeLa-MaTu Cervixtumor-Modell als Xenograft auf NMRI Nacktmäusen geprüft. Beispiel 2-SI-2 wurde in dem Lösungsvermittler 40% Polyethylenglykol 400 (PEG 400) / 60% Wasser zu einer finalen Konzentration von 0.15 mg/ml (Gruppe 2), 0.2 mg/ml (Gruppe 3), oder 0.25 mg/ml (Gruppe 4) vollständig gelöst. Die Behandlung der etablierten Tumore wurde an Tag 5 nach der Inokulation der Tumore begonnen. Die Studie wurde an Tag 20 beendet nachdem die Tumorgröße der Tiere der Gruppe 1 (Kontrolle) die Größe von ca. 160 mm² überschritten hatten.

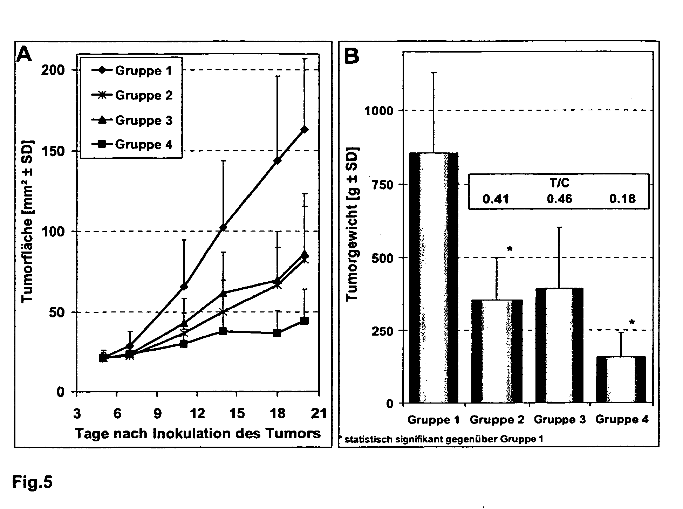

Die Ergebnisse der Studien (Fig. 5) zeigen, dass das Beispiel 2-SI-2 in einem Behandlungsschema bestehend aus zwei täglichen oralen Applikationen an 2 aufeinanderfolgenden Tagen gefolgt von 5 behandlungsfreien Tagen das Tumorwachstum im HeLa-MaTu-Xenograftmodell Dosis-abhängig hemmt. In der höchsten Dosisgruppe (2.5 mg/kg, Gruppe 4) wird das Tumorwachstum fast vollständig gehemmt und eine Reduktion des Tumorgewichtes am Ende der Studie auf 18% der Kontrollgruppe erreicht (T/C=0.18, p<0.05).

### 5-Br-Vergleichssubstanz (=V13)

HeLa-MaTu (EPO-GmbH, Berlin) humane Cervix-Karzinomzellen, die in Zellkultur gezüchtet wurden, wurden subkutan in die Flanke von weiblichen NMRI Nacktmäusen implantiert. Die Behandlung wurde begonnen sobald die Tumoren zu einer Größe von ca. 20 mm² herangewachsen waren. Die Studie wurde beendet sobald die Tumoren in einer der Gruppen eine Größe von ca. 160 mm² erreichten.

Folgende Versuchsgruppen wurden verwendet:
- Gruppe 1:: Kontrolle, Behandlung mit Lösungsvermittler (40% PEG 400/60% Wasser)
- Gruppe 2:: Verbindung gemäß Beispiel 3.13 aus WO 2005/037800 (= V13)
(6 mg/kg, oral, 2x täglich an Tag 5, 6, 12, 13, 19, 20)
- Gruppe 3:: Verbindung gemäß Beispiel 3.13 aus WO 2005/037800 (= V13)
(8 mg/kg, oral, 2x täglich an Tag 5, 6, 12, 13, 19, 20)
- Gruppe 4:: Verbindung gemäß Beispiel 3.13 aus WO 2005/037800 (= V13)
(10 mg/kg, oral, 2x täglich an Tag 5, 6, 12, 13, 19, 20)

Die Studie war zur Bestimmung des initialen Ansprechens eines humanen Cervixkarzinom-Xenograftmodells auf die Therapien mit der 5-Br-Vergleichssubstanz V13 ausgelegt. Der wachstumshemmende Effekt der Verbindungen wurde im HeLa-MaTu Cervixtumor-Modell als Xenograft auf NMRI Nacktmäusen geprüft. V13 wurde in dem Lösungsvermittler 40% Polyethylenglykol 400 (PEG 400) / 60% Wasser zu einer finalen Konzentration von 0.6 mg/ml (Gruppe 2), 0.8 mg/ml (Gruppe 3), oder 1.0 mg/ml (Gruppe 4) vollständig gelöst. Die Behandlung der etablierten Tumore wurde an Tag 5 nach der Inokulation der Tumore begonnen. Die Studie wurde an Tag 20 beendet nachdem die Tumorgröße der Tiere der Gruppe 1 (Kontrolle) die Größe von ca. 160 mm² überschritten hatten.

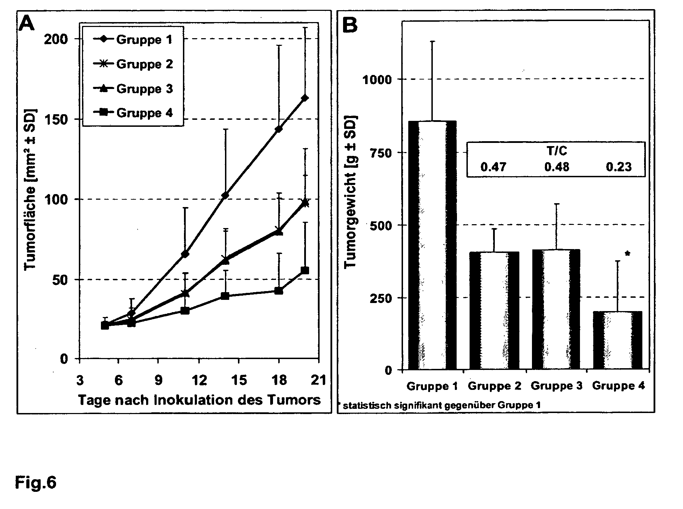

Die Ergebnisse der Studien (Fig. 6) zeigen, dass V13 in einem Behandlungsschema bestehend aus zwei täglichen oralen Applikationen an 2 aufeinanderfolgenden Tagen gefolgt von 5 behandlungsfreien Tagen das Tumorwachstum im HeLa-MaTu-Xenograftmodell Dosis-abhängig hemmt. In der höchsten Dosisgruppe (10 mg/kg, Gruppe 4) wird das Tumorwachstum sehr stark gehemmt und eine Reduktion des Tumorgewichtes am Ende der Studie auf 23% der Kontrollgruppe erreicht (T/C=0.23, p<0.05).

### Schlussfolgerung:

Das Stereoisomer 2 des Beispieles 2 (Beispiel 2-SI-2 (5-CF₃)) erreicht in den zyklischen Behandlungsschema bestehend aus zwei täglichen oralen Applikationen mit einer Dosis von 2.5 mg/kg an zwei aufeinanderfolgenden Tagen gefolgt von 5 behandlungsfreien Tagen eine fast vollständige Tumorwachstumshemmung im HeLa-MaTu Xenograftmodell (Behandlungs-/Kontroll-Verhaltnis T/C=0.18). Die Verbindung gemäß Beispiel 3.13 aus WO 2005/037800 (= V13 (5-Br)) erreicht in dem entsprechenden verträglichen, zyklischen Behandlungsschema bei einer Dosis von 10 mg/kg eine etwas geringere Tumorwachstumsverzögerung im HeLa-MaTu Xenograftmodell (Behandlungs-/Kontroll-Verhaltnis T/C=0.23). Überraschenderweise zeigt das Beispiel 2-SI-2 (5-CF₃) im Vergleich zu V13 (5-Br) eine deutlich höhere Potenz (2.5 mg/kg Dosis für das Beispiel 2-SI-2 im Vergleich zu 10 mg/kg für V13) und eine etwas bessere antitumorale Effektivität (Tumorwachstumshemmung mit einem T/C=0.18 für das Beispiel 2-SI-2 im Vergleich zu Tumorwachstumshemmung mit einem T/C=0.23 für V13).

### Beispiel 14

HeLa-MaTu (EPO-GmbH, Berlin) humane Cervix-Karzinomzellen, die in Zellkultur gezüchtet wurden, wurden subkutan in die Flanke von weiblichen NMRI Nacktmäusen implantiert. Die Behandlung wurde begonnen sobald die Tumoren zu einer Größe von ca. 20 mm² herangewachsen waren. Die Studie wurde beendet sobald die Tumoren in einer der Gruppen eine Größe von ca. 160 mm² erreichten.

Folgende Versuchsgruppen wurden verwendet:
- Gruppe 1:: Kontrolle, Behandlung mit Lösungsvermittler (40% PEG 400/60% Wasser)
- Gruppe 2:: Stereoisomer 2 hergestellt gemäß Beispiel 1 (= Beispiel 1-SI-2)
(1.5 mg/kg, oral, 2x täglich an Tag 5, 6, 12, 13, 19, 20)
- Gruppe 3:: Stereoisomer 2 hergestellt gemäß Beispiel 1 (= Beispiel 1-SI-2)
(2.0 mg/kg, oral, 2x täglich an Tag 5, 6, 12, 13, 19, 20)
- Gruppe 4:: Stereoisomer 2 hergestellt gemäß Beispiel 1 (= Beispiel 1-SI-2)
(2.5 mg/kg, oral, 2x täglich an Tag 5, 6, 12, 13, 19, 20)

Die Studie war zur Bestimmung des initialen Ansprechens eines humanen Cervixkarzinom-Xenograftmodells auf die Therapien mit Beispiel 1-SI-2 ausgelegt.

Der wachstumshemmende Effekt der Verbindungen wurde im HeLa-MaTu Cervixtumor-Modell als Xenograft auf NMRI Nacktmäusen geprüft. Beispiel 1-SI-2 wurde in dem Lösungsvermittler 40% Polyethylenglykol 400 (PEG 400) / 60% Wasser zu einer finalen Konzentration von 0.15 mg/ml (Gruppe 2), 0.2 mg/ml (Gruppe 3), oder 0.25 mg/ml (Gruppe 4) vollständig gelöst. Die Behandlung der etablierten Tumore wurde an Tag 5 nach der Inokulation der Tumore begonnen. Die Studie wurde an Tag 20 beendet nachdem die Tumorgröße der Tiere der Gruppe 1 (Kontrolle) die Größe von ca. 160 mm² überschritten hatten.

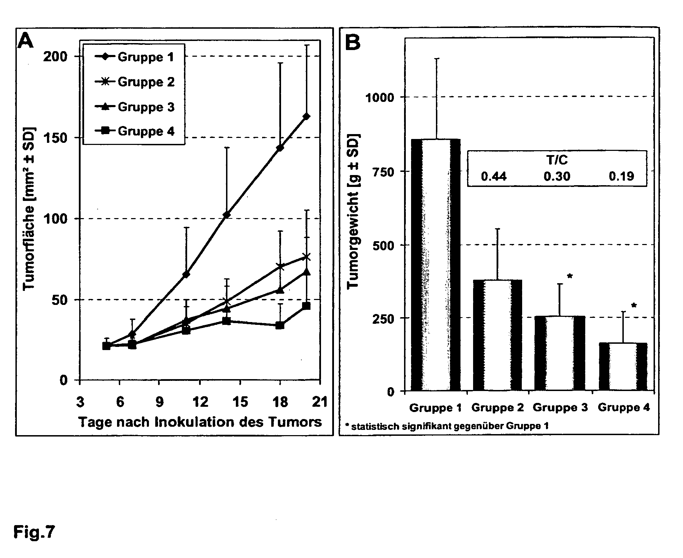

Die Ergebnisse der Studien (Fig. 7) zeigen, dass das Beispiel 1-SI-2 in einem Behandlungsschema bestehend aus zwei täglichen oralen Applikationen an 2 aufeinanderfolgenden Tagen gefolgt von 5 behandlungsfreien Tagen das Tumorwachstum im HeLa-MaTu-Xenograftmodell Dosis-abhängig hemmt. In der höchsten Dosisgruppe (2.5 mg/kg, Gruppe 4) wird das Tumorwachstum fast vollständig gehemmt und eine Reduktion des Tumorgewichtes am Ende der Studie auf 19% der Kontrollgruppe erreicht (T/C=0.19, p<0.05).

### 5-Br-Vergleichssubstanz (= V14)

HeLa-MaTu (EPO-GmbH, Berlin) humane Cervix-Karzinomzellen, die in Zellkultur gezüchtet wurden, wurden subkutan in die Flanke von weiblichen NMRI Nacktmäusen implantiert. Die Behandlung wurde begonnen sobald die Tumoren zu einer Größe von ca. 20 mm² herangewachsen waren. Die Studie wurde beendet sobald die Tumoren in einer der Gruppen eine Größe von ca. 160 mm² erreichten.

Folgende Versuchsgruppen wurden verwendet:
- Gruppe 1:: Kontrolle, Behandlung mit Lösungsvermittler (40% PEG 400/60% Wasser)
- Gruppe 2:: Verbindung gemäß WO 2005/037800 hergestellt nach oben offenbarter Herstellung V14
(6 mg/kg, oral, 2x täglich an Tag 5, 6, 12, 13, 19, 20)
- Gruppe 3:: Verbindung gemäß WO 2005/037800 hergestellt nach oben offenbarter Herstellung V14
(8 mg/kg, oral, 2x täglich an Tag 5, 6, 12, 13, 19, 20)
- Gruppe 4:: Verbindung gemäß WO 2005/037800 hergestellt nach oben offenbarter Herstellung V14
(10 mg/kg, oral, 2x täglich an Tag 5, 6, 12, 13, 19, 20)

Die Studie war zur Bestimmung des initialen Ansprechens eines humanen Cervixkarzinom-Xenograftmodells auf die Therapien mit der 5-Br-Vergleichssubstanz V14 ausgelegt. Der wachstumshemmende Effekt der Verbindungen wurde im HeLa-MaTu Cervixtumor-Modell als Xenograft auf NMRI Nacktmäusen geprüft. V14 wurde in dem Lösungsvermittler 40% Polyethylenglykol 400 (PEG 400) / 60% Wasser zu einer finalen Konzentration von 0.6 mg/ml (Gruppe 2), 0.8 mg/ml (Gruppe 3), oder 1.0 mg/ml (Gruppe 4) vollständig gelöst. Die Behandlung der etablierten Tumore wurde an Tag 5 nach der Inokulation der Tumore begonnen. Die Studie wurde an Tag 20 beendet nachdem die Tumorgröße der Tiere der Gruppe 1 (Kontrolle) die Größe von ca. 160 mm² überschritten hatten.

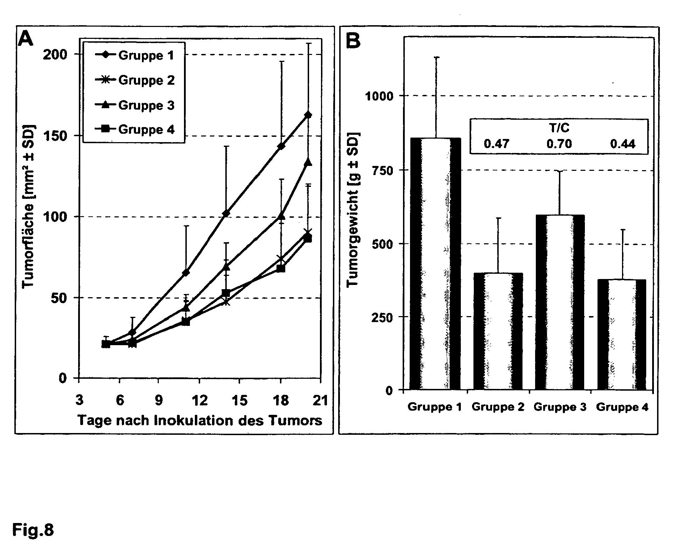

Die Ergebnisse der Studien (Fig. 8) zeigen, dass V14 in einem Behandlungsschema bestehend aus zwei täglichen oralen Applikationen an 2 aufeinanderfolgenden Tagen gefolgt von 5 behandlungsfreien Tagen das Tumorwachstum im HeLa-MaTu-Xenograftmodell Dosis-abhängig hemmt. In der höchsten Dosisgruppe (10 mg/kg, Gruppe 4) wird das Tumorwachstum schwach gehemmt und eine Reduktion des Tumorgewichtes am Ende der Studie auf 44% der Kontrollgruppe erreicht (T/C=0.44, statistische Signifikanz wurde nicht erreicht).

### Schlussfolgerung:

Das Stereoisomer 2 des Beispieles 1 (Beispiel 1-SI-2 (5-CF₃)) erreicht in den zyklischen Behandlungsschema bestehend aus zwei täglichen oralen Applikationen mit einer Dosis von 2.5 mg/kg an zwei aufeinanderfolgenden Tagen gefolgt von 5 behandlungsfreien Tagen eine fast vollständige Tumorwachstumshemmung im HeLa-MaTu Xenograftmodell (Behandlungs-/Kontroll-Verhaltnis T/C=0.19). Die 5-Br-Verbindung gemäß WO 2005/037800 (= V14 (5-Br)) erreicht in dem entsprechenden verträglichen, zyklischen Behandlungsschema bei einer Dosis von 10 mg/kg eine schwache Tumorwachstumshemmung im HeLa-MaTu Xenograftmodell (Behandlungs-/Kontroll-Verhaltnis T/C=0.44). Überraschenderweise zeigt das Beispiel 1-SI-2 (5-CF₃) im Vergleich zu V14 (5-Br) eine deutlich höhere Potenz (2.5 mg/kg Dosis für das Beispiel 1-SI-2 im Vergleich zu 10 mg/kg für V14) und eine deutlich überlegene antitumorale Effektivität (Tumorwachstumshemmung mit einem T/C=0.19 für das Beispiel 1-SI-2 im Vergleich zu Tumorwachstumshemmung mit einem T/C=0.44 für V14).

### Beschreibung der Figuren

**Fig. 1** zeigt die Tumorwachstumshemmung im humanen HeLa-MaTu Cervixtumor Xenograftmodell unter Behandlung mit Beispiel 5-SI-2. HeLa-MaTu Zellen wurden an Tag 0 subkutan in NMRI Nacktmäusen implantiert. Die Behandlung begann an Tag 4 nachdem die Tumoren eine Größe von ca. 20 mm² erreicht hatten. Die Behandlungen wurden in folgenden Dosierungen und Applikationsschemata durchgeführt:
   - Gruppe 1:: (Kontrollgruppe) - Lösungsvermittler (40% PEG400 / 60% Wasser)
   - Gruppe 2:: Beispiel 5-SI-2, zyklisches Behandlungsschema, 2x täglich oral an Tagen 4, 5, 11, 12, Dosierung 5 mg/kg.

   A) zeitlicher Verlauf des Wachstums der HeLa-MaTu Xenografttumore.
   B) Gewicht der HeLa-MaTu Tumore am Tag 17, sowie das Verhältnis des durchschnittlichen Tumorgewichtes der Behandlungsgruppe zu dem durchschnittlichen Tumorgewicht der Kontrollgruppe (T/C).
**Fig. 2** zeigt die Tumorwachstumshemmung im humanen HeLa-MaTu Cervixtumor-Xenograftmodell unter Behandlung mit V11. HeLa-MaTu Zellen wurden an Tag 0 subkutan in NMRI Nacktmäusen implantiert. Die Behandlung begann an Tag 4 nachdem die Tumoren eine Größe von ca. 20 mm² erreicht hatten. Die Behandlungen wurden in folgenden Dosierungen und Applikationsschemata durchgeführt:
   - Gruppe 1:: (Kontrollgruppe) - Lösungsvermittler (30% HPßCD / 70% Wasser, 1x täglich oral an Tag 4-17);
   - Gruppe 2:: V11, zyklisches Behandlungsschema, 2x täglich oral an Tagen 4, 5, 11, 12, Dosierung 8 mg/kg.

   A) zeitlicher Verlauf des Wachstums der HeLa-MaTu Xenografttumore.
   B) Gewicht der HeLa-MaTu Tumore am Tag 17, sowie das Verhältnis des durchschnittlichen Tumorgewichtes der jeweiligen Behandlungsgruppen zu dem durchschnittlichen Tumorgewicht der Kontrollgruppe (T/C).
**Fig. 3****:** zeigt die Tumorwachstumshemmung im humanen HeLa-MaTu Cervixtumor-Xenograftmodell unter Behandlung mit dem Beispiel 6-SI-2. HeLa-MaTu Zellen wurden an Tag 0 subkutan in NMRI Nacktmäusen implantiert. Die Behandlung begann an Tag 4 nachdem die Tumoren eine Größe von ca. 20 mm² erreicht hatten. Die Behandlungen wurden in folgenden Dosierungen und in einem zyklischen Behandlungsschema bestehend aus zwei täglichen oralen Applikationen an Tagen 4, 5, 11, 12, 17, und 18 durchgeführt:
   - Gruppe 1:: (Kontrollgruppe) - Lösungsvermittler (40% PEG400 / 60% Wasser);
   - Gruppe 2:: Beispiel 6-SI-2, Dosierung 3 mg/kg;
   - Gruppe 3:: Beispiel 6-SI-2, Dosierung 4 mg/kg;
   - Gruppe 4:: Beispiel 6-SI-2, Dosierung 5 mg/kg.

   A) zeitlicher Verlauf des Wachstums der HeLa-MaTu Xenografttumore.
   B) Gewicht der HeLa-MaTu Tumore am Tag 20, sowie das Verhältnis des durchschnittlichen Tumorgewichtes der jeweiligen Behandlungsgruppen zu dem durchschnittlichen Tumorgewicht der Kontrollgruppe (T/C).
**Fig. 4****:** zeigt die Tumorwachstumshemmung im humanen HeLa-MaTu Cervixtumor-Xenograftmodell unter Behandlung mit V12. HeLa-MaTu Zellen wurden an Tag 0 subkutan in NMRI Nacktmäusen implantiert. Die Behandlung begann an Tag 4 nachdem die Tumoren eine Größe von ca. 20 mm² erreicht hatten. Die Behandlungen wurden in folgenden Dosierungen und in einem zyklischen Behandlungsschema bestehend aus zwei täglichen oralen Applikationen an Tagen 4, 5, 11, 12, 17, 18, 23 und 24 durchgeführt:
   - Gruppe 1:: (Kontrollgruppe) - Lösungsvermittler (40% PEG400 / 60% Wasser);
   - Gruppe 2:: V12, Dosierung 7 mg/kg;
   - Gruppe 3:: V12, Dosierung 8.5 mg/kg;
   - Gruppe 4:: V12, Dosierung 10 mg/kg.

   A) zeitlicher Verlauf des Wachstums der HeLa-MaTu Xenografttumore.
   B) Gewicht der HeLa-MaTu Tumore am Tag 28, sowie das Verhältnis des durchschnittlichen Tumorgewichtes der jeweiligen Behandlungsgruppen zu dem durchschnittlichen Tumorgewicht der Kontrollgruppe (T/C).
**Fig. 5****:** zeigt die Tumorwachstumshemmung im humanen HeLa-MaTu Cervixtumor-Xenograftmodell unter Behandlung mit dem Beispiel 2-SI-2. HeLa-MaTu Zellen wurden an Tag 0 subkutan in NMRI Nacktmäusen implantiert. Die Behandlung begann an Tag 5 nachdem die Tumoren eine Größe von ca. 20 mm² erreicht hatten. Die Behandlungen wurden in folgenden Dosierungen und in einem zyklischen Behandlungsschema bestehend aus zwei täglichen oralen Applikationen an Tagen 5, 6, 12, 13, 19, und 20 durchgeführt:
   - Gruppe 1:: (Kontrollgruppe) - Lösungsvermittler (40% PEG400 / 60% Wasser);
   - Gruppe 2:: Beispiel 2-SI-2, Dosierung 1.5 mg/kg;
   - Gruppe 3:: Beispiel 2-SI-2, Dosierung 2 mg/kg;
   - Gruppe 4:: Beispiel 2-SI-2, Dosierung 2.5 mg/kg.

   A) zeitlicher Verlauf des Wachstums der HeLa-MaTu Xenografttumore.
   B) Gewicht der HeLa-MaTu Tumore am Tag 20, sowie das Verhältnis des durchschnittlichen Tumorgewichtes der jeweiligen Behandlungsgruppen zu dem durchschnittlichen Tumorgewicht der Kontrollgruppe (T/C).
**Fig. 6****:** zeigt die Tumorwachstumshemmung im humanen HeLa-MaTu Cervixtumor-Xenograftmodell unter Behandlung mit V13. HeLa-MaTu Zellen wurden an Tag 0 subkutan in NMRI Nacktmäusen implantiert. Die Behandlung begann an Tag 5 nachdem die Tumoren eine Größe von ca. 20 mm² erreicht hatten. Die Behandlungen wurden in folgenden Dosierungen und in einem zyklischen Behandlungsschema bestehend aus zwei täglichen oralen Applikationen an Tagen 5, 6, 12, 13, 19, und 20 durchgeführt:
   - Gruppe 1:: (Kontrollgruppe) - Lösungsvermittler (40% PEG400 / 60% Wasser);
   - Gruppe 2:: V13, Dosierung 6 mg/kg;
   - Gruppe 3:: V13, Dosierung 8 mg/kg;
   - Gruppe 4:: V13, Dosierung 10 mg/kg.

   A) zeitlicher Verlauf des Wachstums der HeLa-MaTu Xenografttumore.
   B) Gewicht der HeLa-MaTu Tumore am Tag 20, sowie das Verhältnis des durchschnittlichen Tumorgewichtes der jeweiligen Behandlungsgruppen zu dem durchschnittlichen Tumorgewicht der Kontrollgruppe (T/C).
**Fig. 7****:** zeigt die Tumorwachstumshemmung im humanen HeLa-MaTu Cervixtumor-Xenograftmodell unter Behandlung mit dem Beispiel 1-SI-2. HeLa-MaTu Zellen wurden an Tag 0 subkutan in NMRI Nacktmäusen implantiert. Die Behandlung begann an Tag 5 nachdem die Tumoren eine Größe von ca. 20 mm² erreicht hatten. Die Behandlungen wurden in folgenden Dosierungen und in einem zyklischen Behandlungsschema bestehend aus zwei täglichen oralen Applikationen an Tagen 5, 6, 12, 13, 19, und 20 durchgeführt:
   - Gruppe 1:: (Kontrollgruppe) - Lösungsvermittler (40% PEG400 / 60% Wasser);
   - Gruppe 2:: Beispiel 1-SI-2, Dosierung 1.5 mg/kg;
   - Gruppe 3:: Beispiel 1-SI-2, Dosierung 2 mg/kg;
   - Gruppe 4:: Beispiel 1-SI-2, Dosierung 2.5 mg/kg.

   A) zeitlicher Verlauf des Wachstums der HeLa-MaTu Xenografttumore.
   B) Gewicht der HeLa-MaTu Tumore am Tag 20, sowie das Verhältnis des durchschnittlichen Tumorgewichtes der jeweiligen Behandlungsgruppen zu dem durchschnittlichen Tumorgewicht der Kontrollgruppe (T/C).
**Fig. 8****:** zeigt die Tumorwachstumshemmung im humanen HeLa-MaTu Cervixtumor-Xenograftmodell unter Behandlung mit V14. HeLa-MaTu Zellen wurden an Tag 0 subkutan in NMRI Nacktmäusen implantiert. Die Behandlung begann an Tag 5 nachdem die Tumoren eine Größe von ca. 20 mm² erreicht hatten. Die Behandlungen wurden in folgenden Dosierungen und in einem zyklischen Behandlungsschema bestehend aus zwei täglichen oralen Applikationen an Tagen 5, 6, 12, 13, 19, und 20 durchgeführt:
   - Gruppe 1:: (Kontrollgruppe) - Lösungsvermittler (40% PEG400 / 60% Wasser);
   - Gruppe 2:: V14, Dosierung 6 mg/kg;
   - Gruppe 3:: V14, Dosierung 8 mg/kg;
   - Gruppe 4:: V14, Dosierung 10 mg/kg.

   A) zeitlicher Verlauf des Wachstums der HeLa-MaTu Xenografttumore.
   B) Gewicht der HeLa-MaTu Tumore am Tag 20, sowie das Verhältnis des durchschnittlichen Tumorgewichtes der jeweiligen Behandlungsgruppen zu dem durchschnittlichen Tumorgewicht der Kontrollgruppe (T/C).

## Patentansprüche

1. Verbindungen der allgemeinen Formel (I) 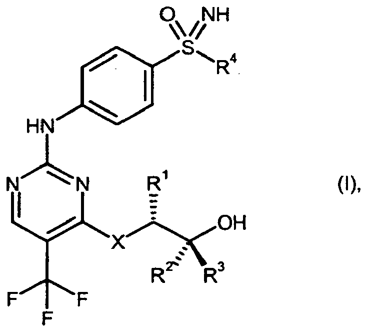 in der
X für -O- oder -NH- steht, und
R¹ für eine Methyl-, Ethyl-, Propyl- oder Isopropylgruppe steht, und
R² und R³ unabhängig voneinander für Wasserstoff, eine Methyl- oder Ethylgruppe stehen, und
R⁴ für eine C₁-C₆-Alkylgruppe oder einen C₃-C₇-Cycloalkylring steht,
sowie deren Salze, Diastereomere und Enantiomere

2. Verbindungen gemäß Anspruch 1,
wobei X für -O- steht,
sowie deren Salze, Diastereomere und Enantiomere

3. Verbindungen gemäß einem der Ansprüche 1 oder 2,
wobei R¹ für eine Methylgruppe steht,
sowie deren Salze, Diastereomere und Enantiomere

4. Verbindungen gemäß einem der Ansprüche 1 bis 3,
wobei R² für eine Methylgruppe steht,
sowie deren Salze, Diastereomere und Enantiomere

5. Verbindungen gemäß einem der Ansprüche 1 bis 4,
wobei R³ für Wasserstoff oder eine Methylgruppe steht,
sowie deren Salze, Diastereomere und Enantiomere

6. Verbindungen gemäß einem der Ansprüche 1 bis 5,
wobei R⁴ für eine Methyl- oder Ethylgruppe oder für einen Cyclopropylring,
sowie deren Salze, Diastereomere und Enantiomere

7. Verbindungen der allgemeinen Formel (I) gemäß Anspruch 1,
in der
X für -O- oder -NH- steht, und
R¹ für eine Methylgruppe steht, und
R² für eine Methylgruppe steht, und
R³ für Wasserstoff oder eine Methylgruppe steht, und
R⁴ eine Methyl- oder Ethylgruppe oder für einen Cyclopropylring steht,
sowie deren Salze, Diastereomere und Enantiomere

8. Verfahren zur Herstellung von Verbindungen der allgemeinen Formel (la), umfassend mindestens einen der Schritte a)-h)
a) Oxidation einer Verbindung der Formel (**IVd**) zum Sulfoxid der Formel (**IVc**). 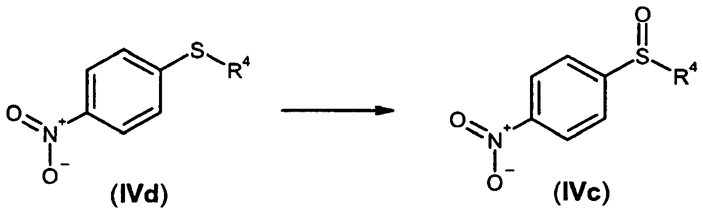
b₁) Direkte Iminierung des Sulfoxides der Formel **(IVc)** zu einem geschützten Sulfoximin der Formel **(IVa).** 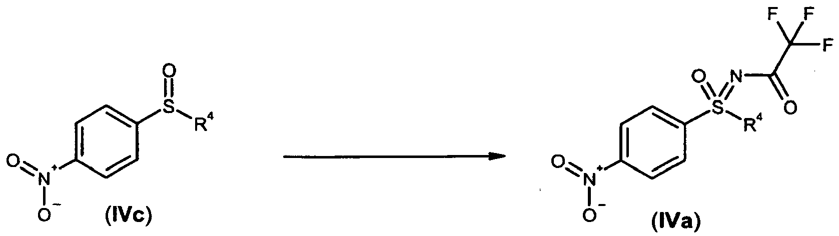 oder
b₂) Iminierung des Sulfoxides der Formel **(IVc)** zu einem ungeschützten Sulfoximin der Formel **(IVb)** und anschließende Einführung der Schutzgruppe zu einer Verbindung der Formel **(IVa).** 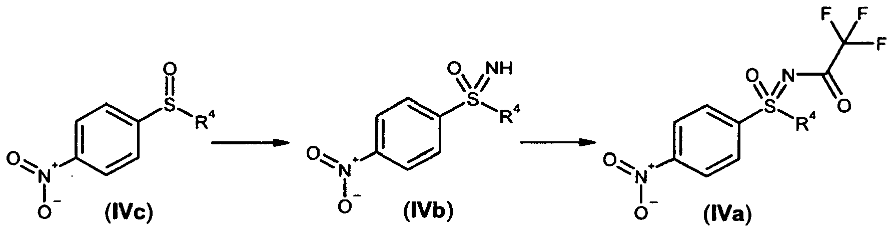
c) Reduktion der Verbindung der Formel **(IVa)** zu einer Verbindung der Formel **(IV)** 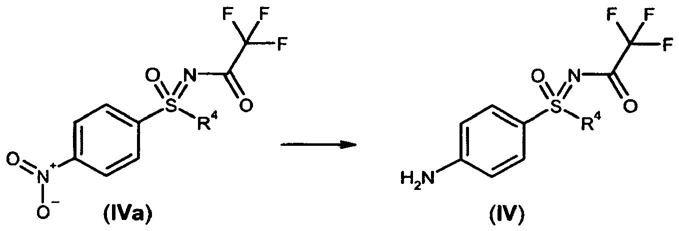
d) Funktionalisierung der 4-Position von 2,4-Dichlor-5-jod-pyrimidin **(VII)** durch Umsetzung mit einem mono-geschützten Diol der Formel **(VI)** unter Bildung eines Intermediates der Formel (**Va**). 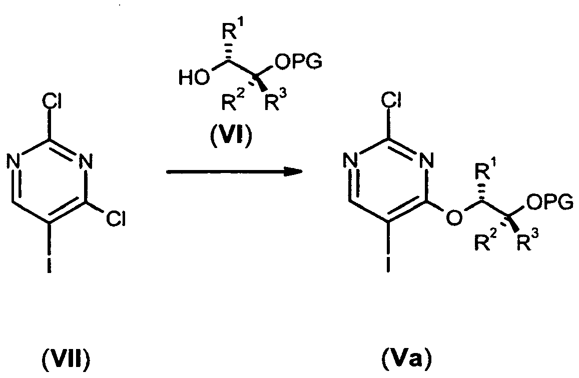
e) Herstellung des 5-CF₃ Intermediates (V). 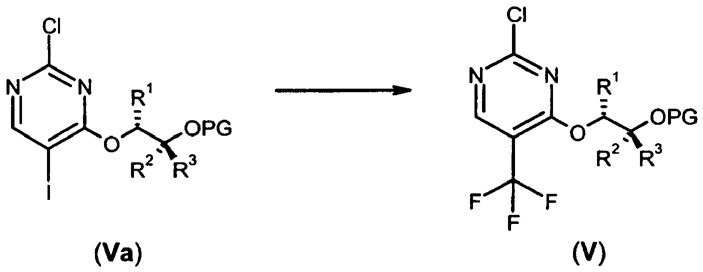
f) Kupplung der Verbindungen der Formel **(IV)** und (**V**) zum Intermediat der Formel (**III)**. 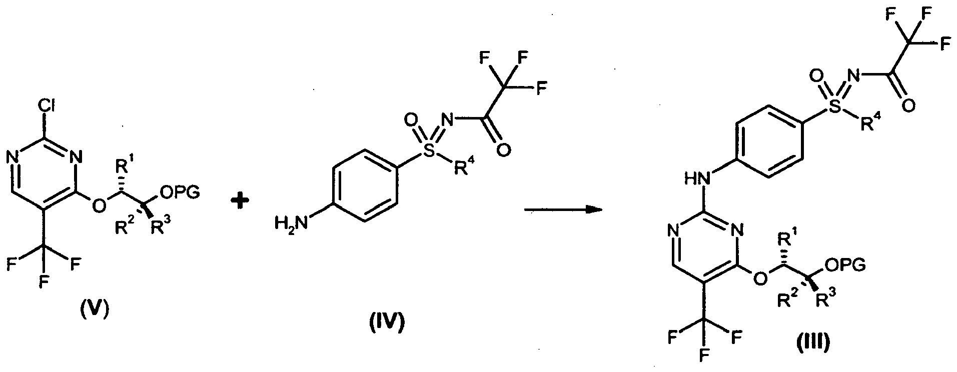
g) Abspaltung der Schutzgruppe PG unter Bildung von **(II).** 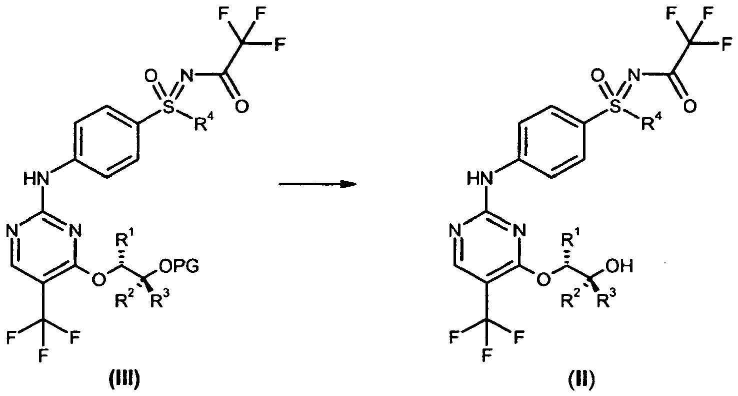
h) Abspaltung der Schutzgruppe am Sulfoximin unter Bildung von **(Ia).** 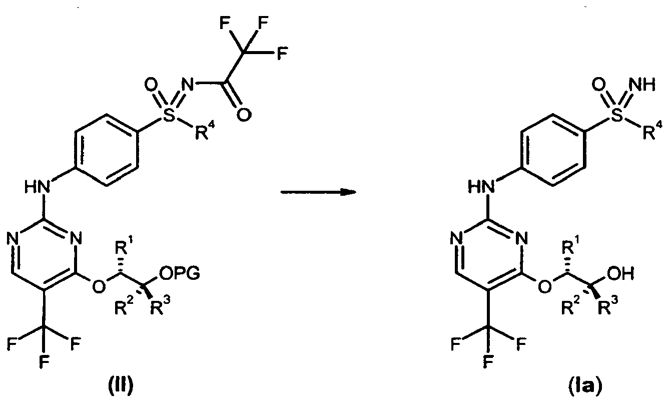
wobei die Substituenten R¹, R², R³ und R⁴ die in der allgemeinen Formel **(I)** der Ansprüche 1 bis 7 angegebenen Bedeutungen aufweisen.

9. Verfahren zur Herstellung von Verbindungen der allgemeinen Formel (Ib), umfassend mindestens einen der Schritte a)-f)
a) Oxidation einer Verbindung der Formel (**IVd**) zum Sulfoxid der Formel (**IVc**). 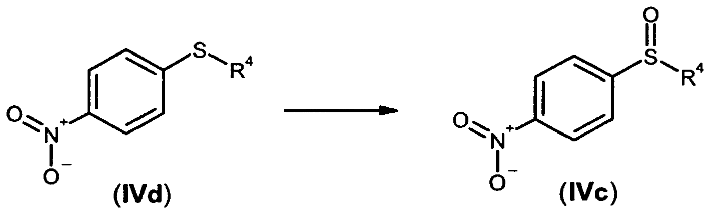
b₁) Direkte Iminierung des Sulfoxides der Formel **(IVc)** zu einem geschützten Sulfoximin der Formel **(IVa).** 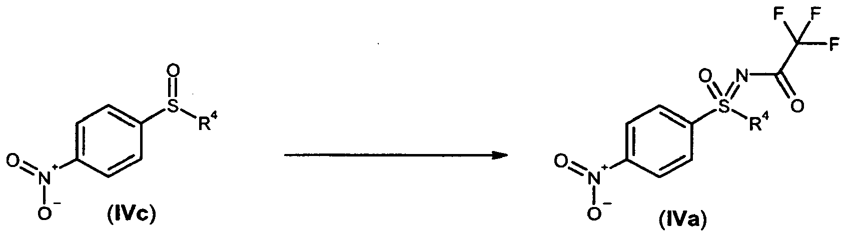 oder
b₂) Iminierung des Sulfoxides der Formel **(IVc)** zu einem ungeschützten Sulfoximin der Formel (**IVb**) und anschließende Einführung der Schutzgruppe zu einer Verbindung der Formel **(IVa).** 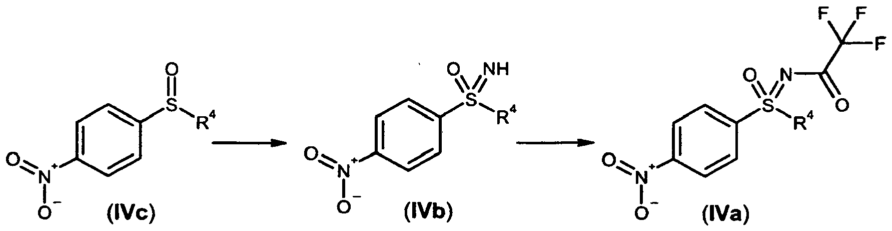
c) Reduktion der Verbindung der Formel (**IVa**) zu einer Verbindung der Formel **(IV)** 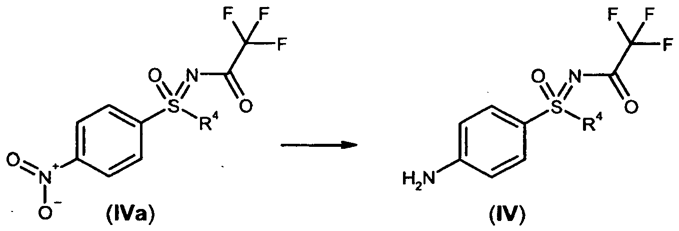
d) Funktionalisierung der 4-Position von 2,4-Dichlor-5-trifluormethyl-pyrimidin (**VIIb**) durch Umsetzung mit einem Amin der Formel (**VIa**) unter Bildung eines Intermediates der Formel (Vb). 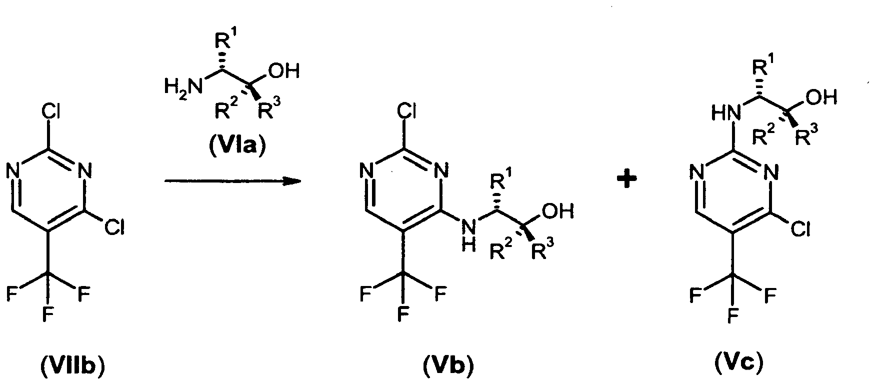
e) Kupplung der Verbindungen der Formel (Vb) und **(IV)** zum Intermediat der Formel (**IIb**). 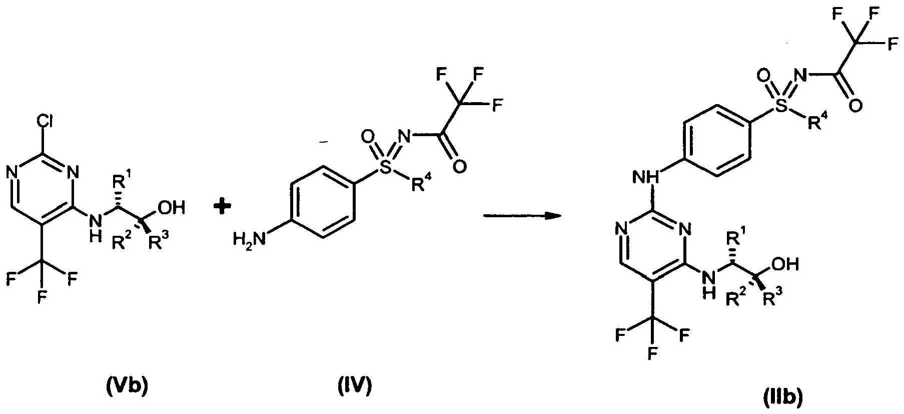
f) Abspaltung der Schutzgruppe am Sulfoximin unter Bildung von (**Ib**). 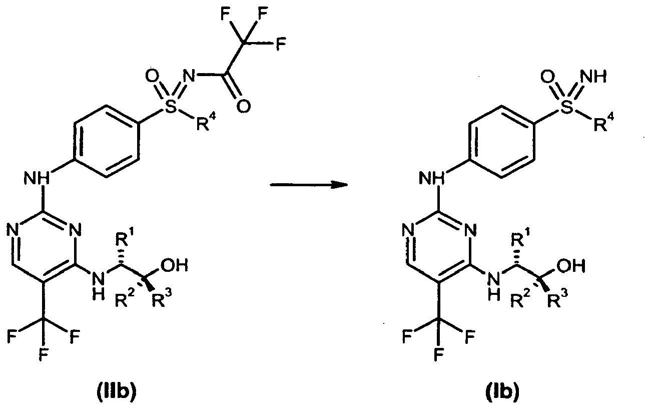
wobei die Substituenten R¹, R², R³ und R⁴ die in der allgemeinen Formel (I) der Ansprüche 1 bis 7 angegebenen Bedeutungen aufweisen.

10. Verbindungen gemäß einem der Ansprüche 1 bis 7 zur Verwendung als Arzneimittel.

11. Verwendung einer Verbindung gemäß einem der Ansprüche 1 bis 7 zur Herstellung eines Arzneimittels zur Behandlung von Krebs.

12. Verbindungen gemäß einem der Ansprüche 1 bis 7 zur Verwendung als Arzneimittel gegen Krebs.

13. Pharmazeutische Formulierung enthaltend eine Verbindung gemäß einem der Ansprüche 1 bis 7.

## Claims

1. Compounds of general formula (I) 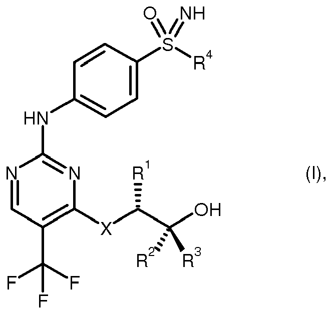 in which
X stands for -O- or -NH-, and
R¹ stands for a methyl, ethyl, propyl or isopropyl group, and
R² and R³ independently of one another, stand for hydrogen, a methyl or ethyl group, and
R⁴ stands for a C₁-C₆-alkyl group or a C₃-C₇-cycloalkyl ring,
and salts, diastereomers and enantiomers thereof.

2. Compounds according to Claim 1,
wherein X stands for -O-,
and salts, diastereomers and enantiomers thereof.

3. Compounds according to one of Claims 1 or 2,
wherein R¹ stands for a methyl group,
and salts, diastereomers and enantiomers thereof.

4. Compounds according to one of Claims 1 to 3,
wherein R² stands for a methyl group,
and salts, diastereomers and enantiomers thereof.

5. Compounds according to one of Claims 1 to 4,
wherein R³ stands for hydrogen or a methyl group,
and salts, diastereomers and enantiomers thereof.

6. Compounds according to one of Claims 1 to 5,
wherein R⁴ stands for a methyl or ethyl group or for a cyclopropyl ring,
and salts, diastereomers and enantiomers thereof.

7. Compounds of general formula (I) according to Claim 1,
in which
X stands for -O- or -NH-, and
R¹ stands for a methyl group, and
R² stands for a methyl group, and
R³ stands for hydrogen or a methyl group, and
R⁴ stands for a methyl or ethyl group or for a cyclopropyl ring,
and salts, diastereomers and enantiomers thereof.

8. Method of production of compounds of general formula (Ia), comprising at least one of steps a)-h)
a) Oxidation of a compound of formula **(IVd)** to the sulfoxide of formula (**IVc**). 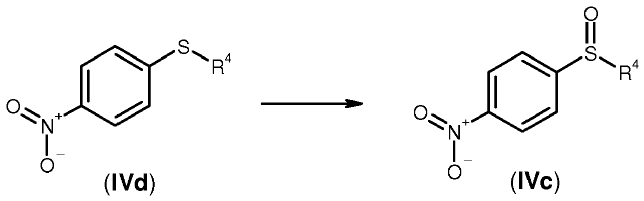
b₁) Direct imination of the sulfoxide of formula **(IVc)** to a protected sulfoximine of formula (**IVa**). 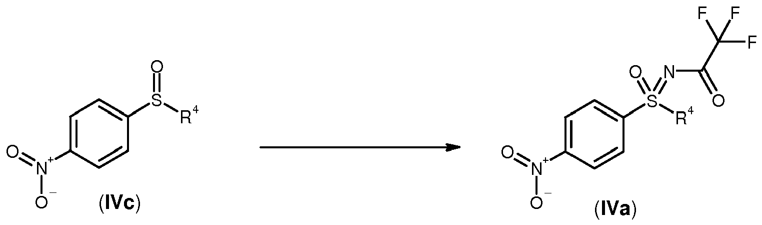 or
b₂) Imination of the sulfoxide of formula **(IVc)** to an unprotected sulfoximine of formula **(IVb)** and subsequent introduction of the protective group to a compound of formula (**IVa**). 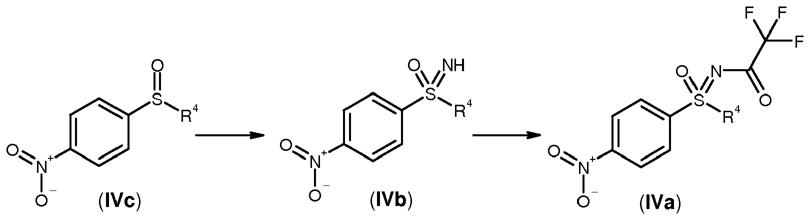
c) Reduction of the compound of formula **(IVa)** to a compound of formula **(IV)** 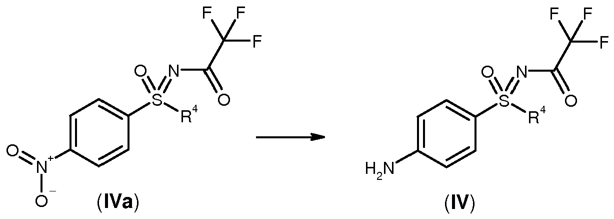
d) Functionalization of the 4-position of 2,4-dichloro-5-iodo-pyrimidine **(VII)** by reaction with a mono-protected diol of formula **(VI)** with formation of an intermediate of formula (Va). 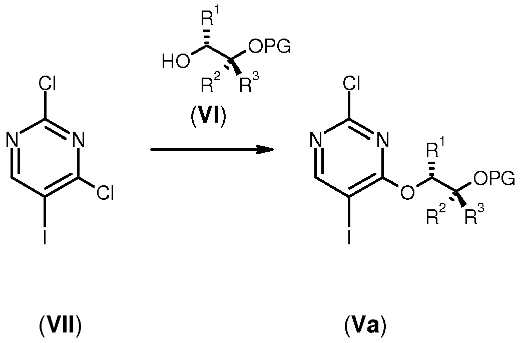
e) Production of the 5-CF₃ intermediate **(V).** 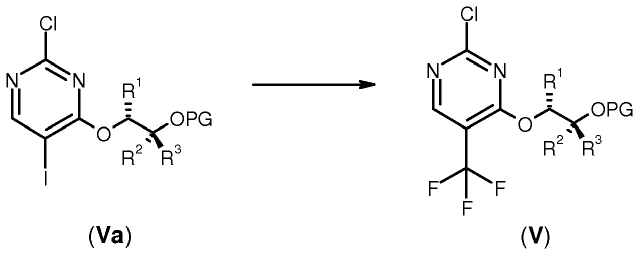
f) Coupling of the compounds of formula **(IV)** and **(V)** to form the intermediate of formula **(III).** 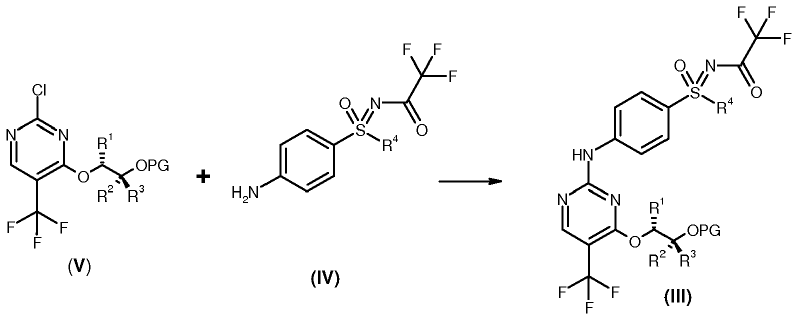
g) Cleavage of the protective group PG with formation of (II). 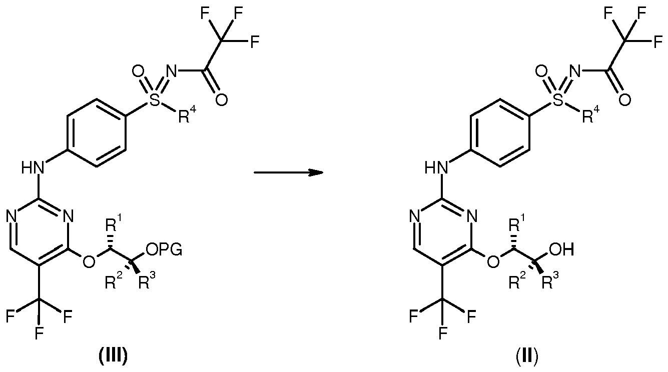
h) Cleavage of the protective group on the sulfoximine with formation of (**Ia**). 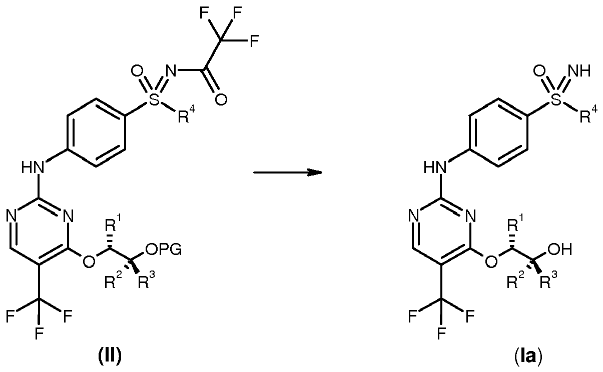
wherein the substituents R¹, R², R³ and R⁴ have the meanings given in general formula (I) of Claims 1 to 7.

9. Method of production of compounds of general formula (Ib), comprising at least one of steps a)-f)
a) Oxidation of a compound of formula **(IVd)** to the sulfoxide of formula (**IVc**). 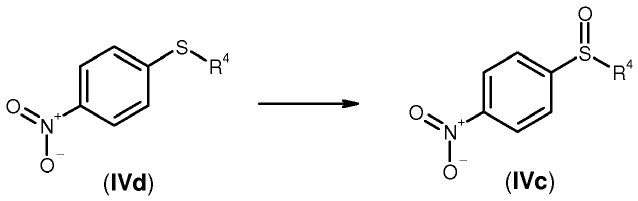
b₁) Direct imination of the sulfoxide of formula **(IVc)** to form a protected sulfoximine of formula (**IVa**). 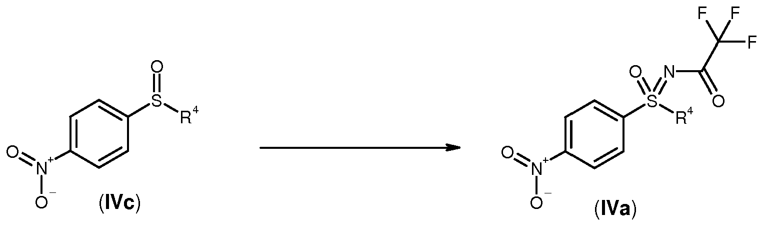 or
b₂) Imination of the sulfoxide of formula **(IVc)** to form an unprotected sulfoximine of formula **(IVb)** and subsequent introduction of the protective group to a compound of formula (**IVa**). 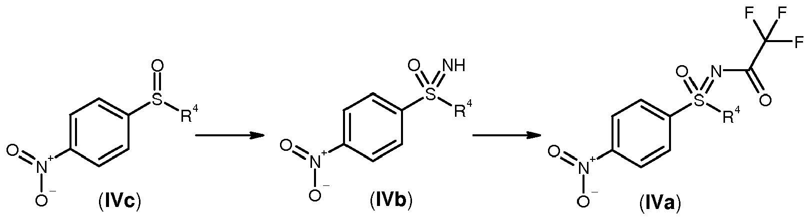
c) Reduction of the compound of formula **(IVa)** to a compound of formula **(IV)** 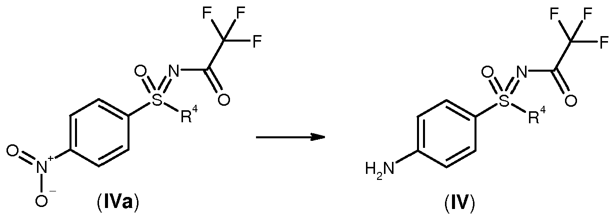
d) Functionalization of the 4-position of 2,4-dichloro-5-trifluoromethyl-pyrimidine (VIIb) by reaction with an amine of formula (**VIa**) with formation of an intermediate of formula **(Vb).** 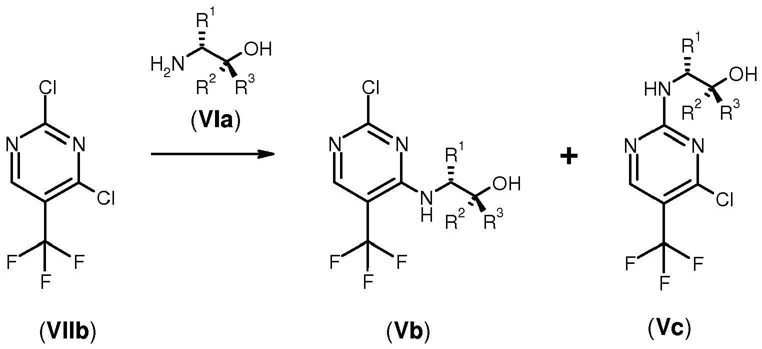
e) Coupling of the compounds of formula (Vb) and (IV) to form the intermediate of formula (**IIb**). 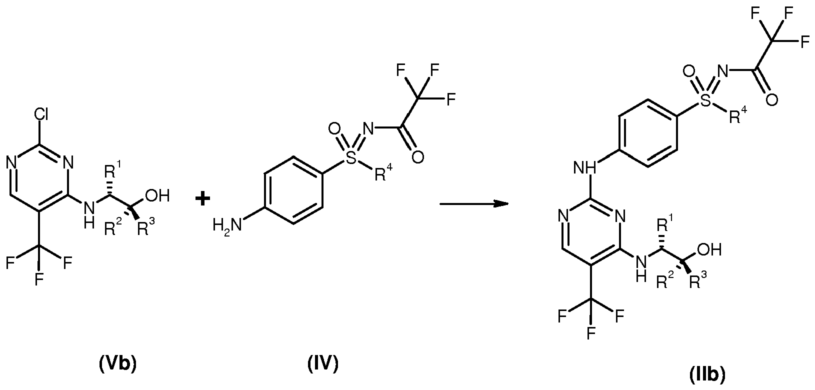
f) Cleavage of the protective group on the sulfoximine with formation of **(Ib).** 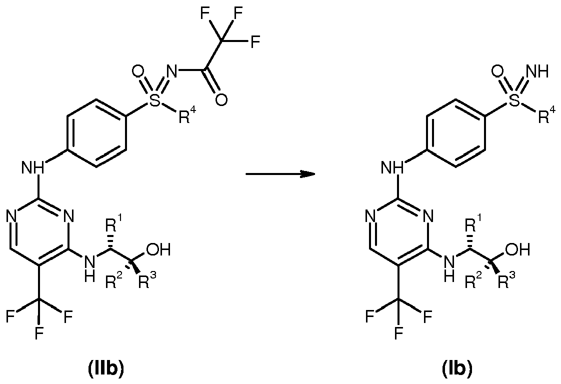
wherein the substituents R¹, R², R³ and R⁴ have the meanings given in general formula (I) of Claims 1 to 7.

10. Compounds according to one of Claims 1 to 7 for use as medicinal products.

11. Use of a compound according to one of Claims 1 to 7 for the production of a medicinal product for the treatment of cancer.

12. Compounds according to one of Claims 1 to 7 for use as medicinal products against cancer.

13. Pharmaceutical formulation containing a compound according to one of Claims 1 to 7.

## Revendications

1. Composés de formule générale (I) 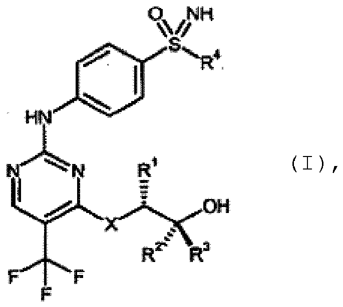 dans laquelle
X représente -O- ou -NH-, et
R¹ représente un groupe méthyle, éthyle, propyle ou isopropyle, et
R² et R³ représentent indépendamment l'un de l'autre un atome d'hydrogène, un groupe méthyle ou éthyle, et
R⁴ représente un groupe alkyle en C₁-C₆ ou un cycle cycloalkyle en C₃-C₇,
ainsi que leurs sels, diastéréoisomères et énantiomères.

2. Composés selon la revendication 1,
dans lesquels X représente -O-,
ainsi que leurs sels, diastéréoisomères et énantiomères.

3. Composés selon l'une quelconque des revendications 1 et 2,
dans lesquels R¹ représente un groupe méthyle,
ainsi que leurs sels, diastéréoisomères et énantiomères.

4. Composés selon l'une quelconque des revendications 1 à 3,
dans lesquels R² représente un groupe méthyle,
ainsi que leurs sels, diastéréoisomères et énantiomères.

5. Composés selon l'une quelconque des revendications 1 à 4,
dans lesquels R³ représente un atome d'hydrogène ou un groupe méthyle,
ainsi que leurs sels, diastéréoisomères et énantiomères.

6. Composés de selon l'une quelconque des revendications 1 à 5,
dans lesquels R⁴ représente un groupe méthyle ou éthyle ou un cycle cyclopropyle,
ainsi que leurs sels, diastéréoisomères et énantiomères.

7. Composés de formule générale (I) selon la revendication 1,
dans lesquels
X représente -O- ou -NH-, et
R¹ représente un groupe méthyle, et
R² représente un groupe méthyle, et
R³ représente un atome d'hydrogène ou un groupe méthyle, et
R⁴ représente un groupe méthyle ou éthyle ou un cycle cyclopropyle,
ainsi que leurs sels, diastéréoisomères et énantiomères.

8. Procédé pour la préparation de composés de formule générale (Ia), comprenant au moins l'une des étapes a)-h)
a) Oxydation d'un composé de formule **(IVd)** en le sulfoxyde de formule (**IVc**). 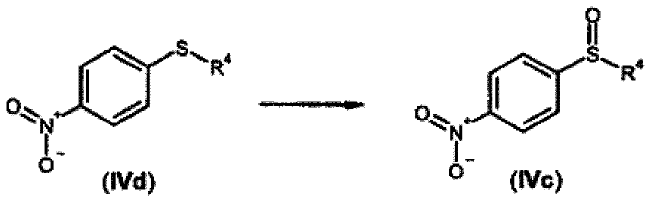
b₁) Imination directe du sulfoxyde de formule **(IVc)** conduisant à en une sulfoximine protégée de formule (**IVa**), 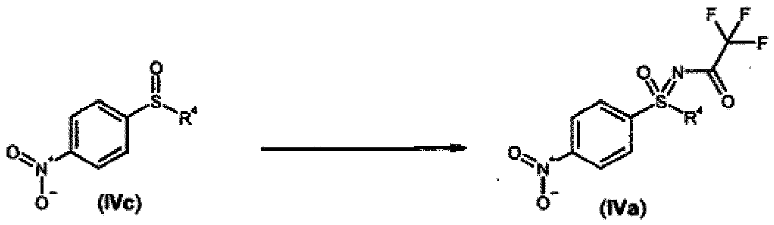 ou
b₂) Imination du sulfoxyde de formule (IVc) conduisant à une sulfoximine non protégée de formule **(IVb)** et introduction subséquente du groupe protecteur conduisant à un composé de formule (**IVa**). 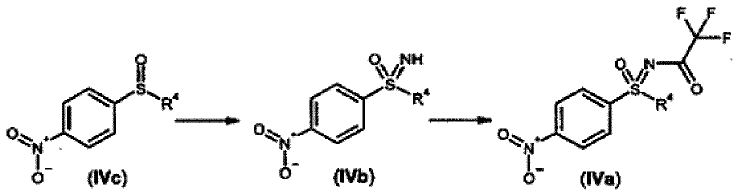
c) Réduction du composé de formule **(IVa)** en un composé de formule (IV) 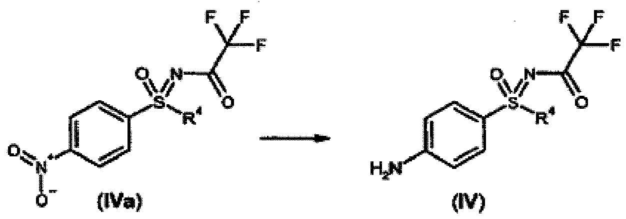
d) Fonctionnalisation de la position 4 de la 2,4-dichloro-5-iodo-pyrimidine (VII) par mise en réaction avec un diol mono-protégé de formule (VI), avec formation d'un composé intermédiaire de formule (Va). 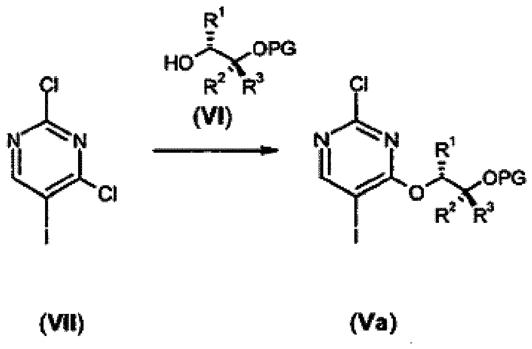
e) Préparation du composé intermédiaire 5-CF₃ (V). 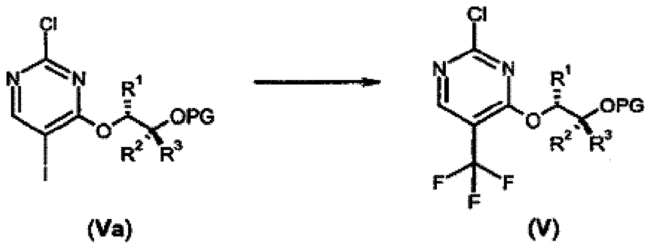
f) Combinaison des composés de formule (IV) et de formule (V), conduisant au composé intermédiaire de formule (III). 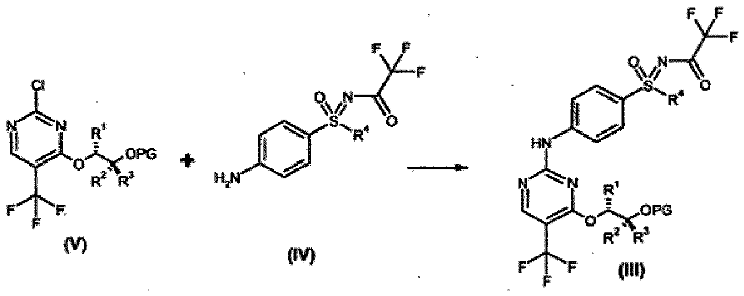
g) Élimination du groupe protecteur PG, avec formation de (II). 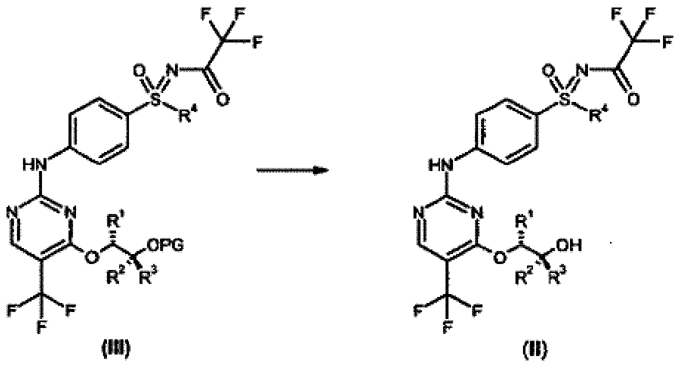
h) Élimination du groupe protecteur sur la sulfoximine avec formation de (**Ia**). 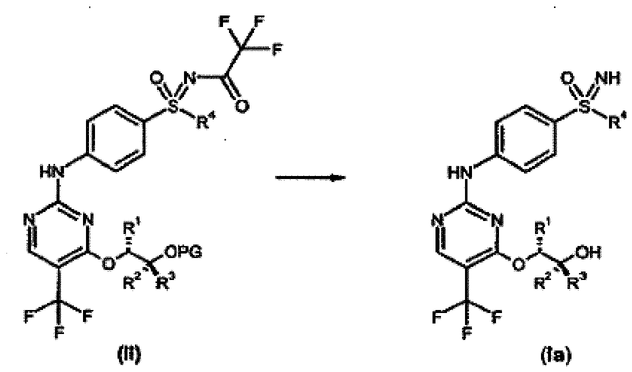
les substituants R¹, R², R³ et R⁴ ayant les significations indiquées dans la formule générale (I) des revendications 1 à 7.

9. Procédé pour la préparation de composés de formule générale (IIb), comprenant au moins l'une des étapes a)-f)
a) Oxydation d'un composé de formule **(IVd)** en le sulfoxyde de formule (**IVc**). 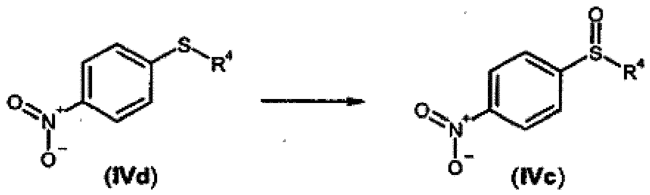
b₁) Imination directe du sulfoxyde de formule **(IVc)** conduisant à en une sulfoximine protégée de formule (**IVa**), 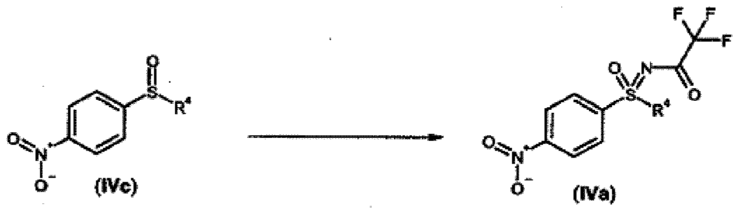 ou
b₂) Imination du sulfoxyde de formule **(IVc)** conduisant à une sulfoximine non protégée de formule **(IVb)** et introduction subséquente du groupe protecteur conduisant à un composé de formule (**IVa**). 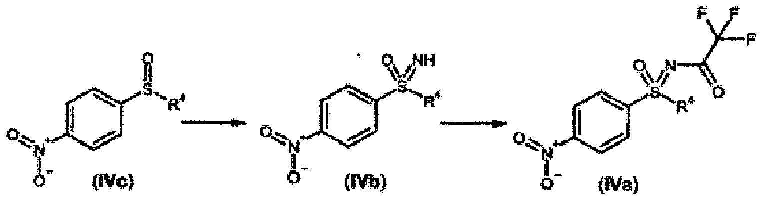
c) Réduction du composé de formule **(IVa)** en un composé de formule (IV) 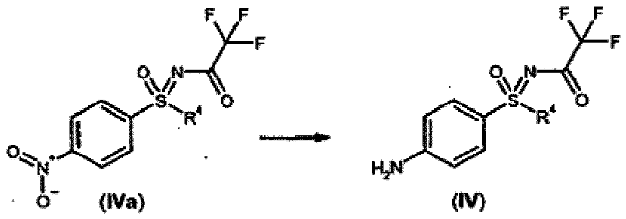
d) Fonctionnalisation de la position 4 de la 2,4-dichloro-5-trifluorométhyl-pyrimidine **(VIIb)** par mise en réaction avec une amine de formule (**VIa**), avec formation d'un composé intermédiaire de formule **(Vb).** 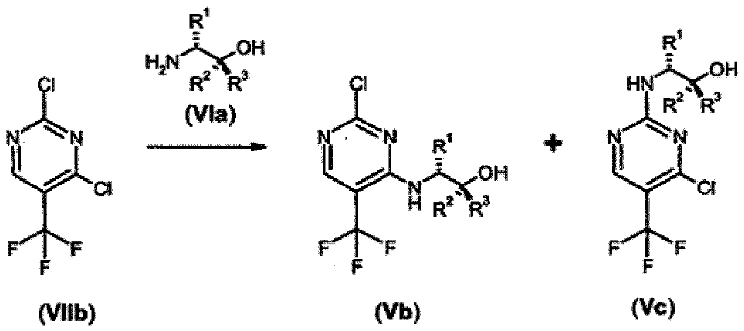
e) Combinaison des composés de formule **(Vb)** et de formule **(IV),** conduisant au composé intermédiaire de formule **(IIb).** 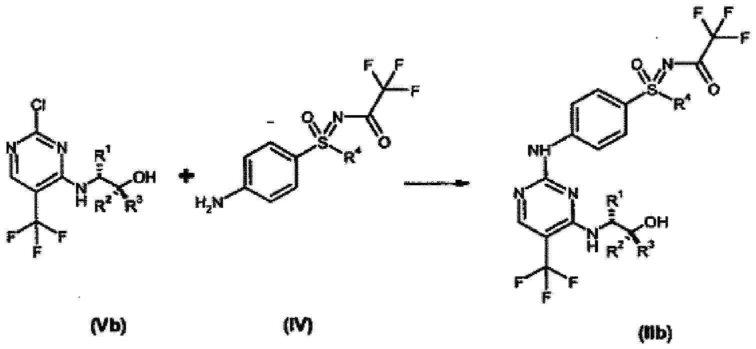
f) Élimination du groupe protecteur sur la sulfoximine avec formation de **(Ib).** 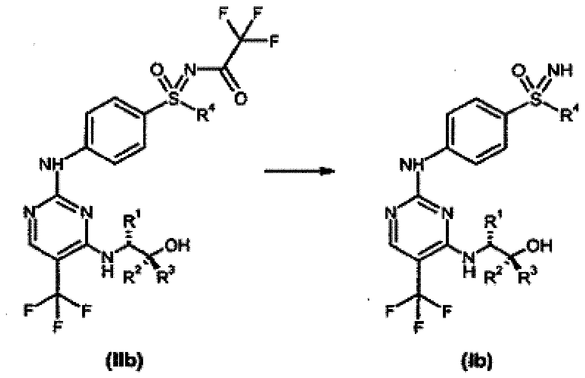
les substituants R¹, R², R³ et R⁴ ayant les significations indiquées dans la formule générale (I) des revendications 1 à 7.

10. Composés selon l'une quelconque des revendications 1 à 7, pour utilisation en tant que médicament.

11. Utilisation d'un composé selon l'une quelconque des revendications 1 à 7, pour la fabrication d'un médicament destiné au traitement du cancer.

12. Composés selon l'une quelconque des revendications 1 à 7, pour utilisation en tant que médicament contre le cancer.

13. Composition pharmaceutique contenant un composé selon l'une quelconque des revendications 1 à 7.
